# EUROPEAN PATENT APPLICATION

(11) **EP 4 212 519 A1**
(43) Date of publication of application: **19.07.2023**
(21) Application number: 21864553.9
(22) Date of filing: 12.08.2021
(51) Int. Cl.: C07D 307/77, C07D 407/12, C07D 409/12, H01L 51/00, H01L 51/50

(54) **HETEROCYCLIC COMPOUND AND ORGANIC LIGHT-EMITTING ELEMENT COMPRISING SAME**

(30) Priority: 04.09.2020 KR 20200113307
(71) Applicant: LT Materials Co., Ltd., Yongin-si, Gyeonggi-do 17118 (KR)
(72) Inventor: LEE, Nam-Jin, Yongin-si, Gyeonggi-do 17118 (KR); LEE, Gi-Back, Yongin-si, Gyeonggi-do 17118 (KR); JEONG, Won-Jang, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/010758
(87) International publication number: WO 2022/050592

(57) **Abstract**

The present specification relates to a heterocyclic compound represented by Chemical Formula 1, and an organic light emitting device comprising the same.

## Description

### [Technical Field]

This application claims priority to and the benefits of Korean Patent Application No. 10-2020-0113307, filed with the Korean Intellectual Property Office on September 4, 2020, the entire contents of which are incorporated herein by reference.

The present specification relates to a heterocyclic compound, and an organic light emitting device comprising the same.

### [Background Art]

An organic electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

Studies on an organic light emitting device comprising a compound capable of satisfying conditions required for materials usable in an organic light emitting device, for example, satisfying proper energy level, electrochemical stability, thermal stability and the like, and having a chemical structure capable of performing various roles required in an organic light emitting device depending on substituents have been required.

### Prior Art Documents

### Patent Documents

(Patent Document 1) US Patent No. 4,356,429

### [Disclosure]

### [Technical Problem]

The present application relates to a heterocyclic compound, and an organic light emitting device comprising the same.

### [Technical Solution]

One embodiment of the present application provides a heterocyclic compound represented by the following Chemical Formula 1.

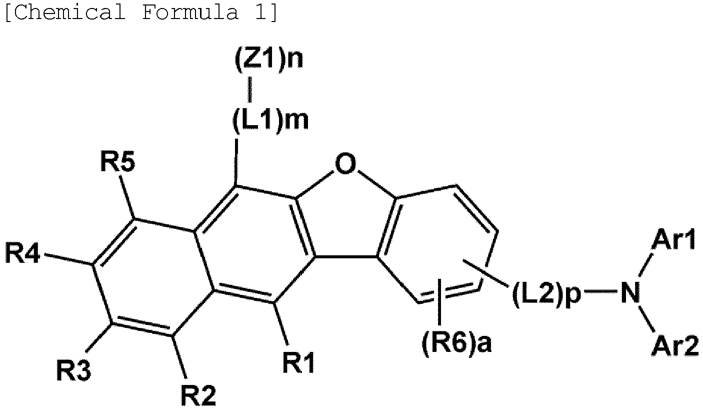

In Chemical Formula 1,
R1 to R6 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring,
L1 and L2 are the same as or different from each other, and each independently a direct bond; a substituted or unsubstituted C6 to C60 arylene group; or a substituted or unsubstituted C2 to C60 heteroarylene group,
Ar1 and Ar2 are the same as or different from each other, and each independently a substituted or unsubstituted C1 to C60 alkyl group; a substituted or unsubstituted C6 to C60 aryl group; a substituted or unsubstituted fluorenyl group; or a substituted or unsubstituted C2 to C60 heteroaryl group,
Z1 is a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted fluorenyl group; a substituted or unsubstituted C2 to C60 heteroaryl group; -P(=O)RR'; or -SiRR'R",
p and m are an integer of 0 to 4,
n is an integer of 1 to 6,
a is an integer of 0 to 3, and
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound represented by Chemical Formula 1.

### [Advantageous Effects]

A compound described in the present specification can be used as a material of an organic material layer of an organic light emitting device. The compound is capable of performing a role of a hole injection material, a hole transfer material, a light emitting material, an electron transfer material, an electron injection material, an electron blocking material, a hole blocking material or the like in the organic light emitting device. Particularly, the compound can be used as a hole transfer material, a light emitting material or an electron blocking material of the organic light emitting device.

Particularly, the heterocyclic compound according to the present application has an amine-based substituent and a -(L1)m-(Z1)n substituent in the naphthobenzofuran structure that is a core structure, and, by strengthening hole properties in the naphthobenzofuran skeleton that is a core structure, is capable of controlling a wide band gap and a high T1 value, and accordingly, the compound shows excellent efficiency when used as a hole transfer material, a light emitting material or an electron blocking material of an organic light emitting device.

### [Description of Drawings]

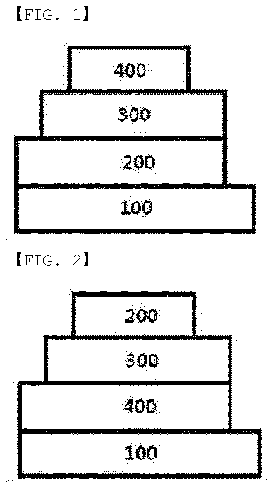

FIG. 1 to FIG. 3 are diagrams each schematically illustrating a lamination structure of an organic light emitting device according to one embodiment of the present application.

### [Reference Numeral]

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

### [Mode for Disclosure]

Hereinafter, the present application will be described in more detail.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The number of carbon atoms of the aryl group may be from 6 to 60, specifically from 6 to 40 and more specifically from 6 to 25. Specific examples of the aryl group may include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, an indenyl group, an acenaphthylenyl group, a 2,3-dihydro-1H-indenyl group, a fused ring group thereof, and the like, but are not limited thereto.

In the present specification, a fluorenyl group may be substituted, and adjacent substituents may bond to each other to form a ring.

When the fluorenyl group is substituted, 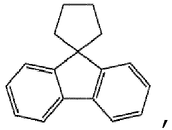 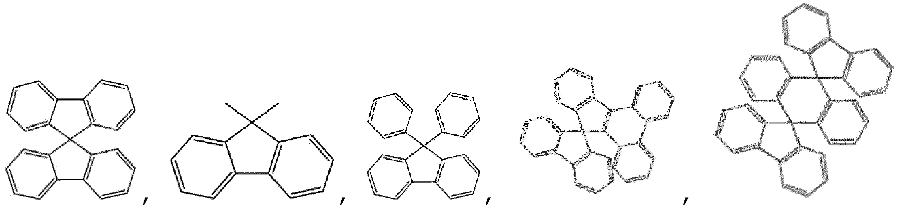 and the like may be included, however, the structure is not limited thereto.

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c] [1,2,5]thiadiazolyl group, a 5,10-dihydrobenzo[b,e] [1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for being divalent. In addition, the heteroarylene group means the heteroaryl group having two bonding sites, that is, a divalent group. The descriptions on the heteroaryl group provided above may be applied thereto except for being divalent.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide may include a diphenylphosphine oxide group, a dinaphthylphosphine oxide group and the like, but are not limited thereto.

In the present specification, the silyl group is a substituent comprising Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

As the aliphatic or aromatic hydrocarbon ring or heteroring that adjacent groups may form, the structures illustrated as the cycloalkyl group, the cycloheteroalkyl group, the aryl group and the heteroaryl group described above may be used except for not being monovalent.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of deuterium; a halogen group; a cyano group; C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; -P(=O)RR'; and -NRR', or being unsubstituted, or being substituted with a substituent linking two or more substituents selected from among the substituents illustrated above, or being unsubstituted, and

R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0% or a hydrogen content being 100%. In other words, an expression of "substituent X is hydrogen" does not exclude deuterium unlike a hydrogen content being 100% or a deuterium content being 0%, and therefore, may mean a state in which hydrogen and deuterium are mixed.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 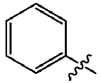 means that the total number of substituents that the phenyl group may have is 5 (T1 in the formula), and the number of deuterium among these is 1 (T2 in the formula) . In other words, having a deuterium content of 20% in a phenyl group may be represented by the following structural formulae.

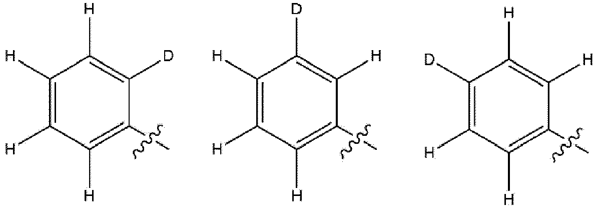

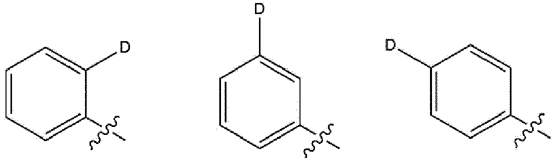

In addition, in one embodiment of the present application, "a phenyl group having a deuterium content of 0%" may mean a phenyl group that does not include a deuterium atom, that is, a phenyl group that has 5 hydrogen atoms.

In the present specification, a structure represented by a form of -(La)m1-(Za)n1 in the chemical formulae may mean a substituent. La is a linker and Za is a substituent, and m1 being 2 or greater may be interpreted as the linkers La being linked to each other. For example, m1 being 3 may be interpreted as -(La)-(La)-(La)-(Za)n1.

One embodiment of the present application provides a compound represented by Chemical Formula 1.

The heterocyclic compound of Chemical Formula 1 of the present application has an amine-based substituent and a -(L1)m-(Z1)n substituent having hole properties, and when used as a hole transfer layer, a hole transfer auxiliary layer or a light emitting layer of an organic light emitting device later, the unshared electron pair of the amine substituent improves the flow of holes enhancing a hole transfer ability of the hole transfer layer, and when used as an electron blocking layer, deterioration of a hole transfer material caused by electrons invading the hole transfer layer may be suppressed.

In addition, by the -(L1)m-(Z1)n substituent with strengthened hole properties and amine part of the amine-based substituent bonding to each other, amine derivative planarity and glass transition temperature increase, which increases thermal stability of the heterocyclic compound, and as a result, a lifetime of an organic light emitting device comprising the same is improved as well.

In addition, through adjusting a band gap and a T1 value, a hole transfer ability is enhanced, and molecular stability increases as well, which may lower a driving voltage of the device, enhance light efficiency, and enhance lifetime properties of the device by thermal stability of the compound.

In one embodiment of the present application, Chemical Formula 1 may be represented by any one of the following Chemical Formulae 2 to 5.

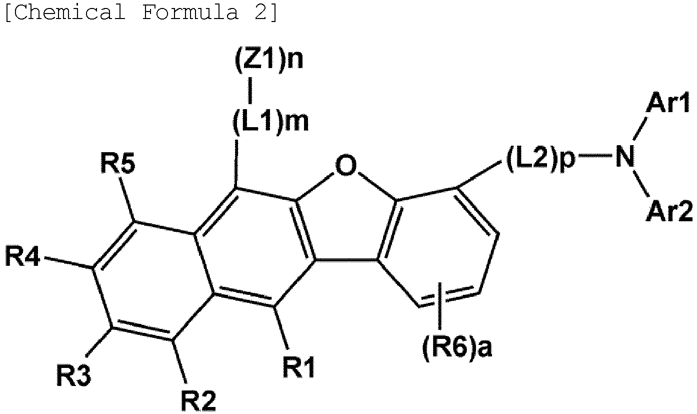

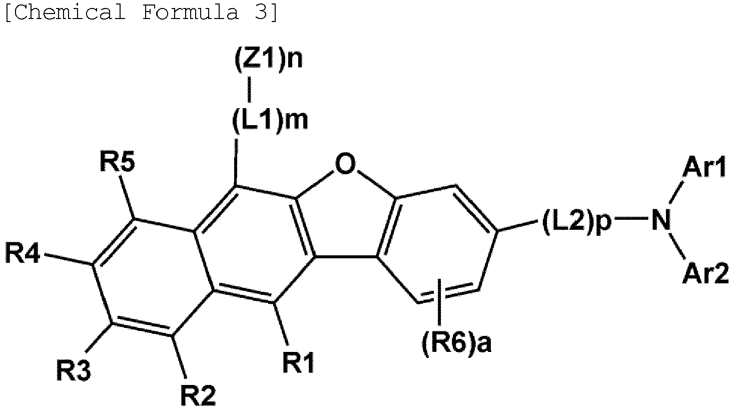

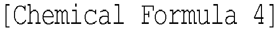

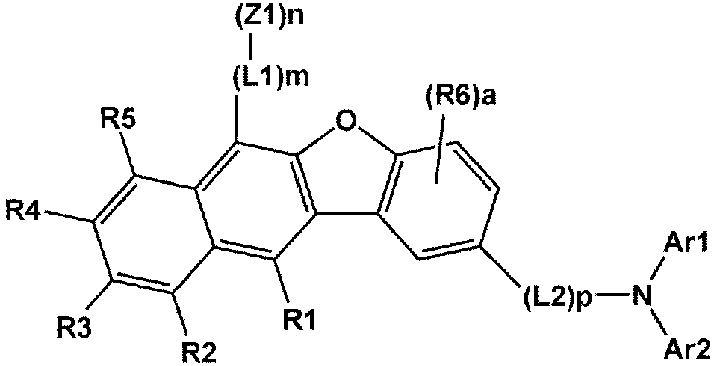

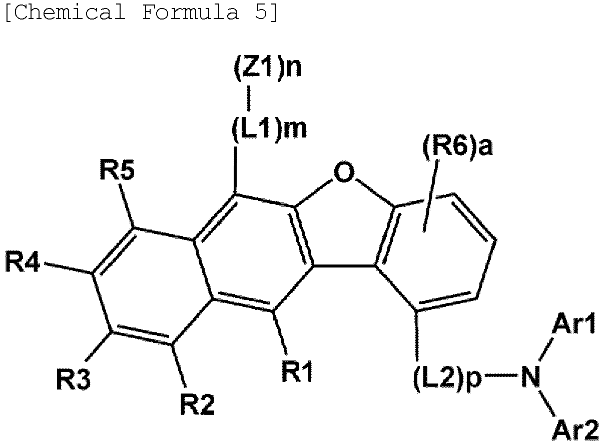

In Chemical Formulae 2 to 5,
R1 to R6, L1, L2, Z1, Ar1, Ar2, p, m, n and a have the same definitions as in Chemical Formula 1.

In one embodiment of the present application, R1 to R6 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more groups adjacent to each other may bond to each other to form a substituted or unsubstituted C6 to C60 aliphatic or aromatic hydrocarbon ring or a substituted or unsubstituted C2 to C60 aliphatic or aromatic heteroring.

In another embodiment, R1 to R6 are the same as or different from each other, and may be each independently hydrogen; deuterium; a substituted or unsubstituted C1 to C60 alkyl group; a substituted or unsubstituted C6 to C60 aryl group; a substituted or unsubstituted C2 to C60 heteroaryl group; -P(=O)RR'; or - SiRR'R".

In another embodiment, R1 to R6 are the same as or different from each other, and may be each independently hydrogen; deuterium; a substituted or unsubstituted C1 to C40 alkyl group; a substituted or unsubstituted C6 to C40 aryl group; a substituted or unsubstituted C2 to C40 heteroaryl group; -P(=O)RR'; or - SiRR'R".

In another embodiment, R1 to R6 are the same as or different from each other, and may be each independently hydrogen; deuterium; a C1 to C40 alkyl group; a C6 to C40 aryl group; a C2 to C40 heteroaryl group; -P(=O)RR'; or -SiRR'R".

In another embodiment, R1 to R6 may be hydrogen; or deuterium.

In one embodiment of the present application, R, R' and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another embodiment, R, R' and R" are the same as or different from each other, and may be each independently a substituted or unsubstituted C6 to C60 aryl group.

In another embodiment, R, R' and R" are the same as or different from each other, and may be each independently a substituted or unsubstituted C6 to C60 monocyclic or polycyclic aryl group.

In another embodiment, R, R' and R" are the same as or different from each other, and may be each independently a substituted or unsubstituted C6 to C40 monocyclic aryl group.

In another embodiment, R, R' and R" are the same as or different from each other, and may be each independently a C6 to C20 monocyclic aryl group.

In another embodiment, R, R' and R" may be a phenyl group.

In one embodiment of the present application, L1 and L2 are the same as or different from each other, and may be each independently a direct bond; a substituted or unsubstituted C6 to C60 arylene group; or a substituted or unsubstituted C2 to C60 heteroarylene group.

In another embodiment, L1 and L2 are the same as or different from each other, and may be each independently a direct bond; or a substituted or unsubstituted C6 to C60 arylene group.

In another embodiment, L1 and L2 are the same as or different from each other, and may be each independently a direct bond; or a substituted or unsubstituted C6 to C40 arylene group.

In another embodiment, L1 and L2 are the same as or different from each other, and may be each independently a direct bond; or a C6 to C20 arylene group.

In another embodiment, L1 and L2 are the same as or different from each other, and may be each independently a direct bond; a monocyclic C6 to C10 arylene group; or a polycyclic C10 to C20 arylene group.

In another embodiment, L1 and L2 are the same as or different from each other, and may be each independently a direct bond; a phenylene group; or a biphenylene group.

In one embodiment of the present application, Z1 may be a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted fluorenyl group; a substituted or unsubstituted C2 to C60 heteroaryl group; -P(=O)RR'; or -SiRR'R".

In another embodiment, Z1 may be a substituted or unsubstituted C6 to C60 aryl group; a substituted or unsubstituted fluorenyl group; a substituted or unsubstituted C2 to C60 heteroaryl group; -P(=O)RR'; or -SiRR'R".

In another embodiment, Z1 may be a substituted or unsubstituted C6 to C60 aryl group; a substituted or unsubstituted fluorenyl group; or a substituted or unsubstituted C2 to C60 heteroaryl group.

In another embodiment, Z1 may be a substituted or unsubstituted C6 to C40 aryl group; a substituted or unsubstituted fluorenyl group; or a substituted or unsubstituted C2 to C40 heteroaryl group.

In another embodiment, Z1 may be a C6 to C40 aryl group; a fluorenyl group unsubstituted or substituted with one or more substituents selected from the group consisting of a C1 to C10 alkyl group and a C6 to C20 aryl group; or a C2 to C40 heteroaryl group.

In another embodiment, Z1 may be a C6 to C40 aryl group; a fluorenyl group unsubstituted or substituted with one or more substituents selected from the group consisting of a C1 to C10 alkyl group and a C6 to C20 aryl group; or a C2 to C40 heteroaryl group comprising one or more of O, S and N as a heteroatom.

In another embodiment, Z1 may be a phenyl group; a biphenyl group; a naphthyl group; a dimethylfluorenyl group; a diphenylfluorenyl group; a spirobifluorenyl group; a phenanthrenyl group; a triphenylenyl group; a terphenyl group; a dibenzofuran group; a dibenzothiophene group or a carbazole group.

In one embodiment of the present application, Chemical Formula 1 may be represented by any one of the following Chemical Formulae 6 to 9.

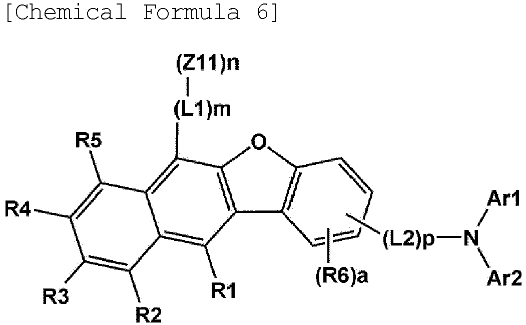

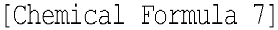

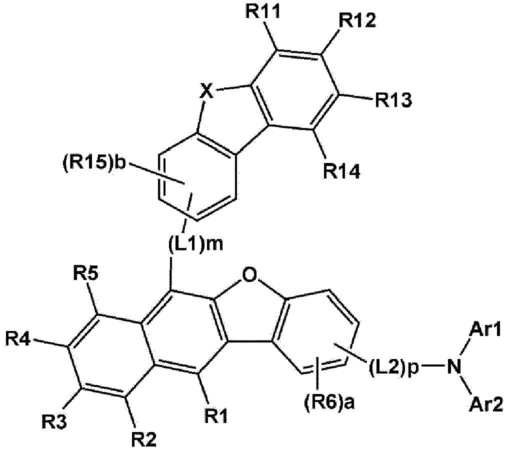

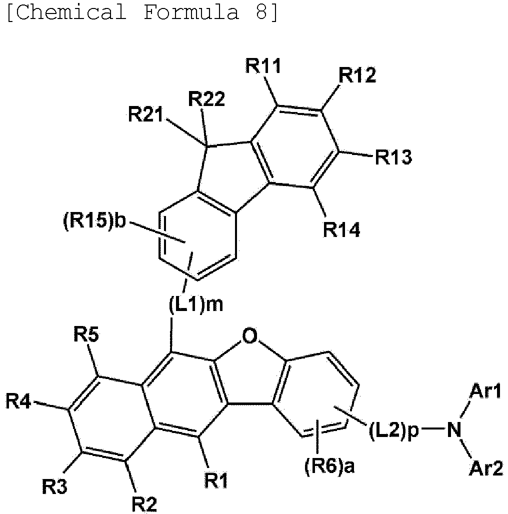

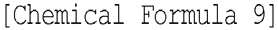

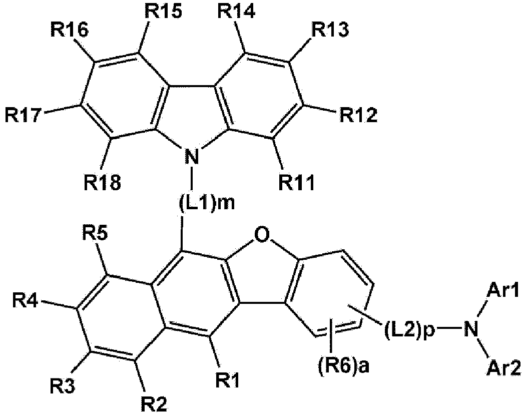

In Chemical Formulae 6 to 9,
R1 to R6, L1, L2, Ar1, Ar2, p, m, n and a have the same definitions as in Chemical Formula 1,
Z11 is a substituted or unsubstituted C6 to C60 aryl group,
X is O; or S,
R11 to R18 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring,
R21 and R22 are the same as or different from each other, and each independently a substituted or unsubstituted C1 to C60 alkyl group; or a substituted or unsubstituted C6 to C60 aryl group, or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring,
b is an integer of 0 to 3, and
R, R' and R" have the same definitions as in Chemical Formula 1.

In one embodiment of the present application, R11 to R18 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and - SiRR'R", or two or more groups adjacent to each other may bond to each other to form a substituted or unsubstituted C6 to C60 aliphatic or aromatic hydrocarbon ring or a substituted or unsubstituted C2 to C60 aliphatic or aromatic heteroring.

In another embodiment, R11 to R18 are the same as or different from each other, and may be each independently selected from the group consisting of hydrogen; deuterium; a substituted or unsubstituted C6 to C60 aryl group; a substituted or unsubstituted C2 to C60 heteroaryl group; -P(=O)RR'; and - SiRR'R".

In another embodiment, R11 to R18 are the same as or different from each other, and may be each independently selected from the group consisting of hydrogen; deuterium; a substituted or unsubstituted C6 to C40 aryl group; a substituted or unsubstituted C2 to C40 heteroaryl group; -P(=O)RR'; and - SiRR'R".

In another embodiment, R11 to R18 may be hydrogen; or deuterium.

In one embodiment of the present application, R21 and R22 are the same as or different from each other, and each independently a substituted or unsubstituted C1 to C60 alkyl group; or a substituted or unsubstituted C6 to C60 aryl group, or two or more groups adjacent to each other may bond to each other to form a substituted or unsubstituted C6 to C60 aliphatic or aromatic hydrocarbon ring or a substituted or unsubstituted C2 to C60 aliphatic or aromatic heteroring.

In another embodiment, R21 and R22 are the same as or different from each other, and each independently a substituted or unsubstituted C1 to C40 alkyl group; or a substituted or unsubstituted C6 to C40 aryl group, or two or more groups adjacent to each other may bond to each other to form a substituted or unsubstituted C6 to C40 aliphatic or aromatic hydrocarbon ring.

In another embodiment, R21 and R22 are the same as or different from each other, and each independently a C1 to C40 alkyl group; or a C6 to C40 aryl group, or two or more groups adjacent to each other may bond to each other to form a C6 to C40 aliphatic or aromatic hydrocarbon ring.

In another embodiment, R21 and R22 are the same as or different from each other, and each independently a C1 to C10 alkyl group; or a C6 to C10 aryl group, or two or more groups adjacent to each other may bond to each other to form a C6 to C30 aliphatic or aromatic hydrocarbon ring.

In another embodiment, R21 and R22 are the same as or different from each other, and each independently a methyl group; or a phenyl group, or two or more groups adjacent to each other may bond to each other to form a fluorenyl ring.

In another embodiment, R21 and R22 may be the same as each other.

In one embodiment of the present application, Ar1 and Ar2 are the same as or different from each other, and may be each independently a substituted or unsubstituted C1 to C60 alkyl group; a substituted or unsubstituted C6 to C60 aryl group; a substituted or unsubstituted fluorenyl group; or a substituted or unsubstituted C2 to C60 heteroaryl group.

In another embodiment, Ar1 and Ar2 are the same as or different from each other, and may be each independently a substituted or unsubstituted C1 to C40 alkyl group; a substituted or unsubstituted C6 to C40 aryl group; a substituted or unsubstituted fluorenyl group; or a substituted or unsubstituted C2 to C40 heteroaryl group.

In another embodiment, Ar1 and Ar2 are the same as or different from each other, and may be each independently a C6 to C40 aryl group unsubstituted or substituted with one or more substituents selected from the group consisting of a C6 to C40 aryl group and a C2 to C40 heteroaryl group; a fluorenyl group unsubstituted or substituted with one or more substituents selected from the group consisting of a C6 to C40 aryl group and a C1 to C20 alkyl group; or a C2 to C40 heteroaryl group.

In another embodiment, Ar1 and Ar2 are the same as or different from each other, and may be each independently a biphenyl group; a terphenyl group; a phenanthrenyl group; a naphthyl group; a triphenylenyl group; a dimethylfluorenyl group; a diphenylfluorenyl group; a spirobifluorenyl group; a dibenzofuran group; a dibenzothiophene group; or a phenyl group unsubstituted or substituted with one or more substituents selected from the group consisting of a dibenzofuran group, a dibenzothiophene group and a biphenyl group.

In one embodiment of the present application, 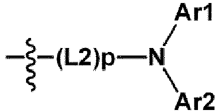 of Chemical Formula 1 may be represented by any one of the following Chemical Formulae 1-1 to 1-3.

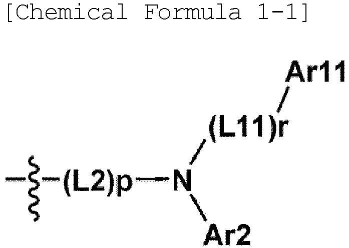

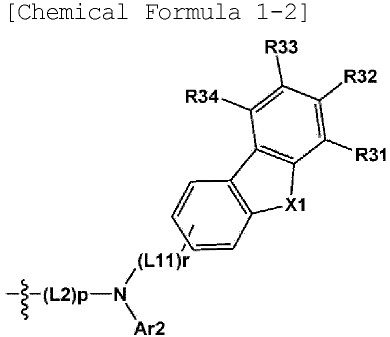

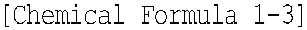

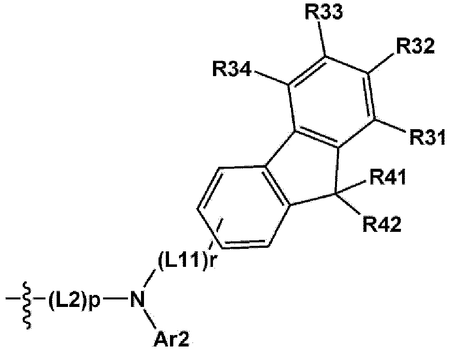

In Chemical Formulae 1-1 to 1-3,
L2, p and Ar2 have the same definitions as in Chemical Formula 1, 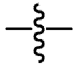
means a position linked to Chemical Formula 1,
X1 is O; or S,
L11 is a direct bond; a substituted or unsubstituted C6 to C60 arylene group; or a substituted or unsubstituted C2 to C60 heteroarylene group,
Ar11 is a substituted or unsubstituted C6 to C60 aryl group,
R31 to R34 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring,
R41 and R42 are the same as or different from each other, and each independently a substituted or unsubstituted C1 to C60 alkyl group; or a substituted or unsubstituted C6 to C60 aryl group, or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring,
r is an integer of 0 to 3, and
R, R' and R" have the same definitions as in Chemical Formula 1.

In one embodiment of the present application, R41 and R42 have the same definitions as R21 and R22.

In one embodiment of the present application, R31 to R34 have the same definitions as R11 to R18.

In one embodiment of the present application, L11 may be a direct bond; a substituted or unsubstituted C6 to C60 arylene group; or a substituted or unsubstituted C2 to C60 heteroarylene group.

In another embodiment, L11 may be a direct bond; a substituted or unsubstituted C6 to C40 arylene group; or a substituted or unsubstituted C2 to C40 heteroarylene group.

In another embodiment, L11 may be a direct bond; or a substituted or unsubstituted C6 to C40 arylene group.

In another embodiment, L11 may be a direct bond; or a C6 to C40 arylene group.

In another embodiment, L11 may be a direct bond; or a C6 to C30 arylene group.

In another embodiment, L11 may be a direct bond; or a phenylene group.

In one embodiment of the present application, Ar11 may be a substituted or unsubstituted C6 to C60 aryl group.

In another embodiment, Ar11 may be a substituted or unsubstituted C6 to C40 aryl group.

In another embodiment, Ar11 may be a substituted or unsubstituted C6 to C20 aryl group.

In another embodiment, Ar11 may be a substituted or unsubstituted phenyl group; a substituted or unsubstituted biphenyl group; a substituted or unsubstituted terphenyl group; a substituted or unsubstituted phenanthrenyl group; a substituted or unsubstituted naphthyl group; or a substituted or unsubstituted triphenylenyl group.

In another embodiment, Ar11 may be a biphenyl group; a terphenyl group; a phenanthrenyl group; a naphthyl group; a triphenylenyl group; or a phenyl group unsubstituted or substituted with one or more substituents selected from the group consisting of a dibenzofuran group, a dibenzothiophene group and a biphenyl group.

In one embodiment of the present application, Chemical Formula 7 may be represented by any one of the following Chemical Formulae 7-1 to 7-4.

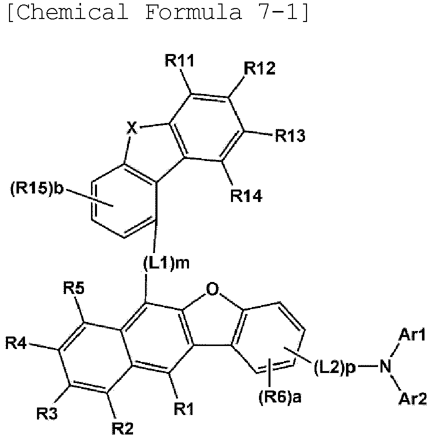

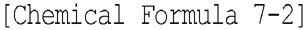

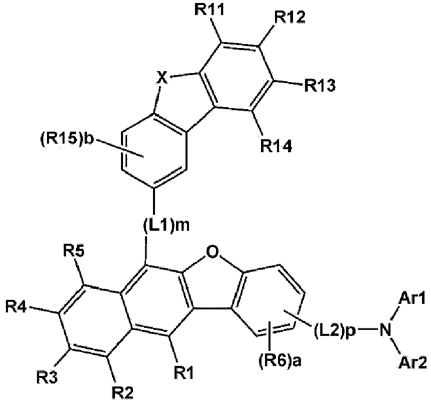

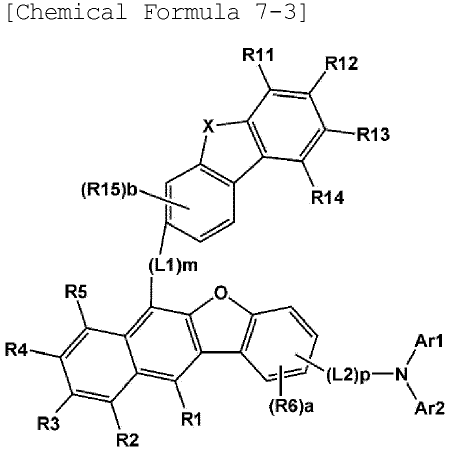

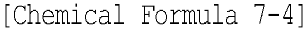

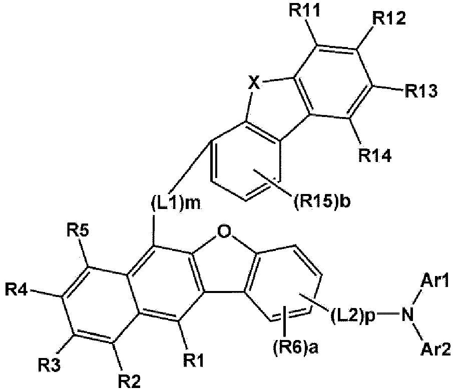

In Chemical Formulae 7-1 to 7-4,
each substituent has the same definition as in Chemical Formula 7.

In one embodiment of the present application, Chemical Formula 8 may be represented by any one of the following Chemical Formulae 8-1 to 8-4.

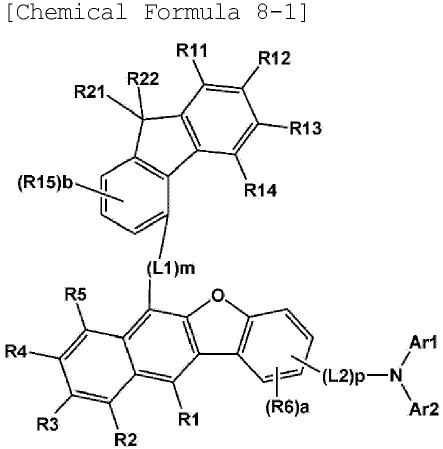

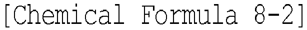

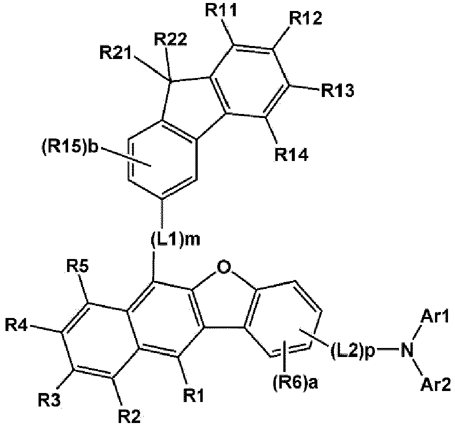

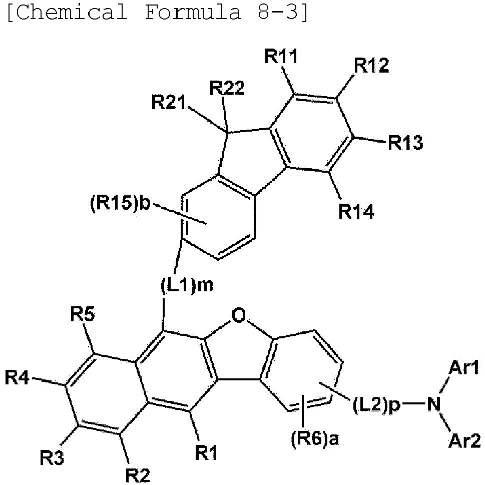

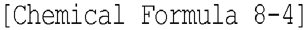

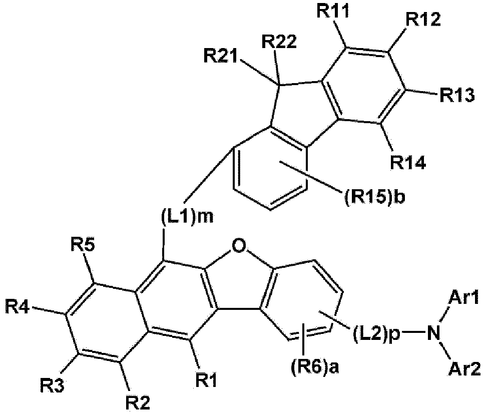

In Chemical Formulae 8-1 to 8-4,
each substituent has the same definition as in Chemical Formula 8.

According to one embodiment of the present application, Chemical Formula 1 may be represented by any one of the following compounds, but is not limited thereto.

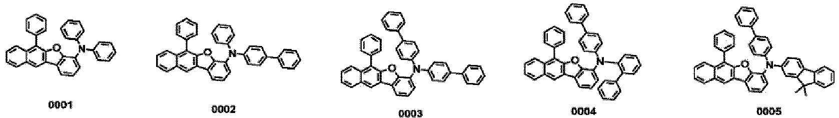

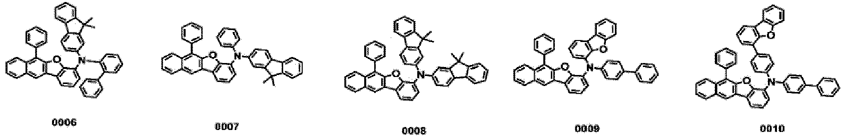

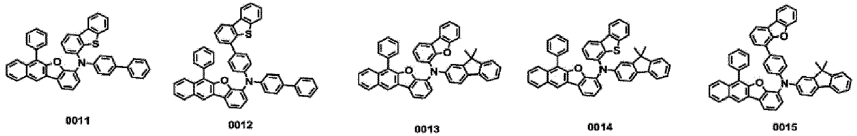

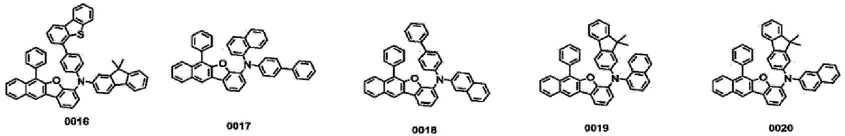

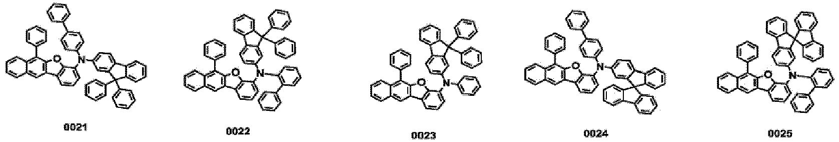

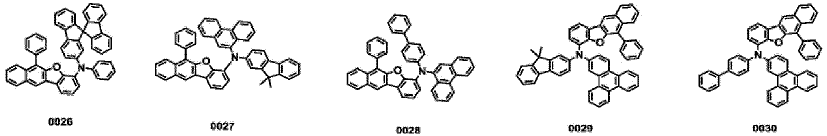

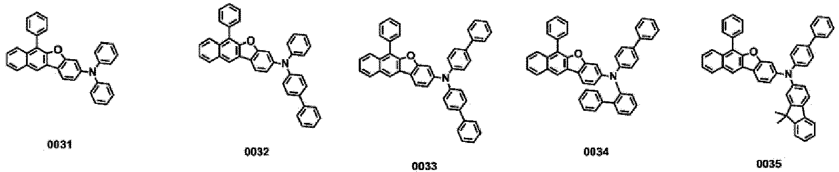

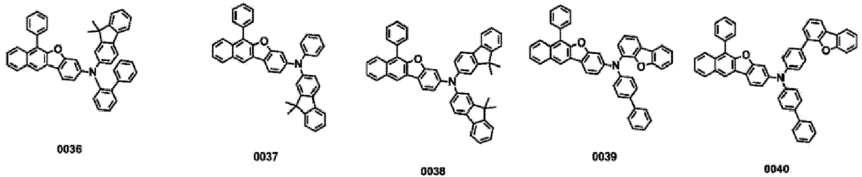

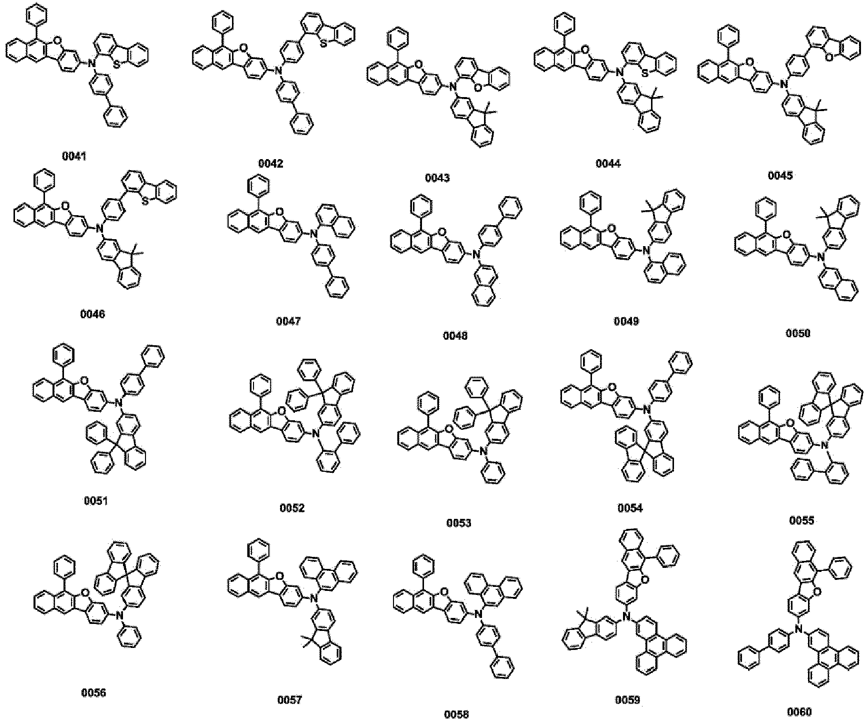

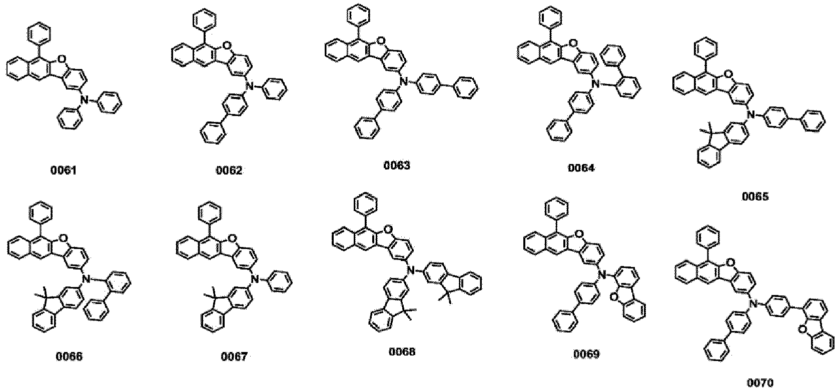

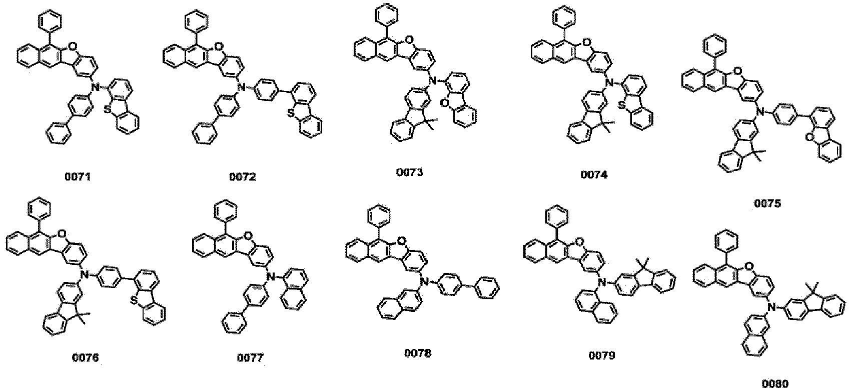

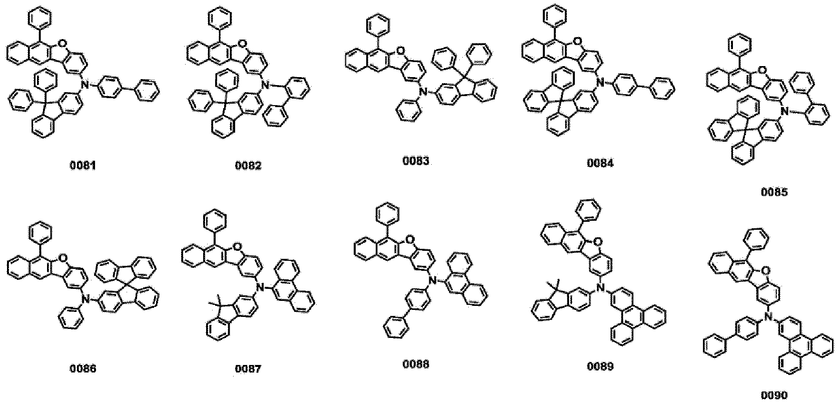

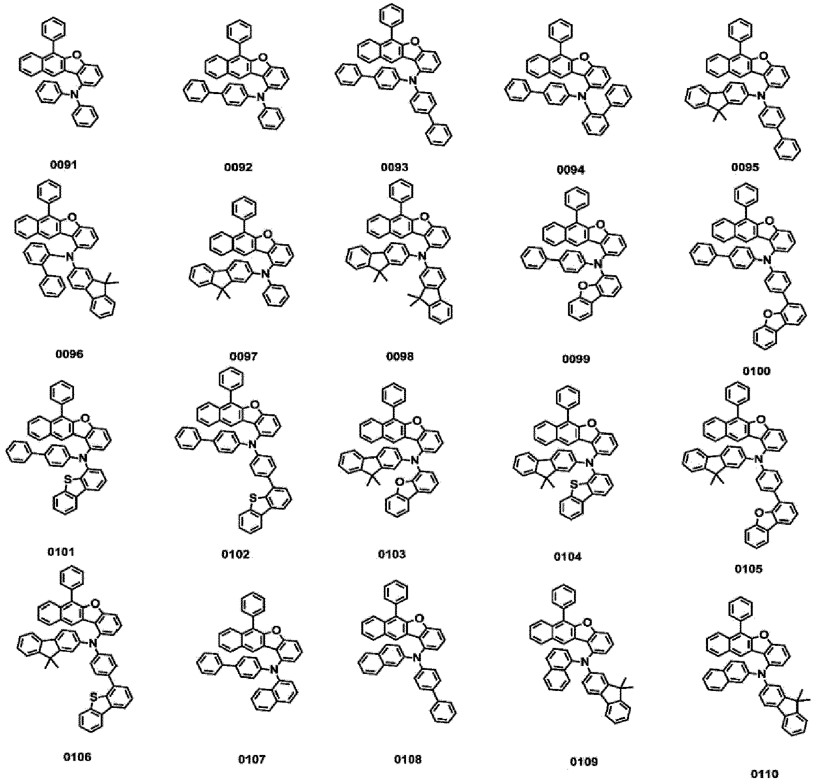

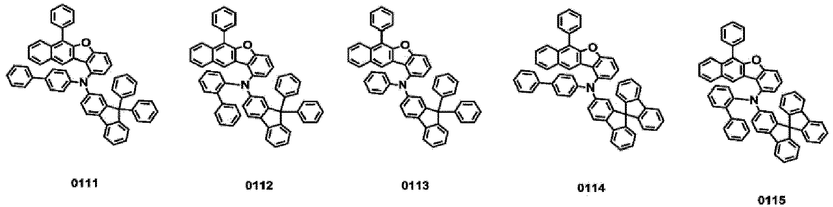

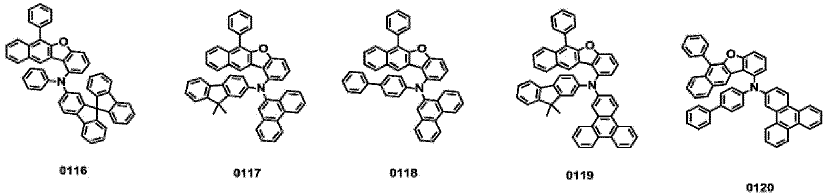

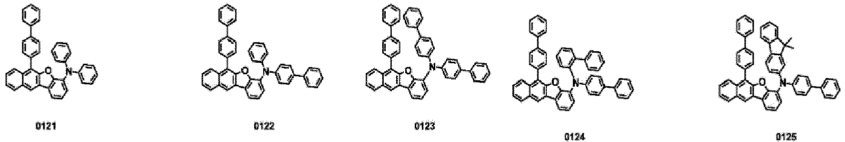

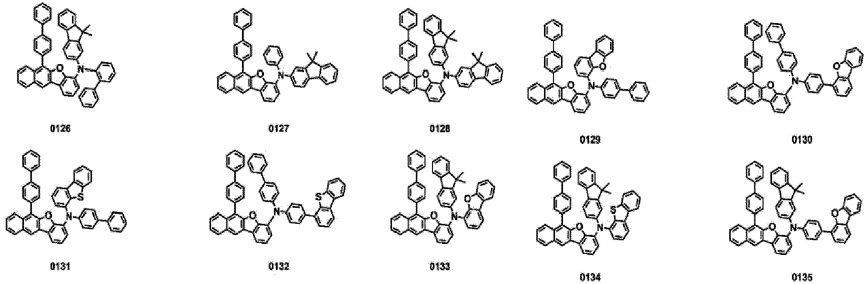

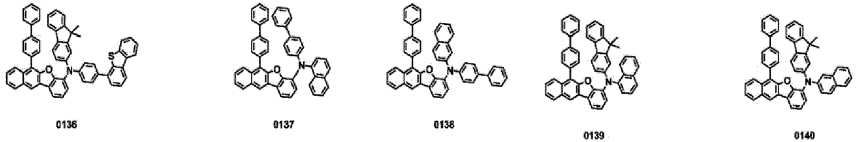

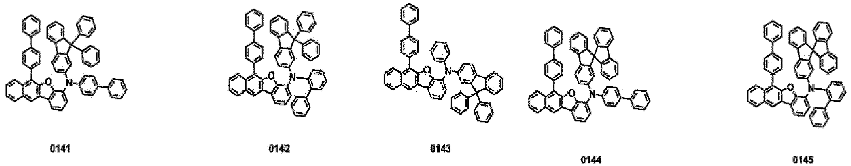

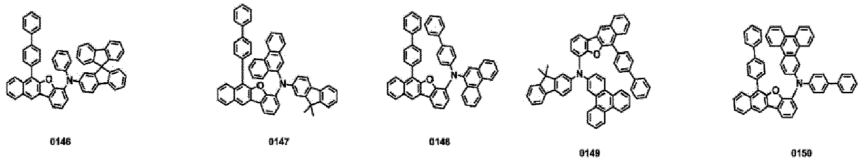

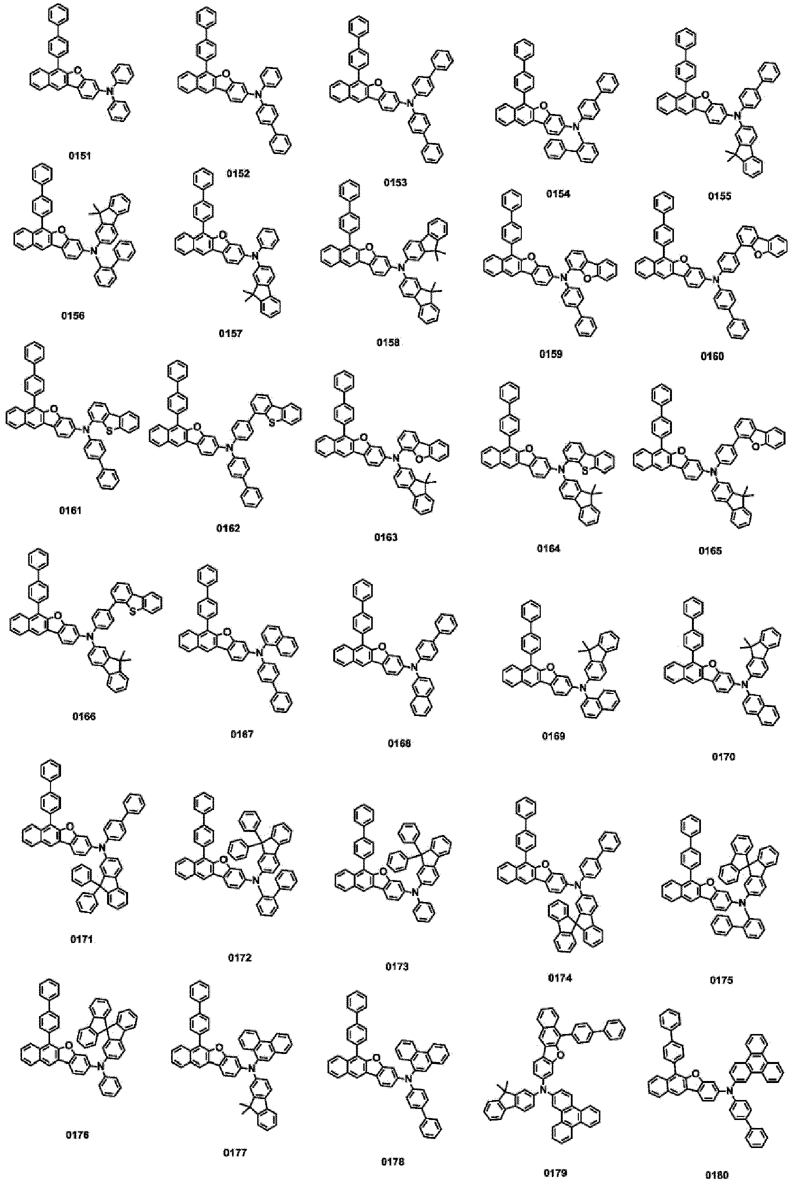

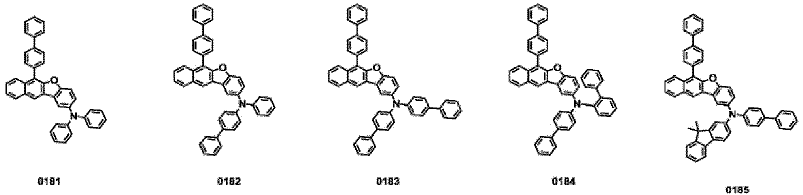

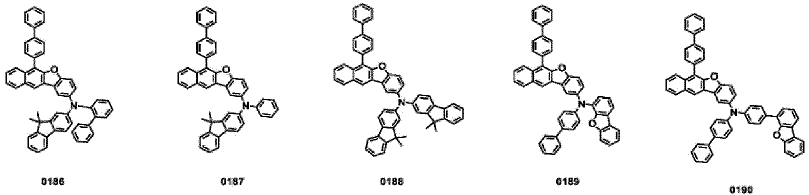

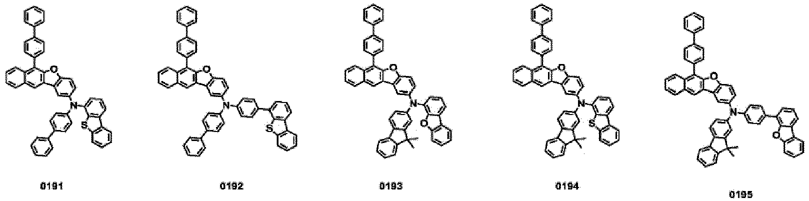

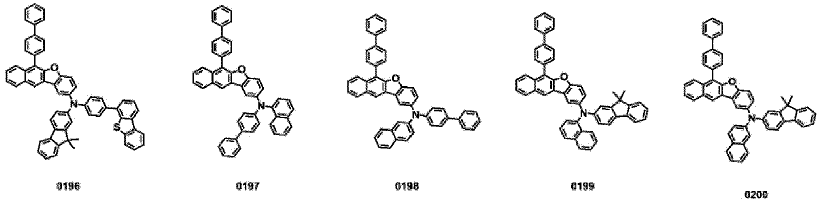

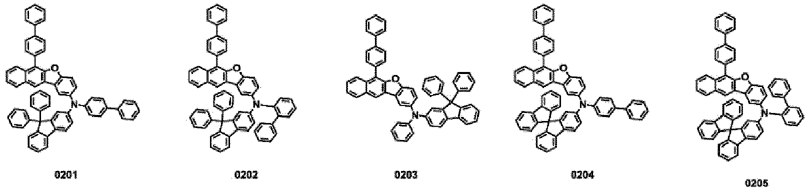

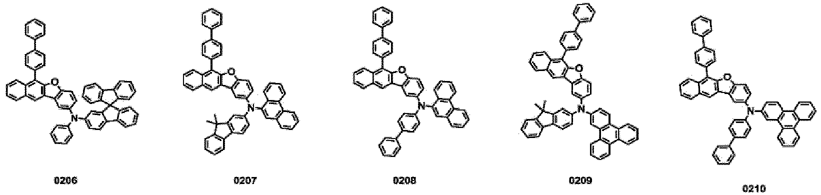

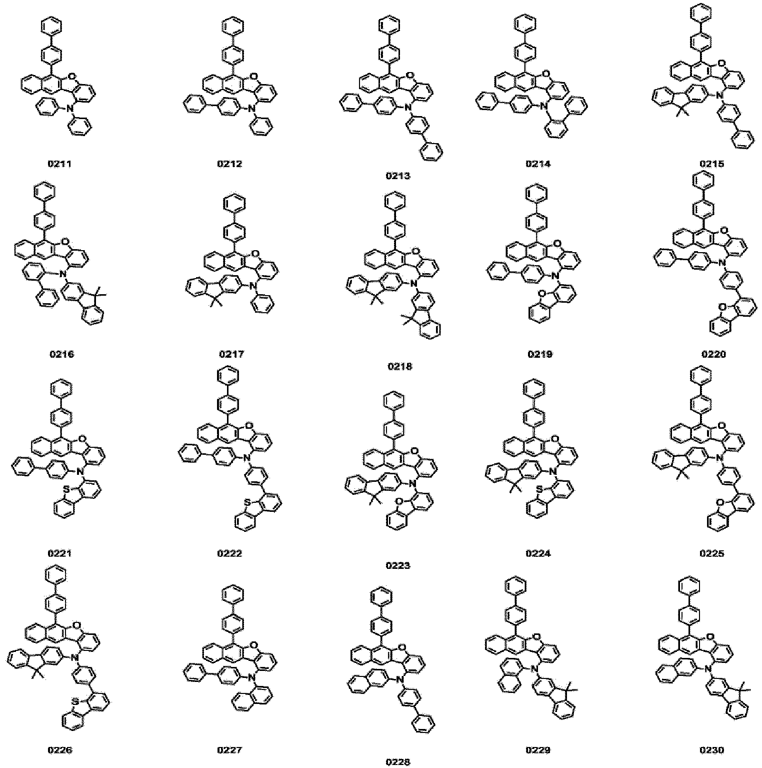

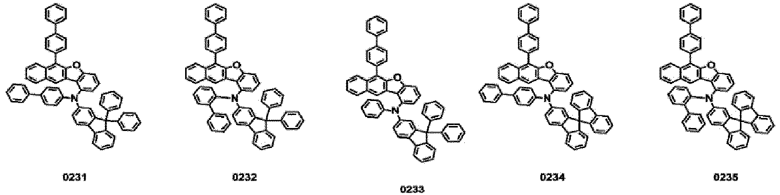

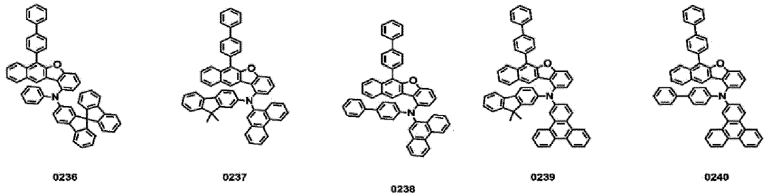

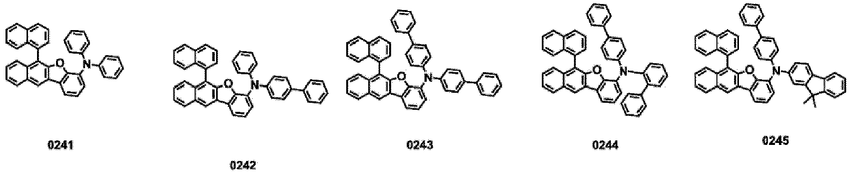

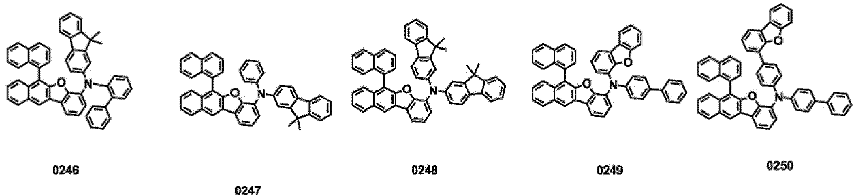

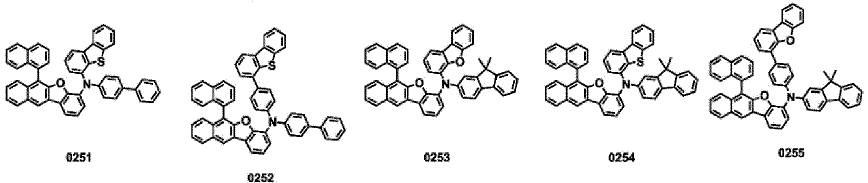

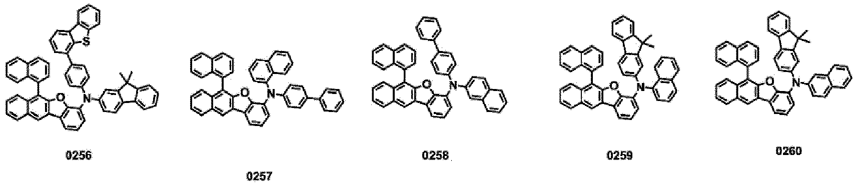

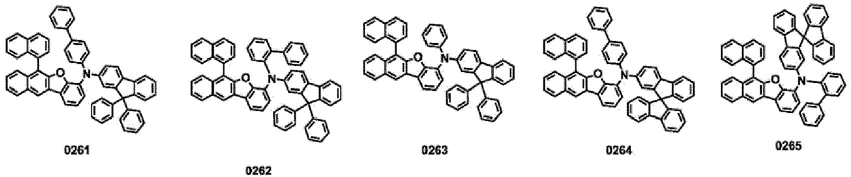

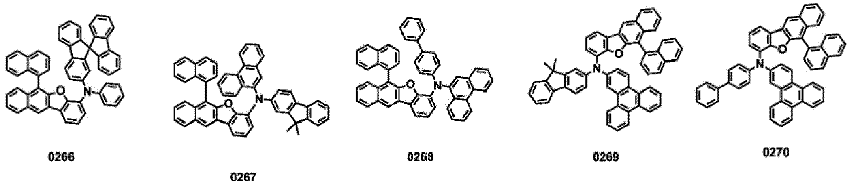

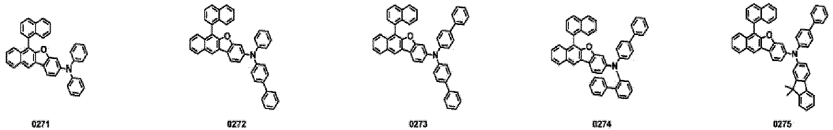

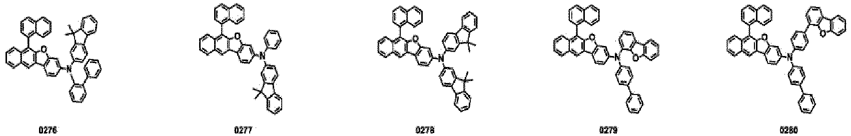

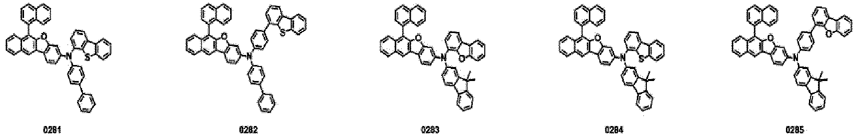

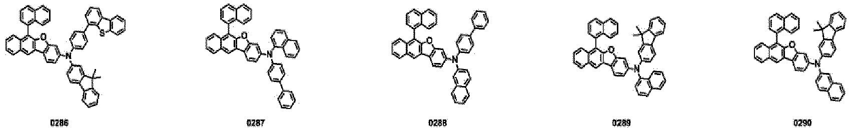

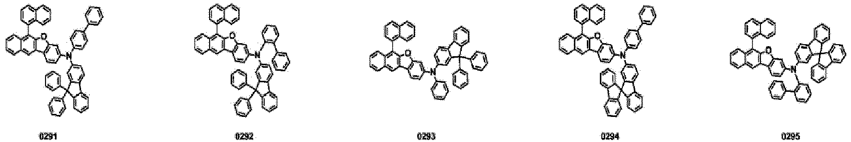

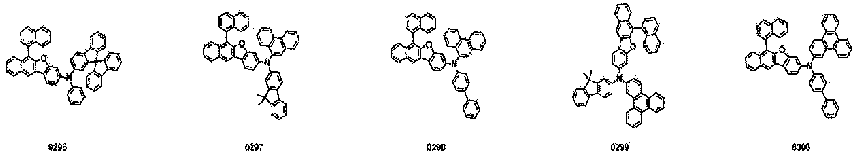

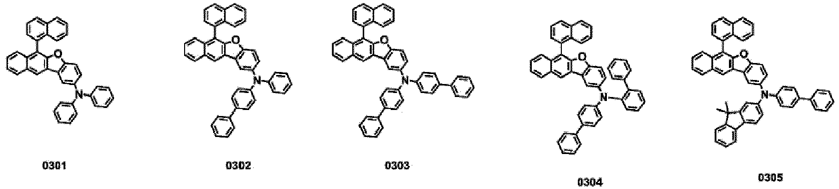

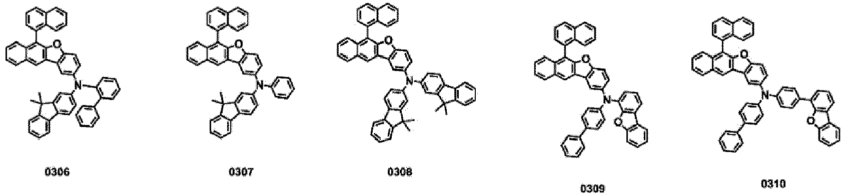

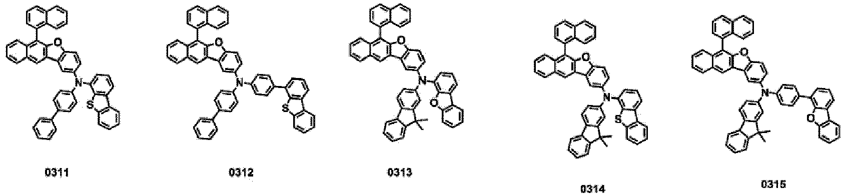

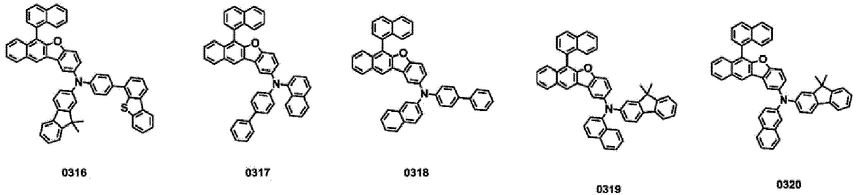

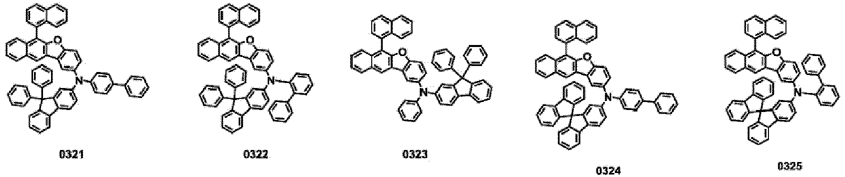

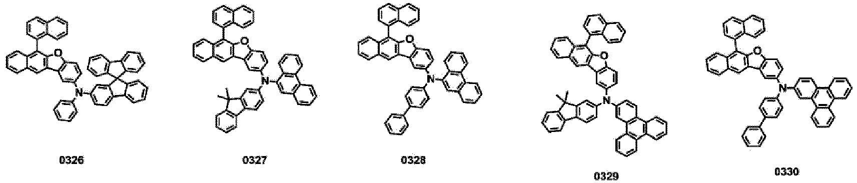

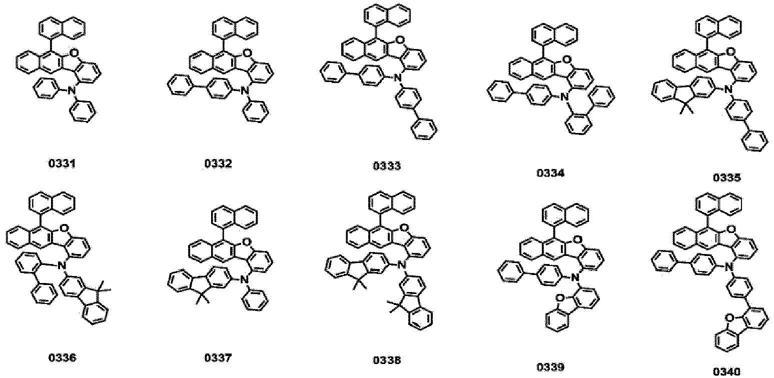

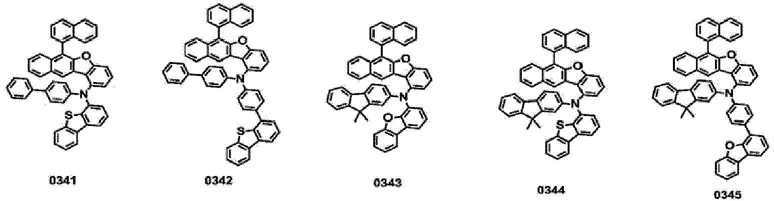

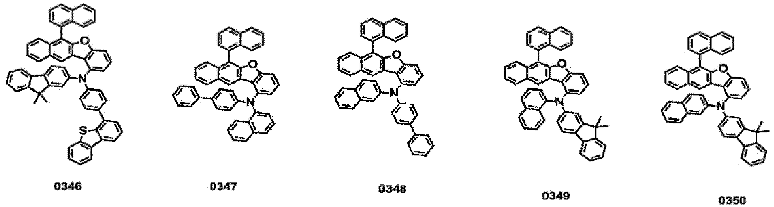

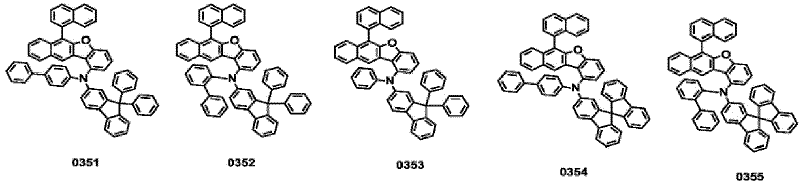

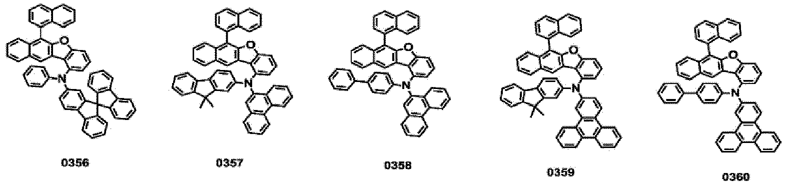

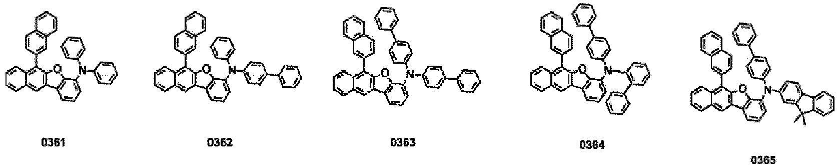

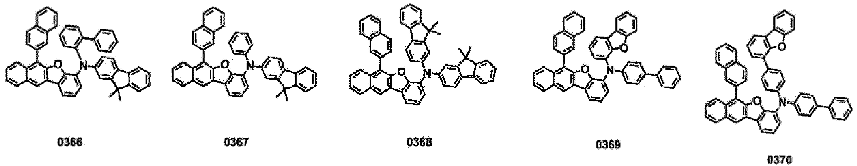

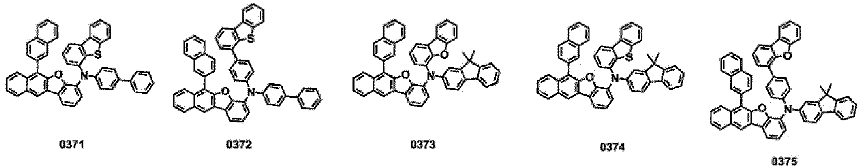

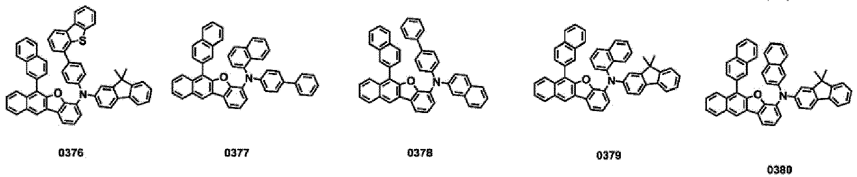

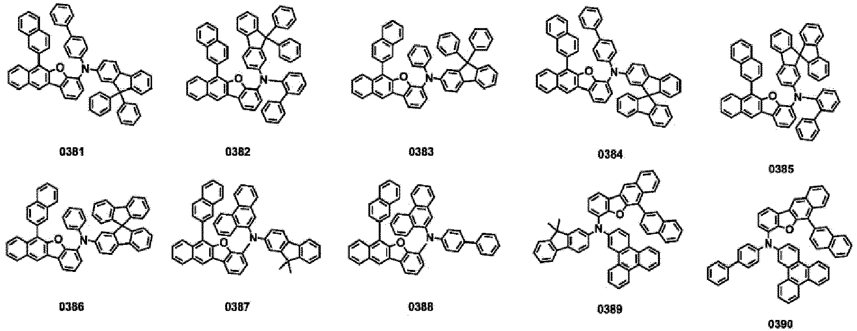

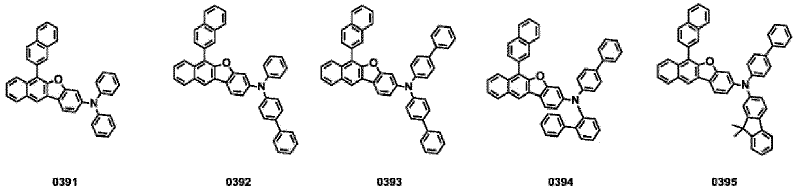

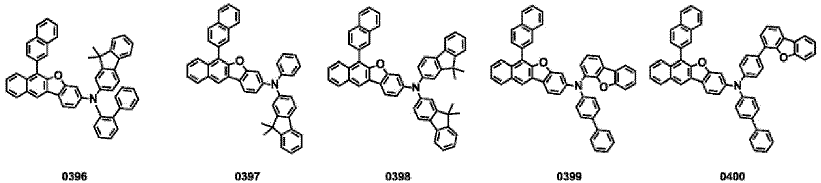

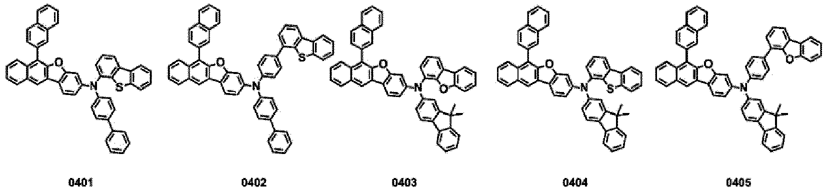

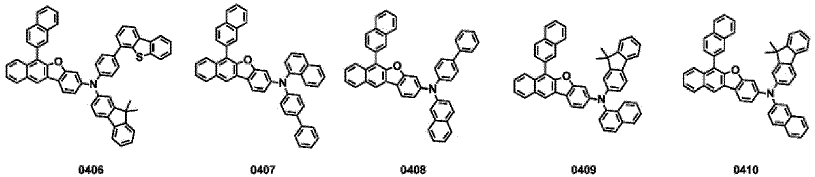

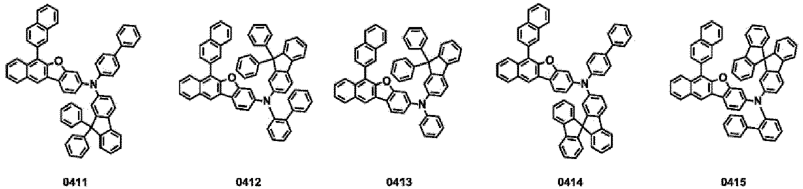

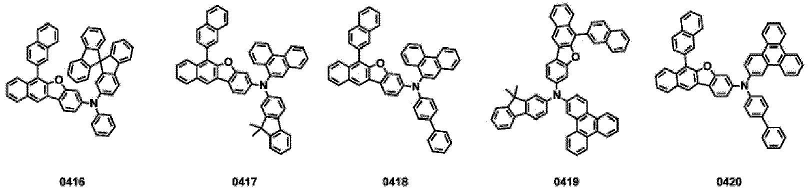

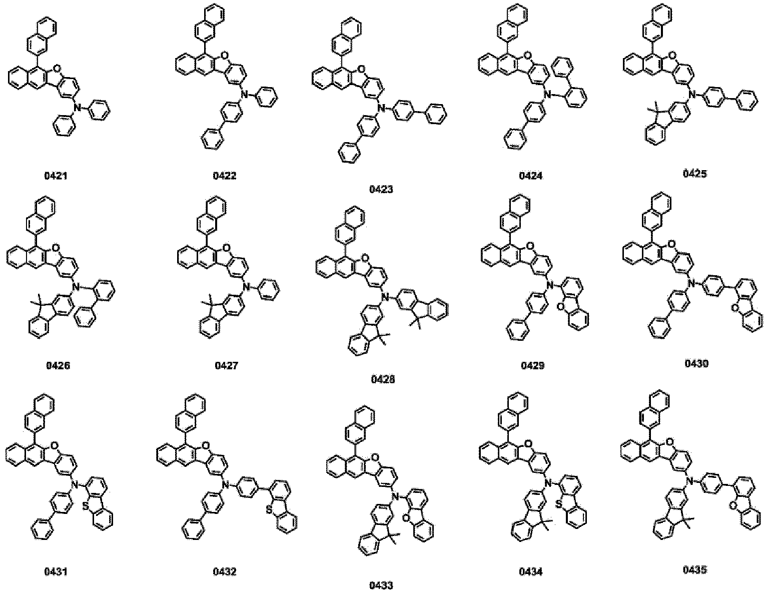

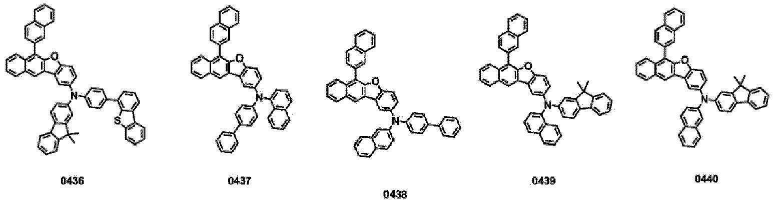

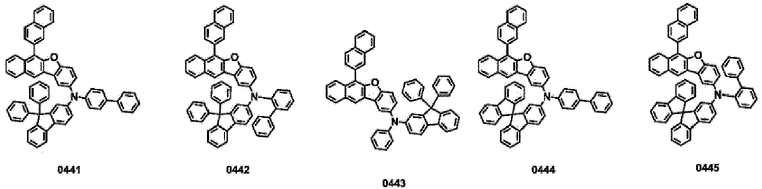

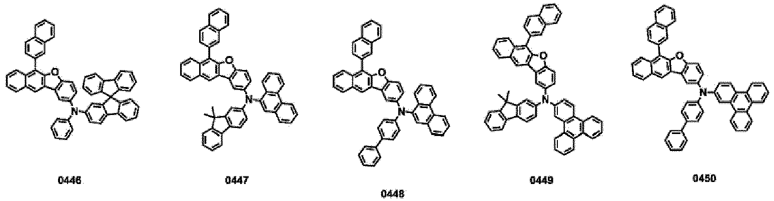

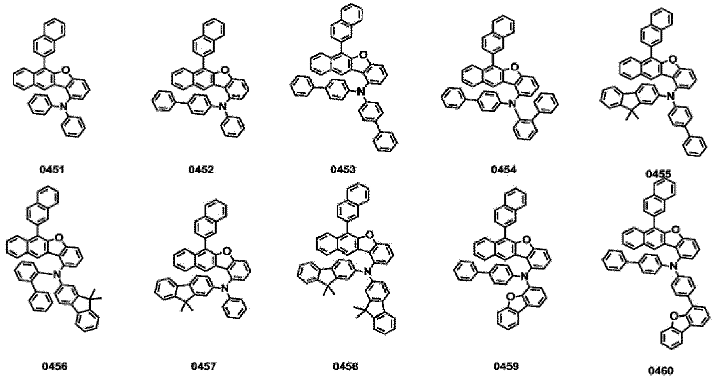

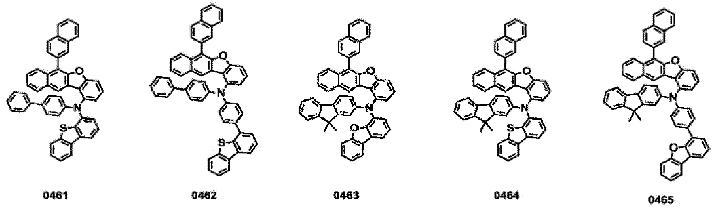

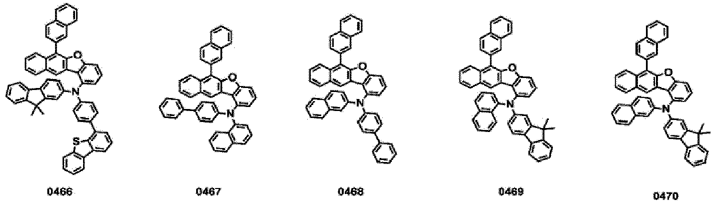

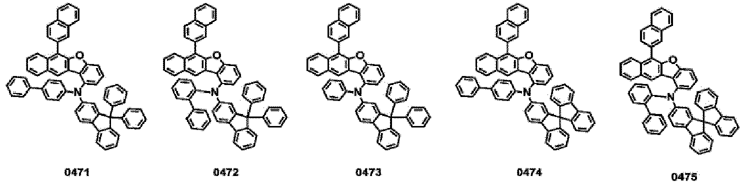

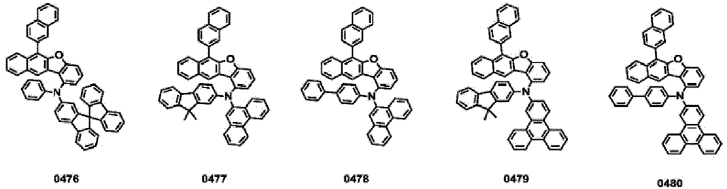

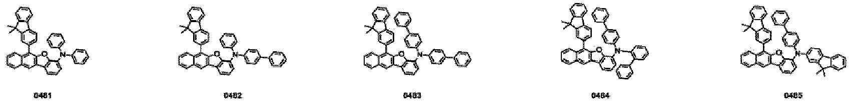

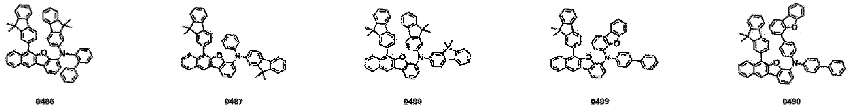

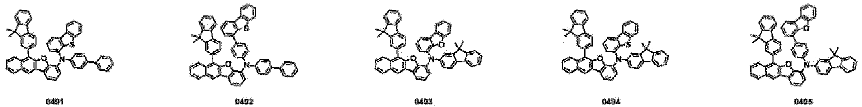

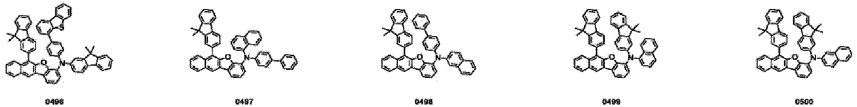

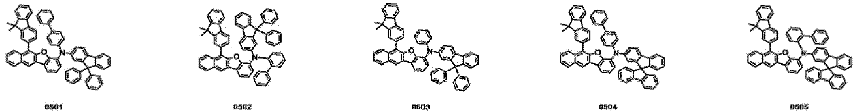

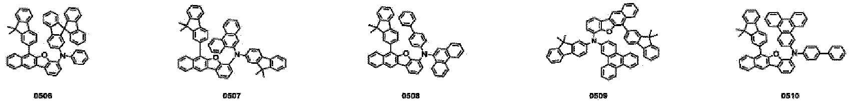

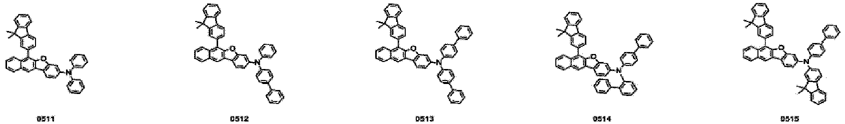

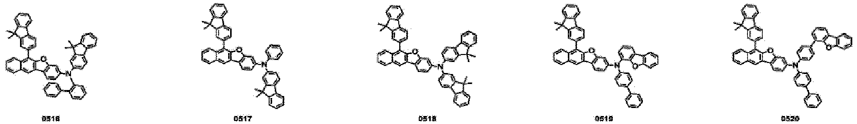

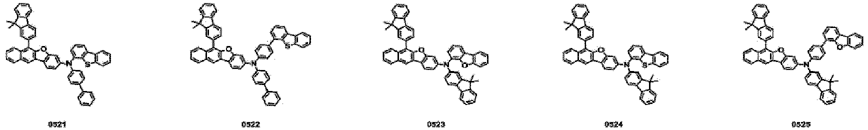

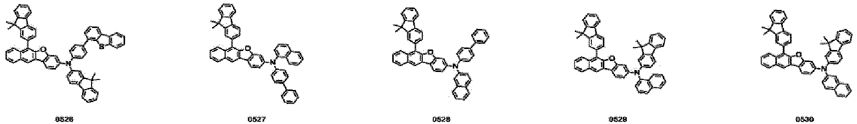

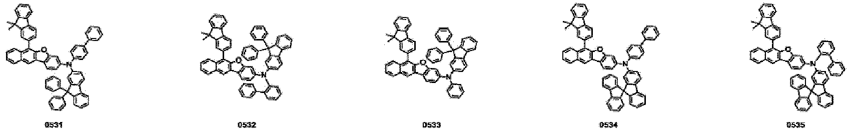

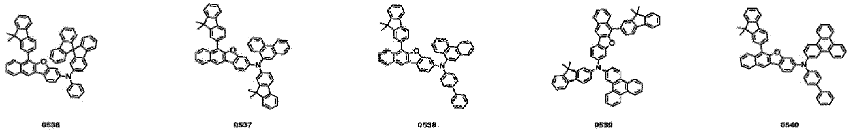

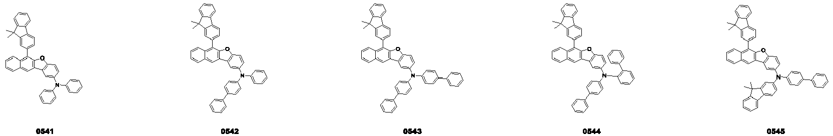

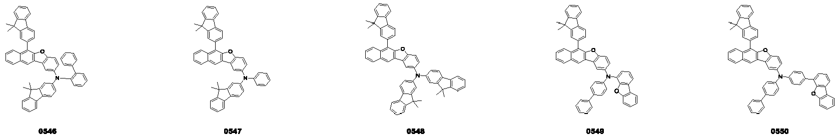

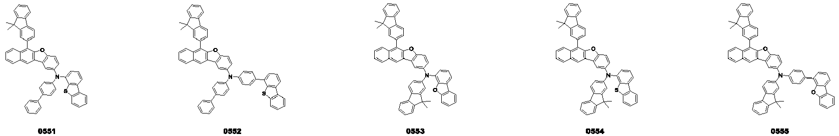

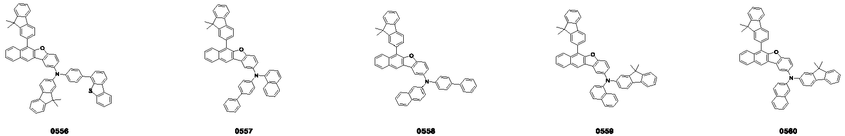

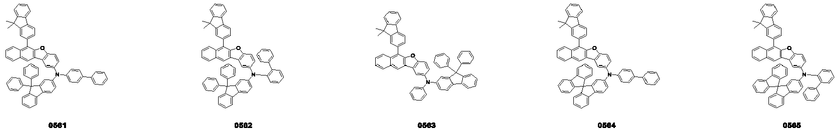

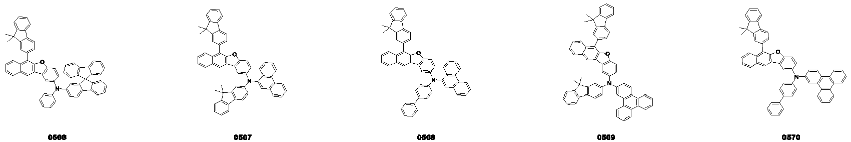

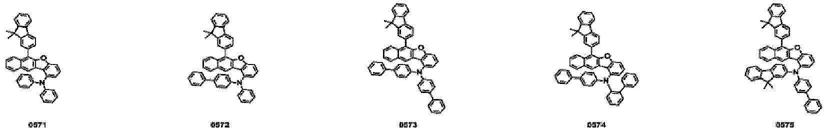

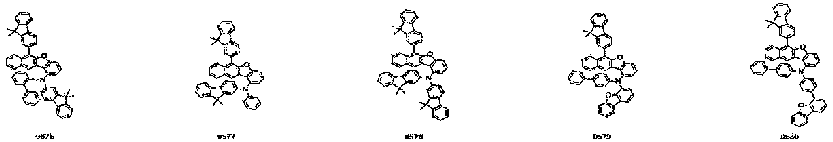

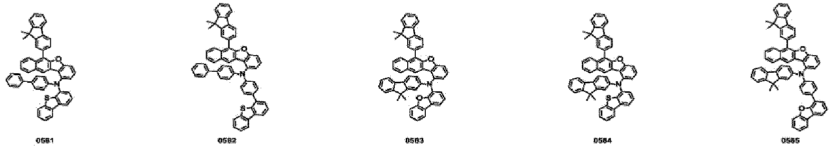

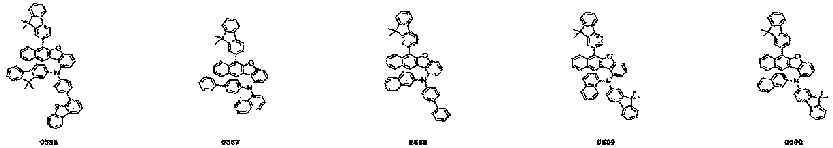

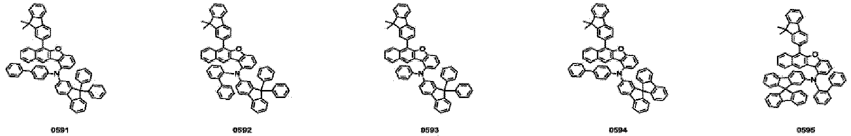

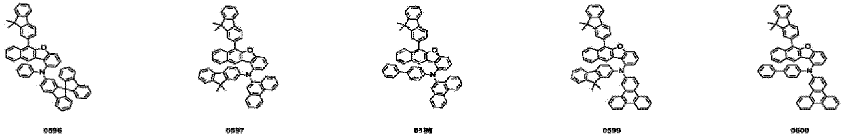

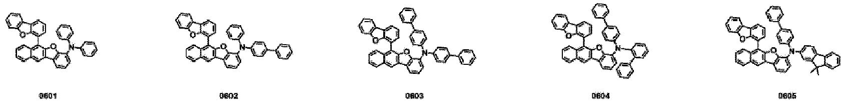

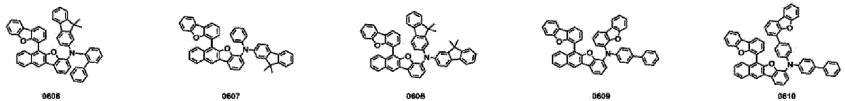

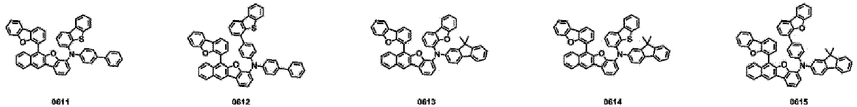

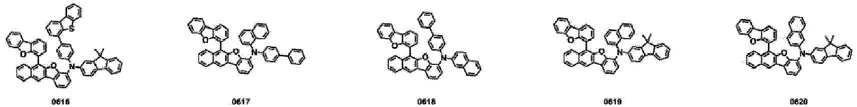

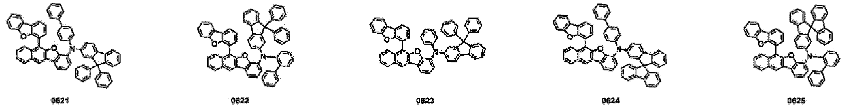

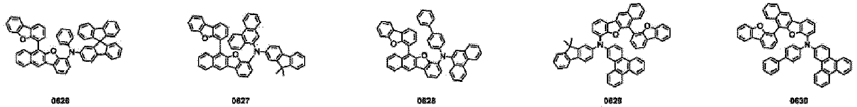

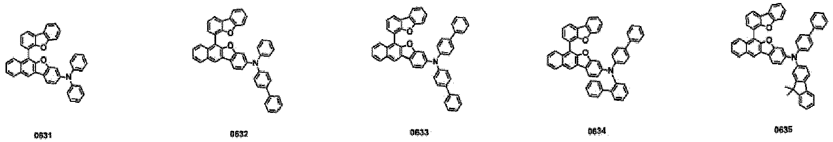

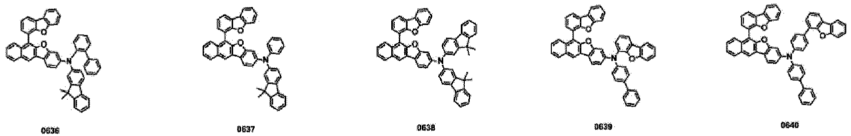

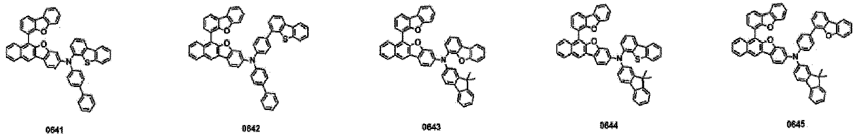

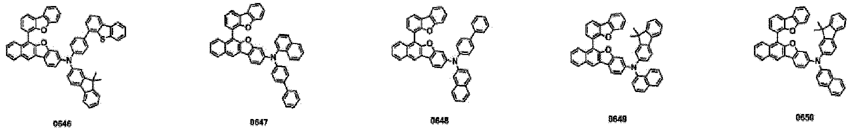

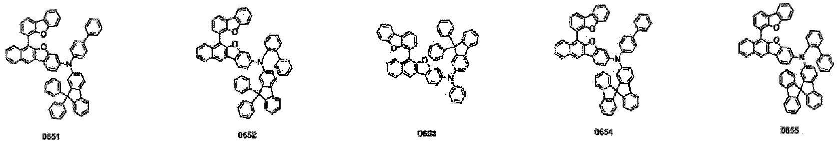

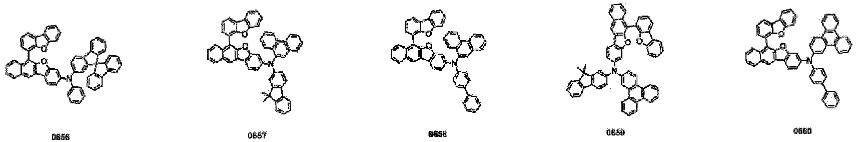

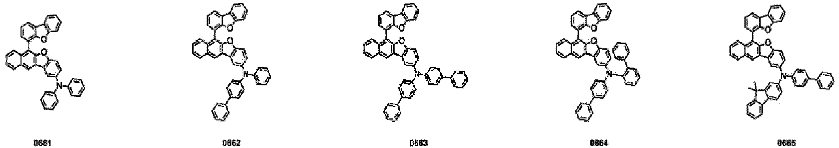

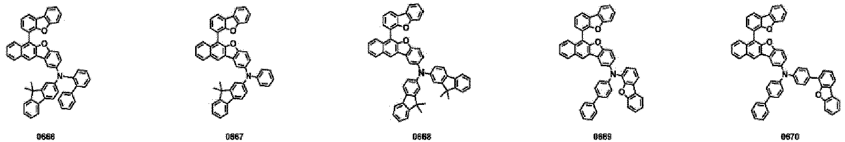

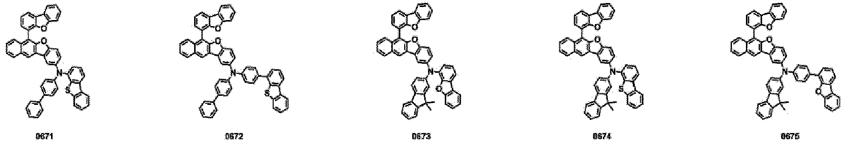

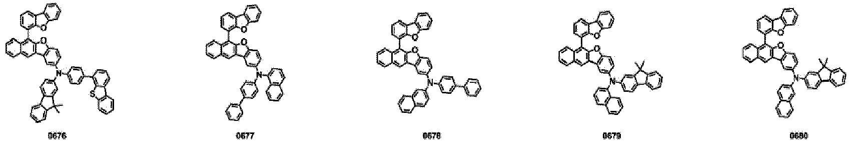

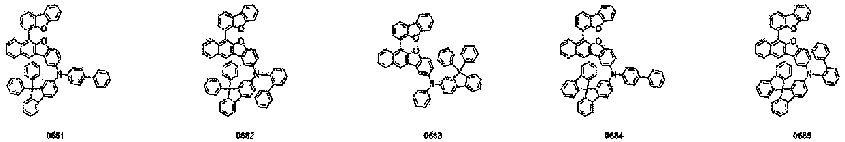

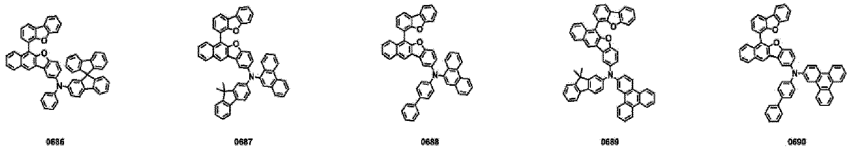

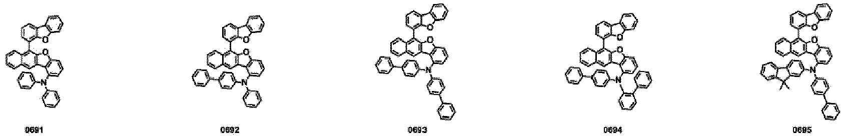

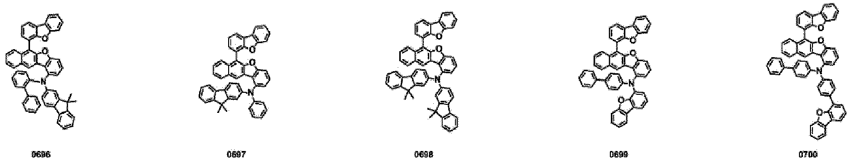

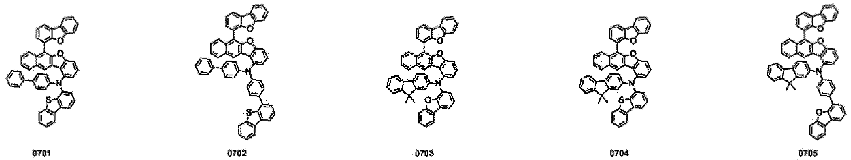

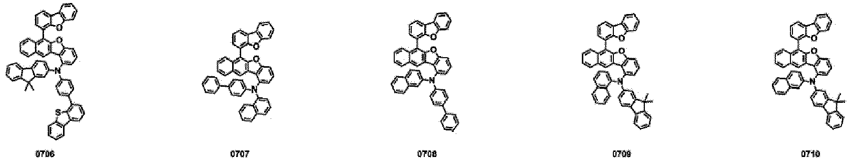

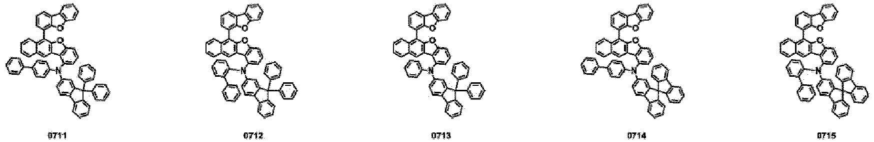

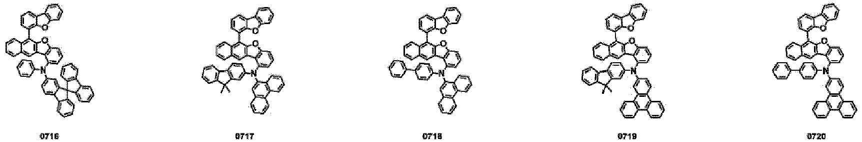

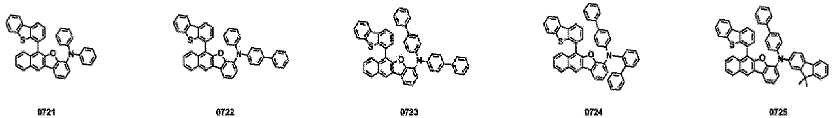

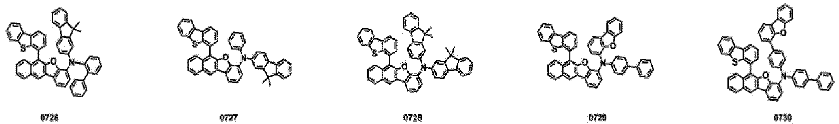

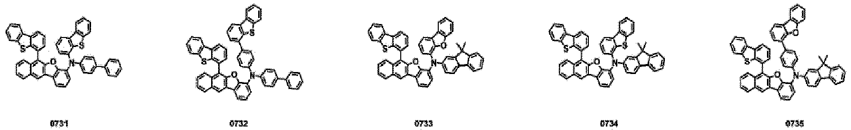

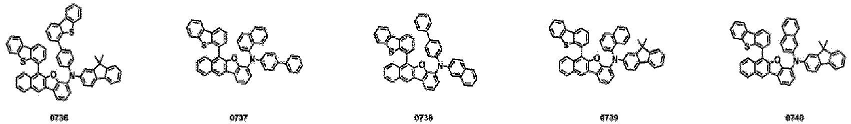

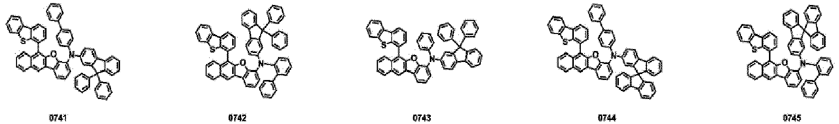

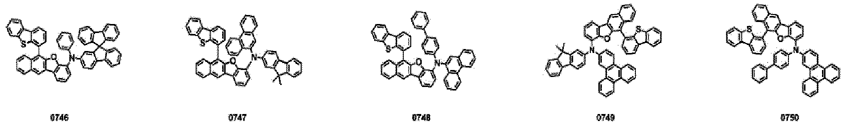

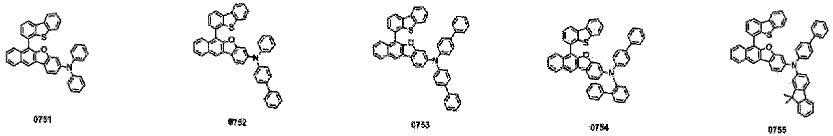

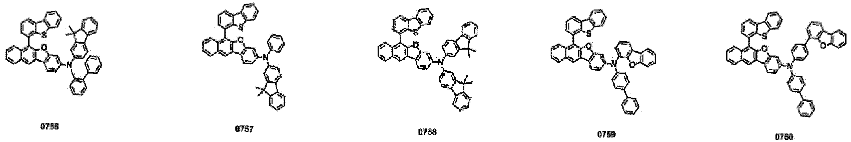

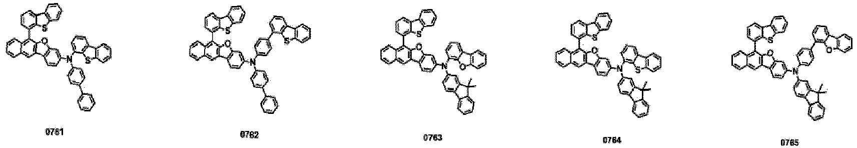

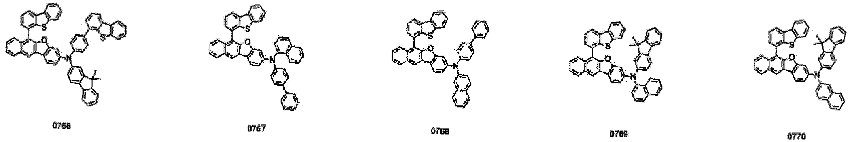

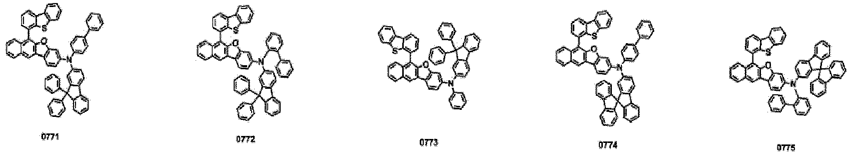

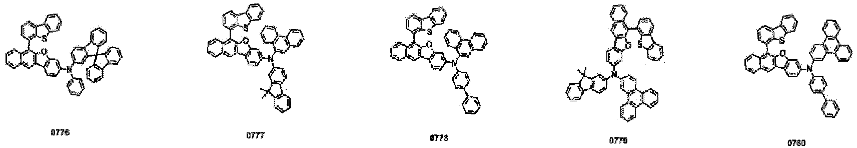

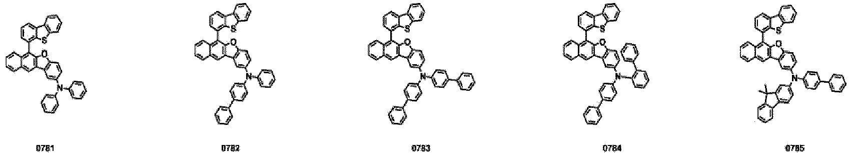

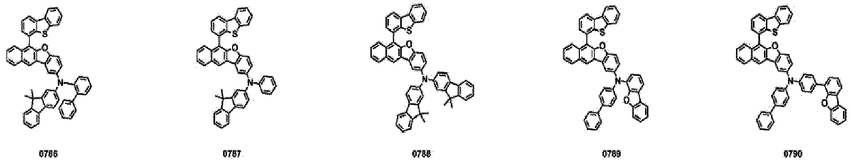

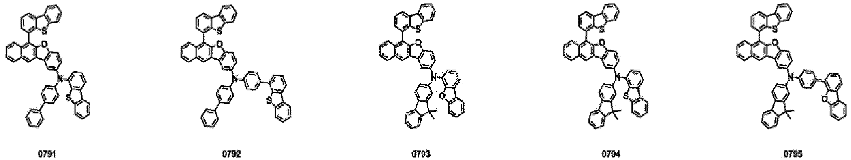

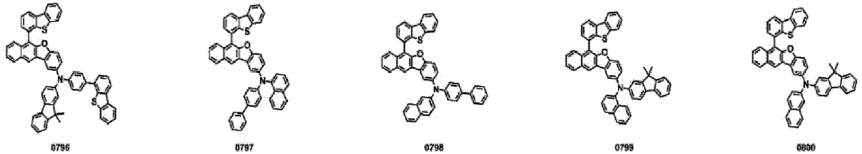

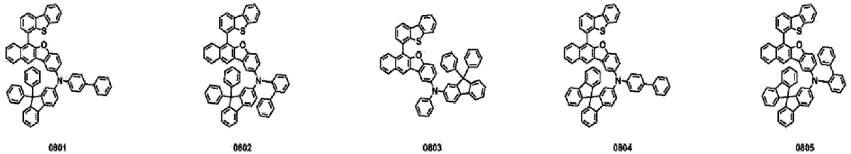

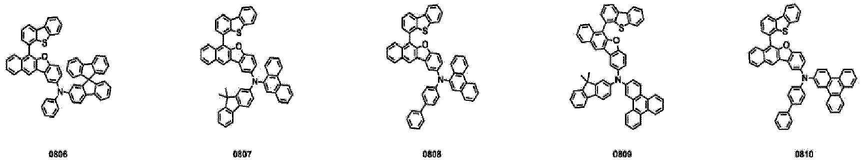

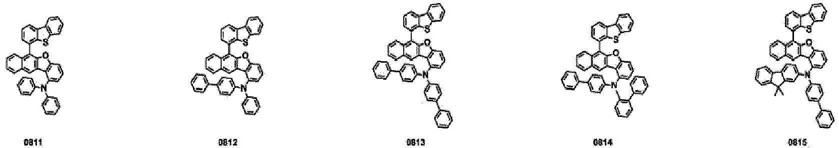

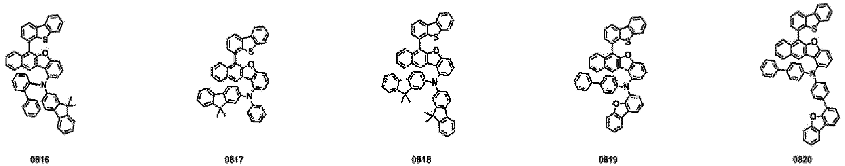

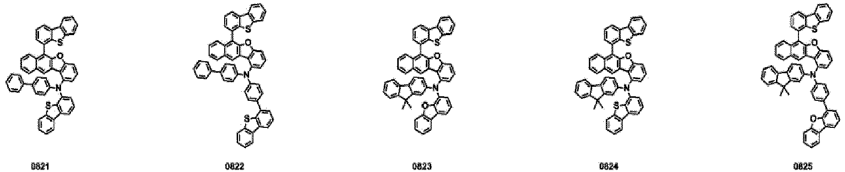

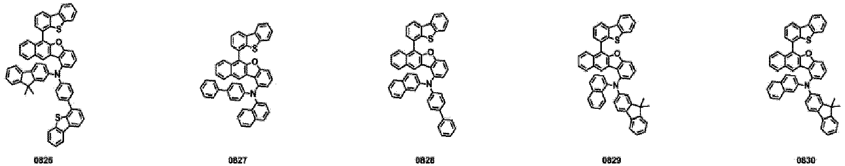

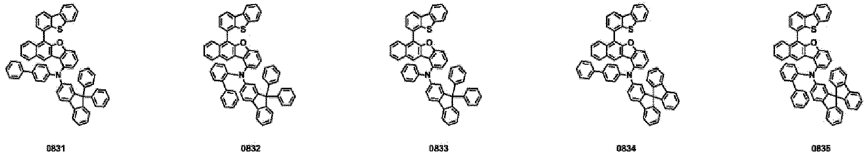

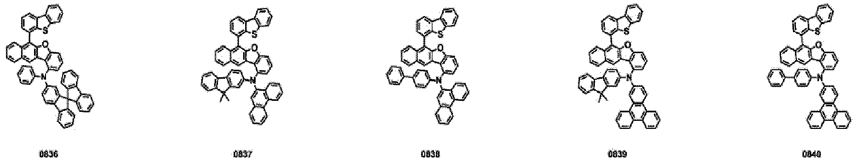

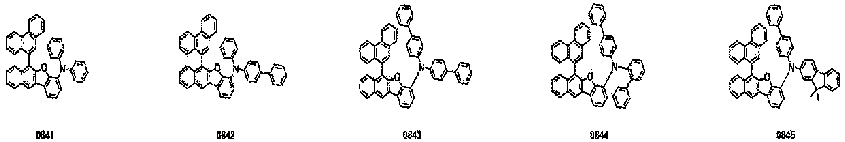

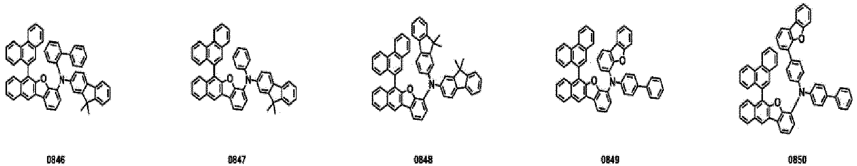

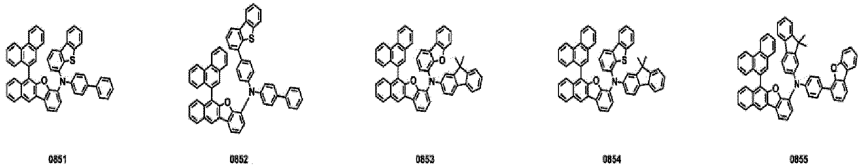

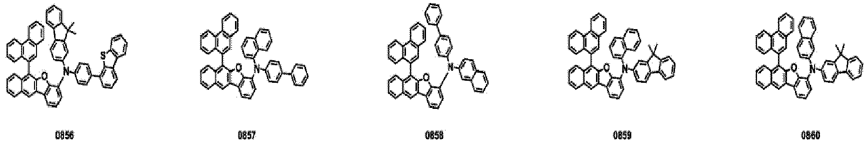

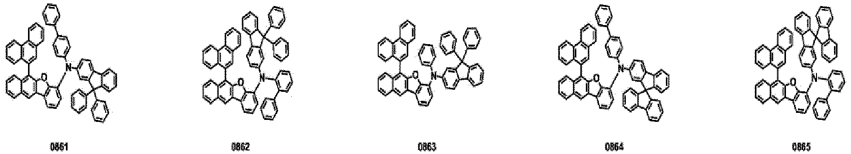

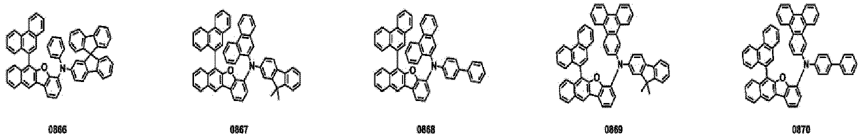

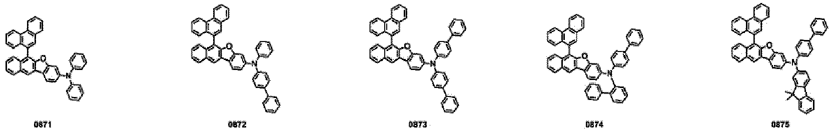

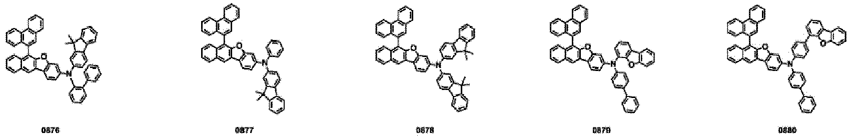

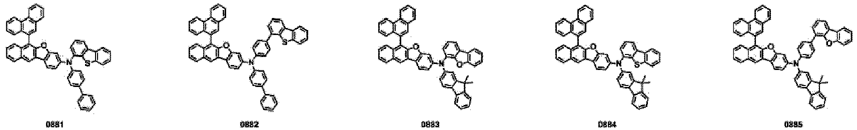

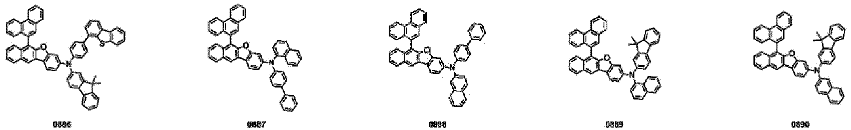

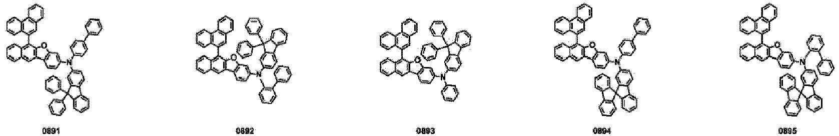

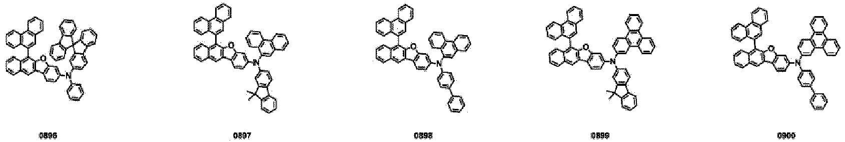

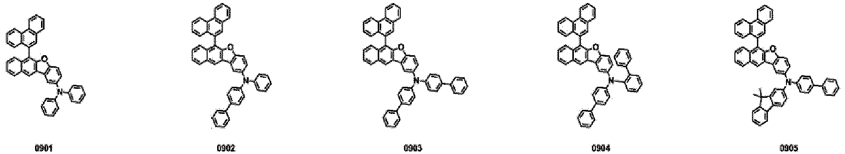

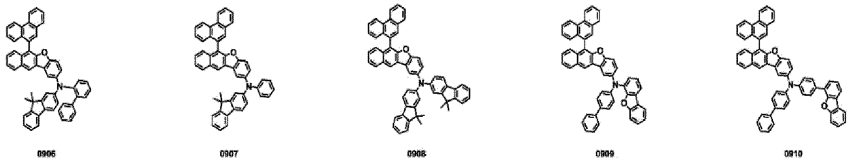

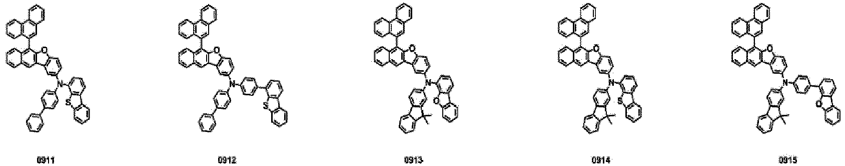

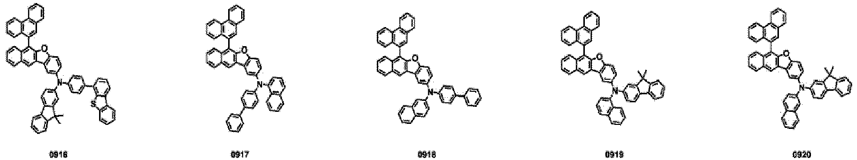

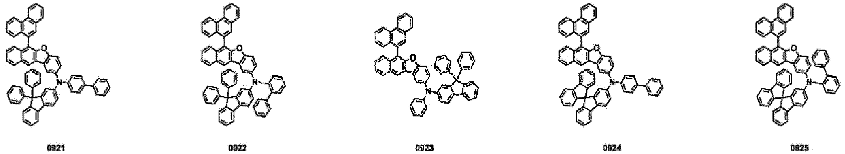

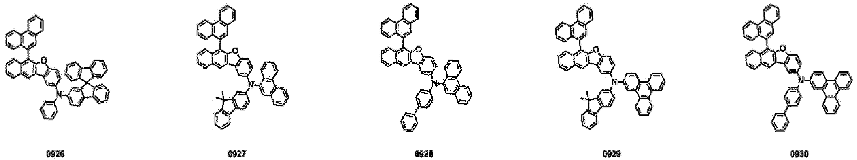

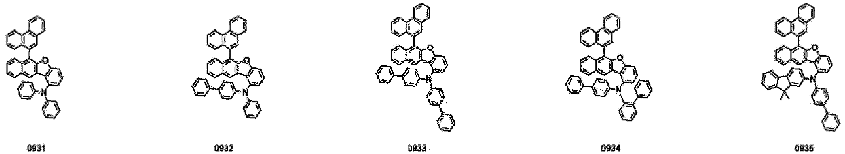

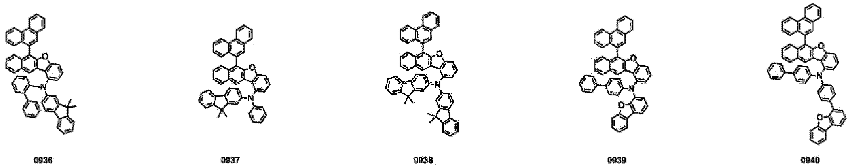

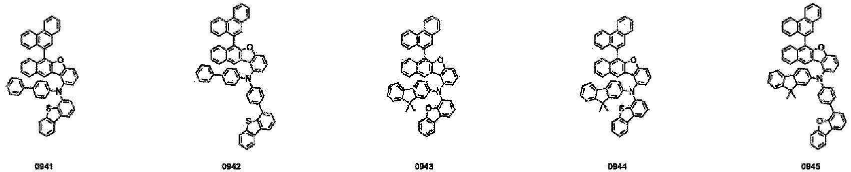

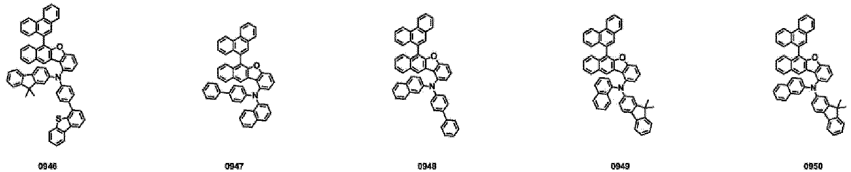

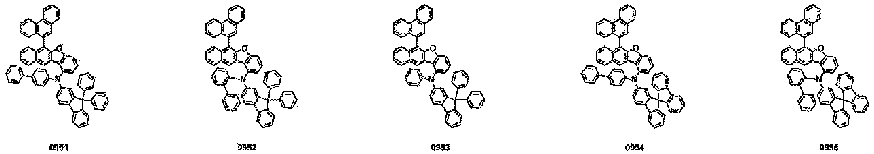

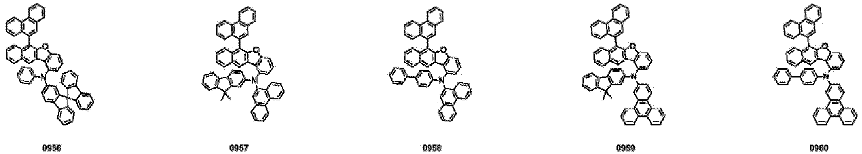

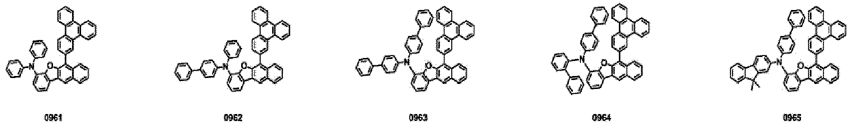

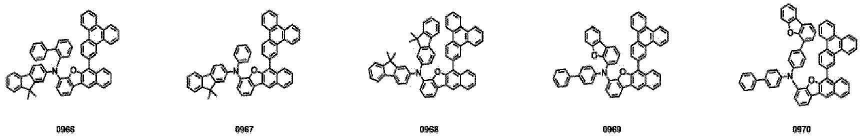

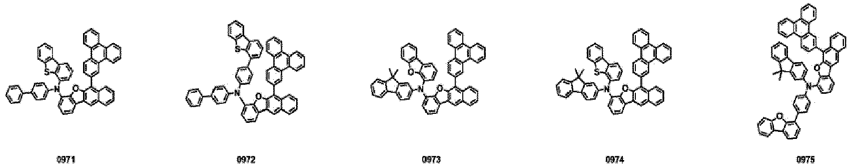

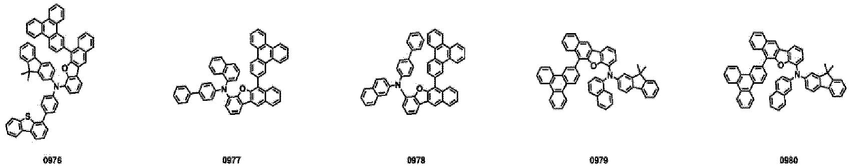

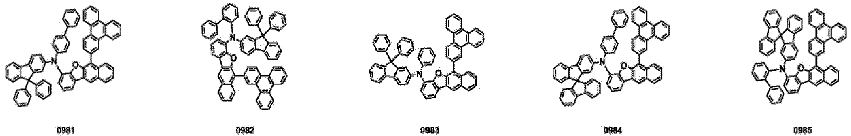

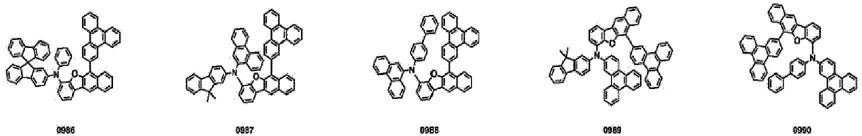

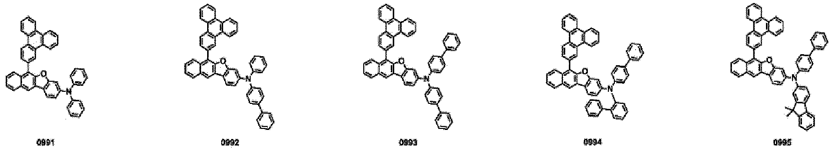

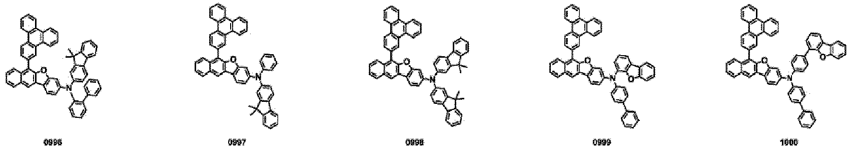

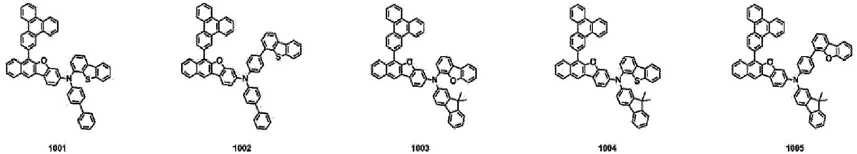

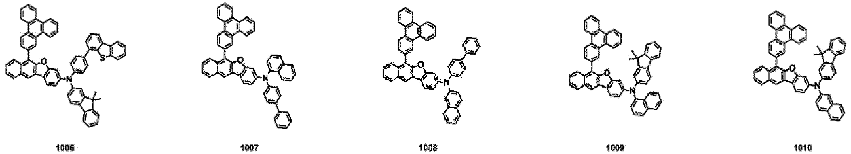

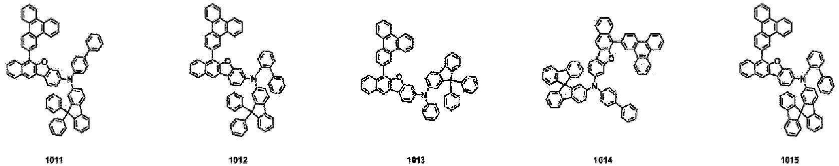

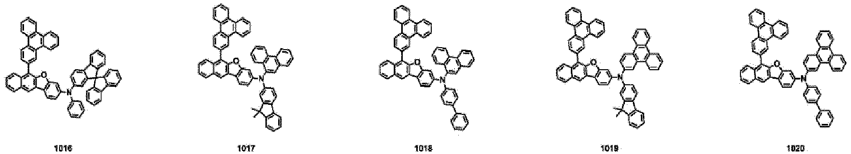

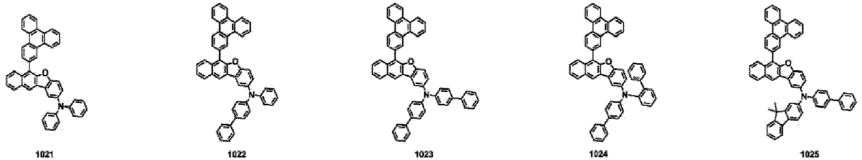

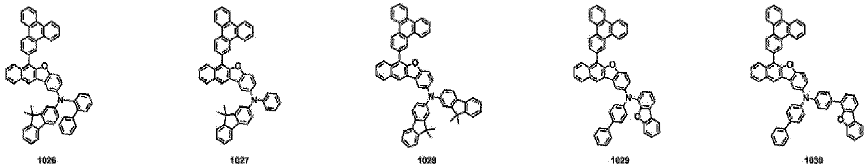

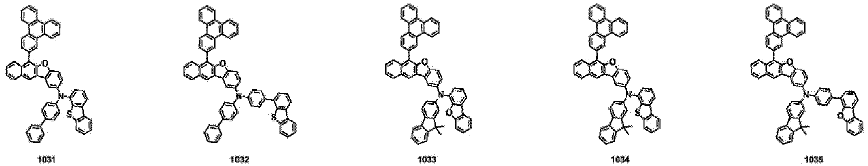

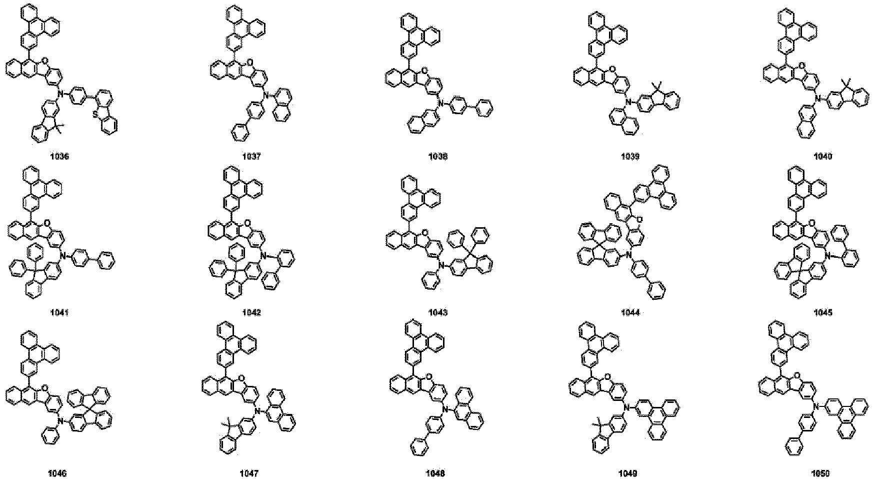

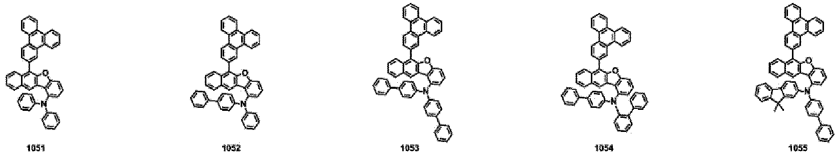

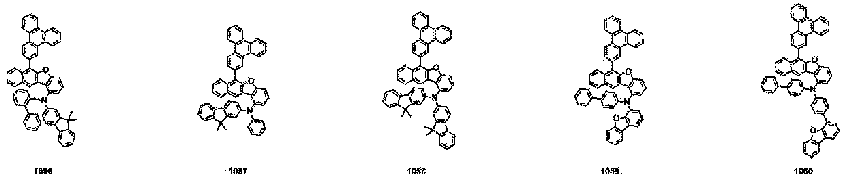

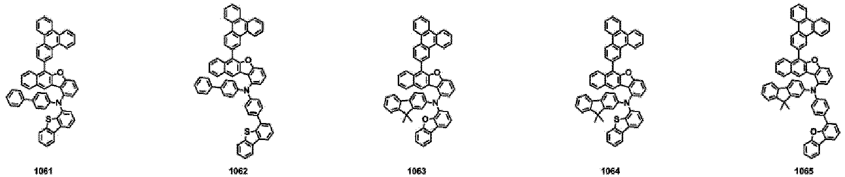

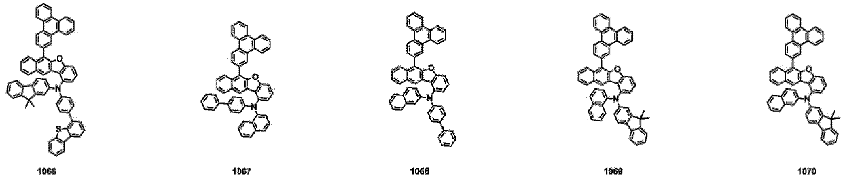

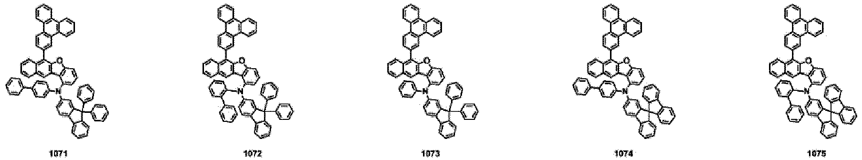

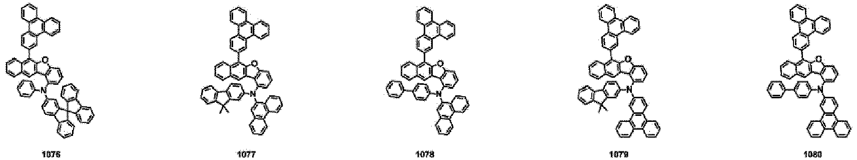

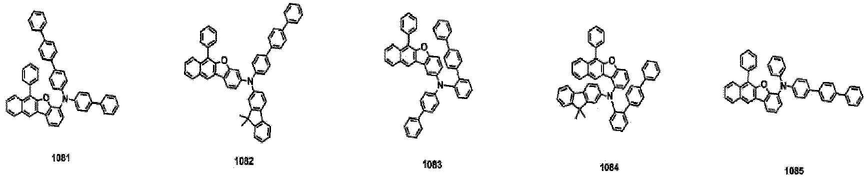

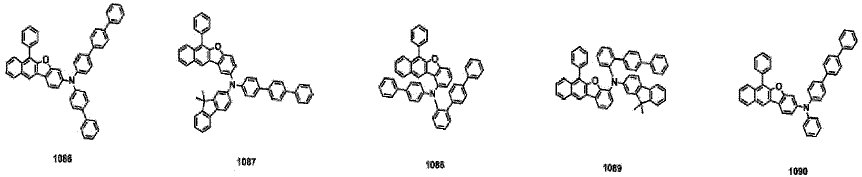

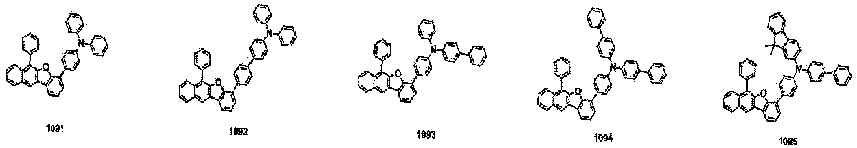

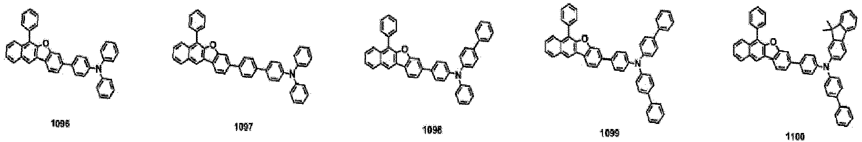

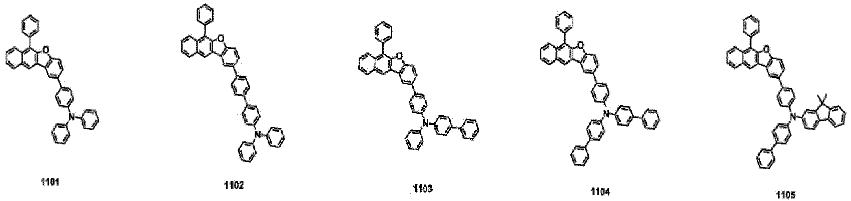

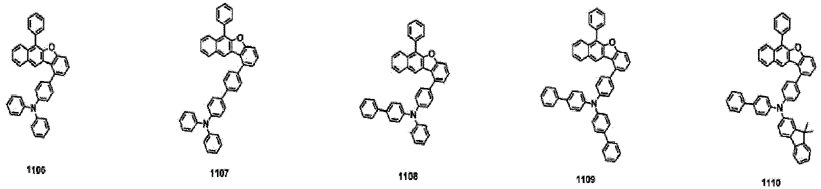

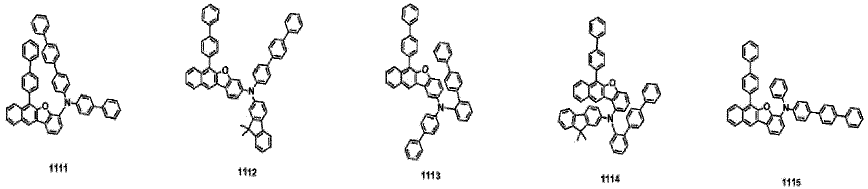

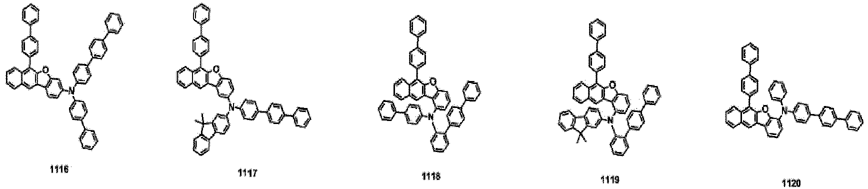

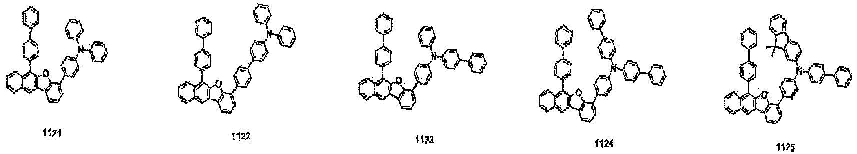

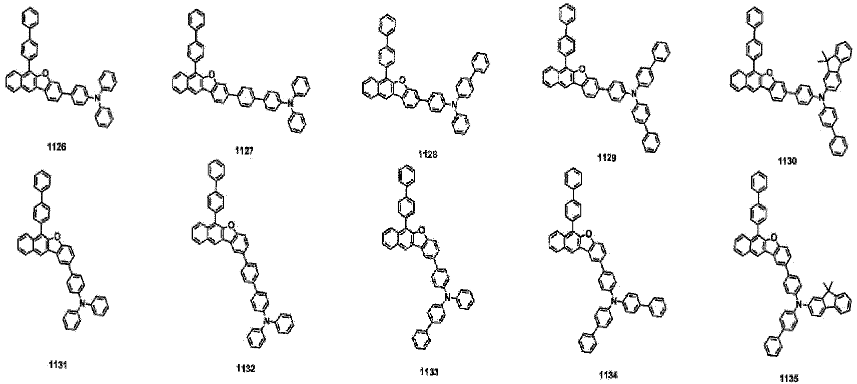

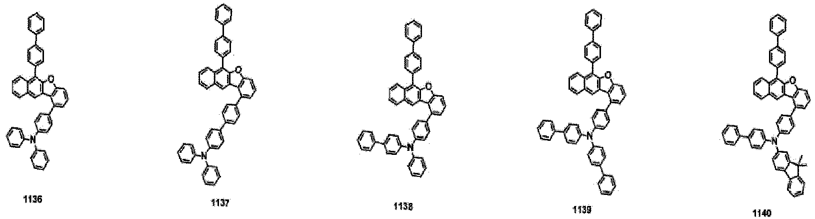

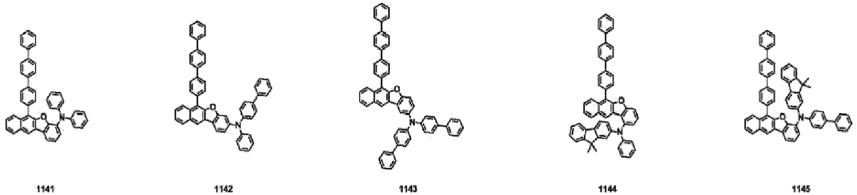

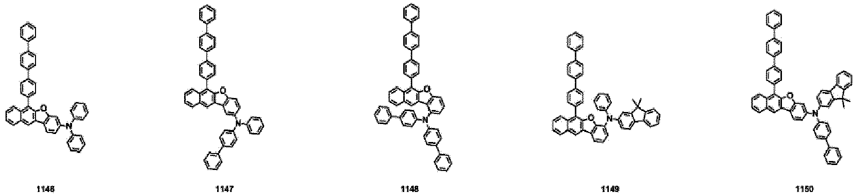

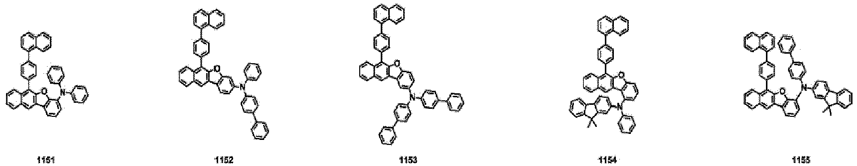

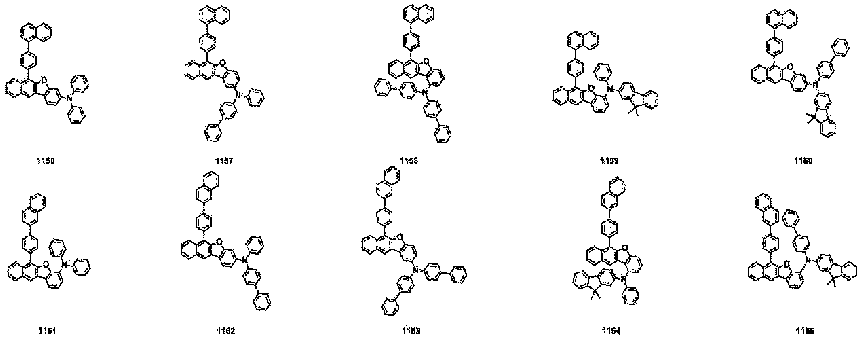

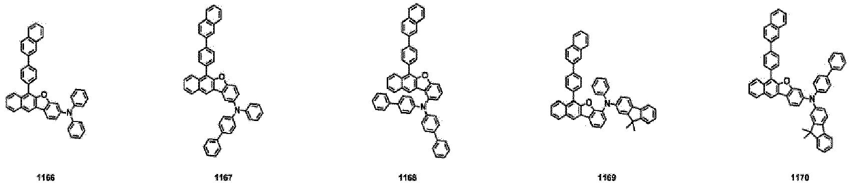

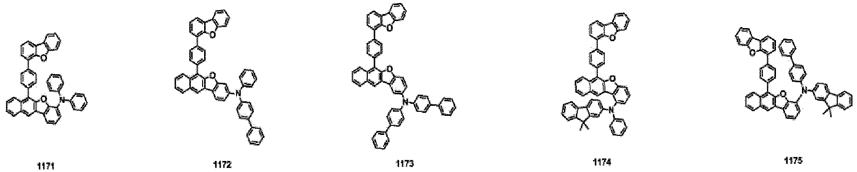

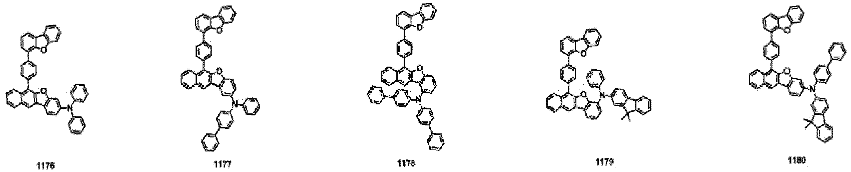

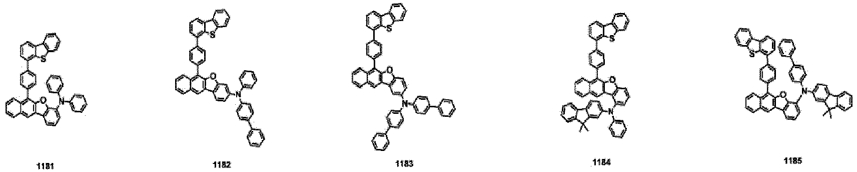

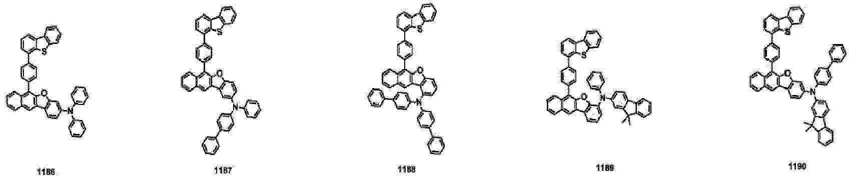

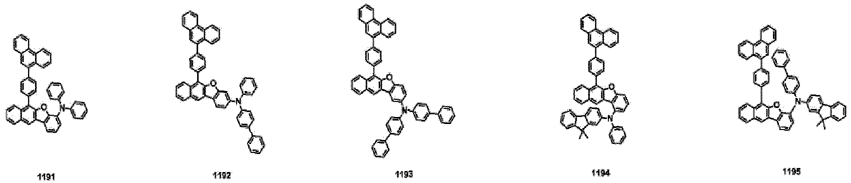

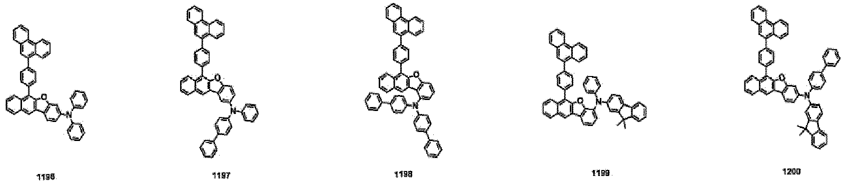

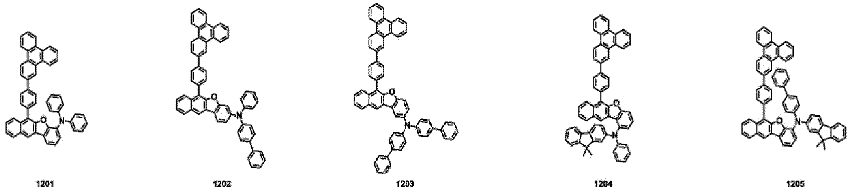

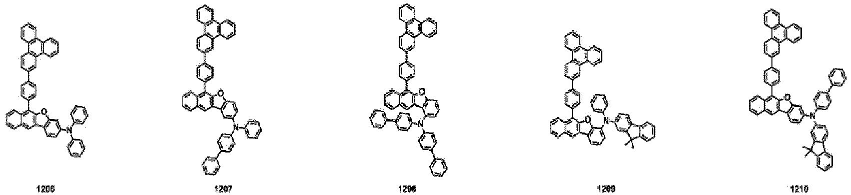

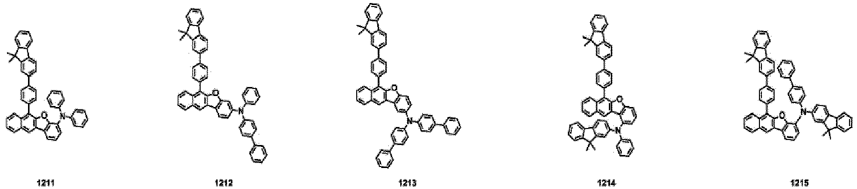

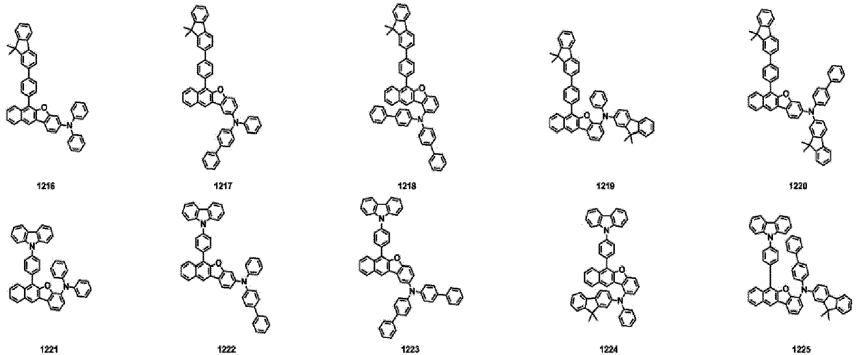

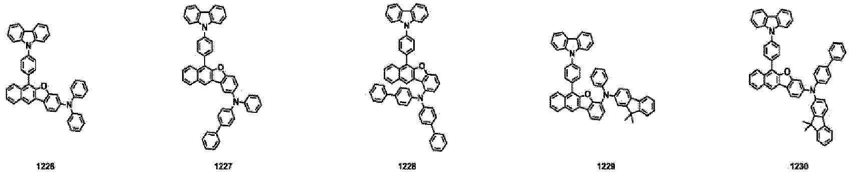

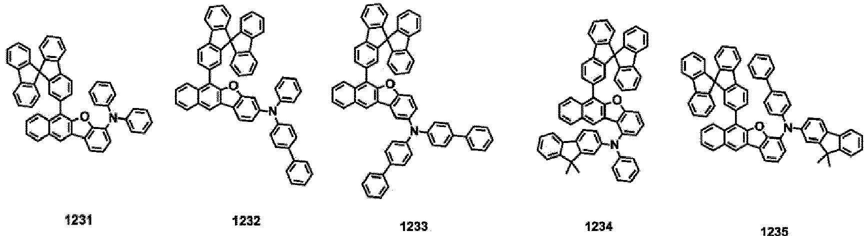

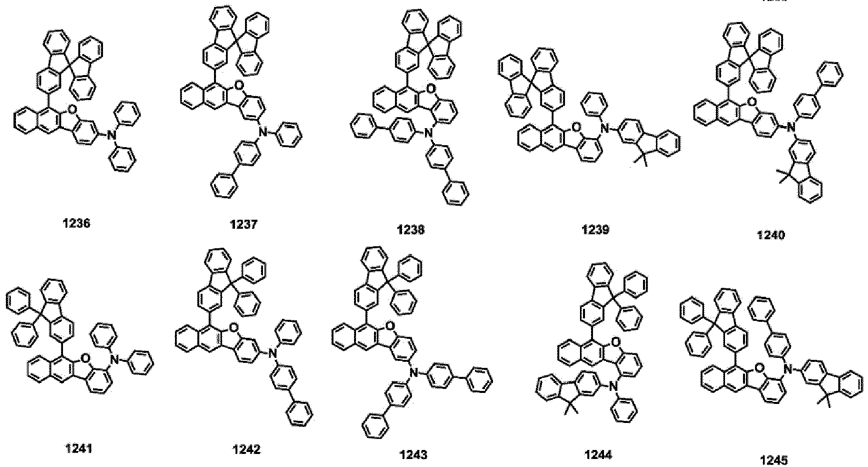

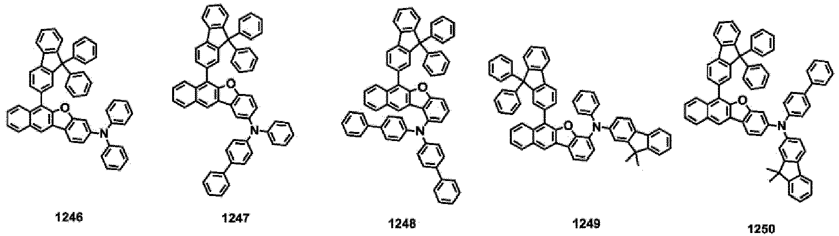

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound according to Chemical Formula 1.

Another embodiment provides an organic light emitting device comprising a first electrode; a second electrode provided opposite to the first electrode; and one or more organic material layers provided between the first electrode and the second electrode, wherein one or more layers of the organic material layers comprise one type of the heterocyclic compound according to Chemical Formula 1.

Another embodiment provides an organic light emitting device comprising a first electrode; a second electrode provided opposite to the first electrode; and one or more organic material layers provided between the first electrode and the second electrode, wherein one or more layers of the organic material layers comprise two types of the heterocyclic compound according to Chemical Formula 1.

Specific details on the heterocyclic compound represented by Chemical Formula 1 are the same as the descriptions provided above.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device. For example, the heterocyclic compound according to Chemical Formula 1 may be included in a host material of a blue light emitting layer of a blue organic light emitting device.

In one embodiment of the present application, the organic light emitting device may be a green organic light emitting device, and the heterocyclic compound according to Chemical Formula 1 may be used as a material of the green organic light emitting device. For example, the heterocyclic compound according to Chemical Formula 1 may be included in a host material of a green light emitting layer of a green organic light emitting device.

In one embodiment of the present application, the organic light emitting device may be a red organic light emitting device, and the heterocyclic compound according to Chemical Formula 1 may be used as a material of the red organic light emitting device. For example, the heterocyclic compound according to Chemical Formula 1 may be included in a host material of a red light emitting layer of a red organic light emitting device.

The organic light emitting device of the present disclosure may be manufactured using common organic light emitting device manufacturing methods and materials except that one or more organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, or may also be formed in a multilayer structure in which two or more organic material layers are laminated. For example, the organic light emitting device according to one embodiment of the present disclosure may have a structure comprising a hole injection layer, a hole transfer layer, a light emitting layer, an electron transfer layer, an electron injection layer and the like as the organic material layer. However, the structure of the organic light emitting device is not limited thereto, and may comprise a smaller number of organic material layers.

In the organic light emitting device of the present disclosure, the organic material layer may comprise a light emitting layer, and the light emitting layer may comprise the heterocyclic compound.

In another organic light emitting device, the organic material layer comprises a light emitting layer, the light emitting layer comprises a host material, and the host material may comprise the heterocyclic compound.

In another embodiment, the organic material layer comprising the heterocyclic compound comprises the heterocyclic compound represented by Chemical Formula 1 as a host, and an iridium-based dopant may be used therewith.

In the organic light emitting device of the present disclosure, the organic material layer comprises an electron injection layer or an electron transfer layer, and the electron transfer layer or the electron injection layer may comprise the heterocyclic compound.

In another organic light emitting device, the organic material layer comprises an electron blocking layer or a hole blocking layer, and the electron blocking layer or the hole blocking layer may comprise the heterocyclic compound.

In the organic light emitting device of the present disclosure, the organic material layer comprises a hole injection layer or a hole transfer layer, and the hole injection layer or the hole transfer layer may comprise the heterocyclic compound.

The organic light emitting device of the present disclosure may further comprise one, two or more layers selected from the group consisting of a light emitting layer, a hole injection layer, a hole transfer layer, an electron injection layer, an electron transfer layer, an electron blocking layer and a hole blocking layer.

FIG. 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

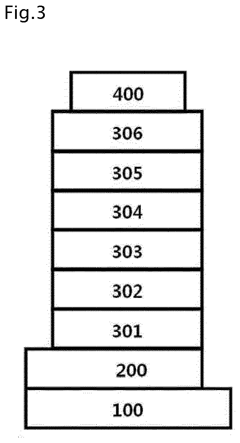

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer comprising the compound of Chemical Formula 1 may further comprise other materials as necessary.

In the organic light emitting device according to one embodiment of the present application, materials other than the heterocyclic compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device including an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Preparation of Compound 0002

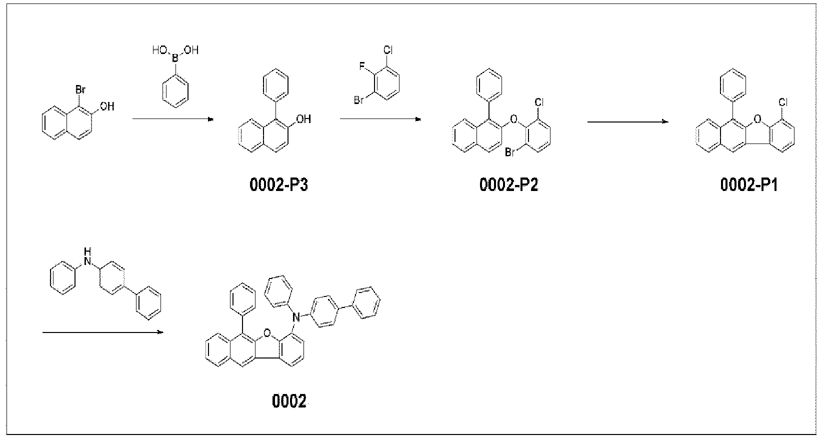

### 1) Preparation of Compound 0002-P3

After dissolving 1-bromonaphthalen-2-ol (100 g, 448.29 mmol) and phenylboronic acid (57.39 g, 470.70 mmol) in toluene (1000 ml), ethanol (200 ml) and distilled water (200 ml), Pd(PPh₃)₄ (10.36 g, 8.97 mmol) and K₂CO₃ (154.90 g, 1120.72 mmol) were introduced thereto, and the result was stirred for 12 hours under reflux. After the reaction was completed, ethyl acetate was introduced to the reaction solution for dissolution, and the result was extracted with distilled water. After drying the organic layer with anhydrous MgSO₄, the solvent was removed using a rotary evaporator, and the result was purified by column chromatography using dichloromethane and hexane as a developing solvent to obtain Compound 0002-P3 (86 g, 87%).

### Preparation of Compound 0002-P2

Compound 0002-P3 (86 g, 390.43 mmol) and 1-bromo-3-chloro-2-fluorobenzene (89.95 g, 429.47 mmol) were dissolved in N,N-dimethylacetamide (1000 ml), heated to 150°C, and, after introducing Cs₂CO₃ (254.42 g, 780.86 mmol) thereto, the result was stirred for 30 minutes under reflux. After the reaction was completed, the result was extracted with dichloromethane and distilled water. After drying the organic layer with anhydrous MgSO₄, the solvent was removed using a rotary evaporator, and the result was purified by column chromatography using dichloromethane and hexane as a developing solvent to obtain Compound 0002-P2 (134 g, 84%).

### Preparation of Compound 0002-P1

After dissolving Compound 0002-P2 (134 g, 327.07 mmol) in 1-methyl-2-pyrrolidinone (1000 ml), Pd(OAc)₂ (3.67 g, 16.35 mmol), PPh₃ (8.58 g, 32.71 mmol) and Cs₂CO₃ (213.13 g, 654.14 mmol) were introduced thereto, and the result was stirred for 12 hours under reflux. After the reaction was completed, the result was extracted with dichloromethane and distilled water. After drying the organic layer with anhydrous MgSO₄, the solvent was removed using a rotary evaporator, and the result was purified by column chromatography using dichloromethane and hexane as a developing solvent to obtain Compound 0002-P1 (79 g, 73%).

### Preparation of Compound 0002

After dissolving Compound 0002-P1 (10 g, 30.41 mmol) and N-phenyl-[1,1'-biphenyl]-4-amine (7.83 g, 31.94 mmol) in xylene (100 ml), Pd₂(dba)₃ (1.39 g, 1.52 mmol), P(t-Bu)₃ (1.42 ml, 3.04 mmol) and t-BuONa (7.31 g, 76.04 mmol) were introduced thereto, and the result was stirred for 3 hours under reflux. After the reaction was completed, MC was introduced to the reaction solution for dissolution, and the result was extracted with distilled water. After drying the organic layer with anhydrous MgSO₄, the solvent was removed using a rotary evaporator, and the result was purified by column chromatography using dichloromethane and hexane as a developing solvent to obtain Compound 0002 (13 g, 79%) .

Target compounds of the following Table 1 were prepared in the same manner as in Preparation Example 1 except that Compound A was used instead of phenylboronic acid, Compound B was used instead of 1-bromo-3-chloro-2-fluorobenzene, and Compound C was used instead of N-phenyl-[1,1'-biphenyl]-4-amine.

**[Table 1]**

| **Compound Number** | **Compound A** | **Compound B** | **Compound C** | **Target Compound** | **Yield** |
|---|---|---|---|---|---|
| **0003** | 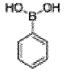 | 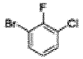 | 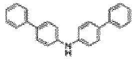 | 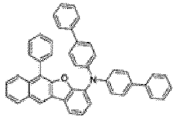 | **66%** |
| **0005** | 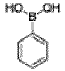 | 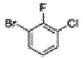 | 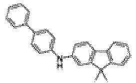 | 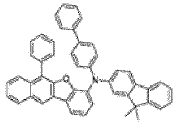 | **71%** |
| **0015** | 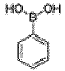 | 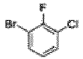 | 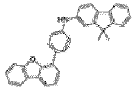 | 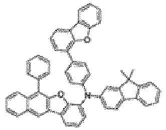 | **78%** |
| **0023** | 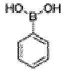 | 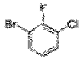 | 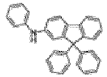 | 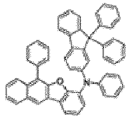 | **65%** |
| **0033** | 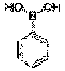 | 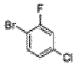 | 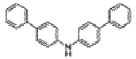 | 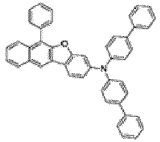 | **74%** |
| **0034** | 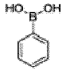 | 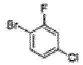 | 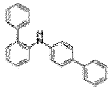 | 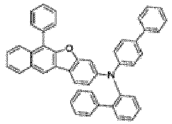 | **79%** |
| **0038** | 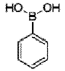 | 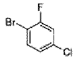 | 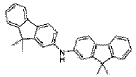 | 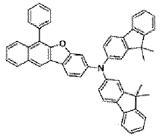 | **80%** |
| **0063** | 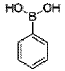 | 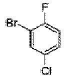 | 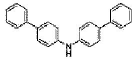 | 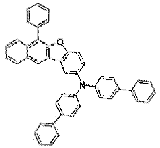 | **73%** |
| **0065** | 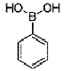 | 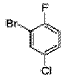 | 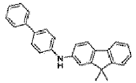 | 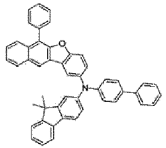 | **73%** |
| **0066** | 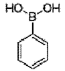 | 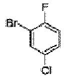 | 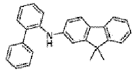 | 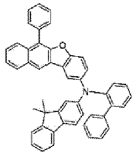 | **73%** |
| **0077** | 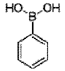 | 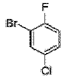 | 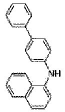 | 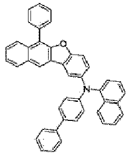 | **77%** |
| **0092** | 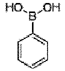 | 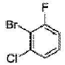 | 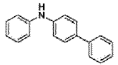 | 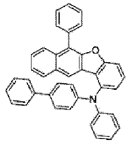 | **67%** |
| **0093** | 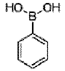 | 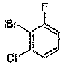 | 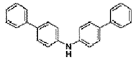 | 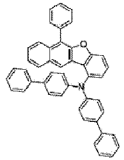 | **80%** |
| **0095** | 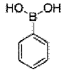 | 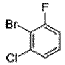 | 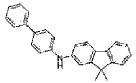 | 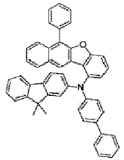 | **71%** |
| **0108** | 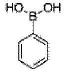 | 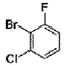 | 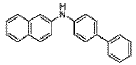 | 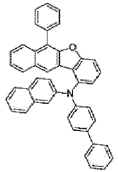 | **75%** |
| **0110** | 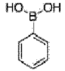 | 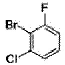 | 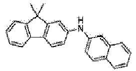 | 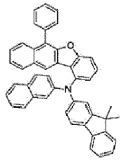 | **82%** |
| **0114** | 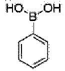 | 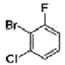 | 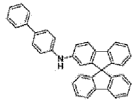 | 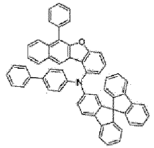 | **61%** |
| **0124** | 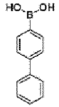 | 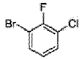 | 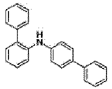 | 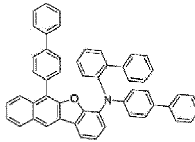 | **70%** |
| **0126** | 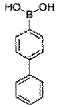 | 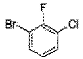 | 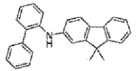 | 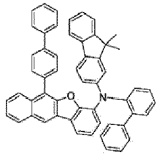 | **75%** |
| **0159** | 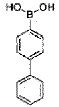 | 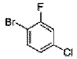 | 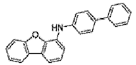 | 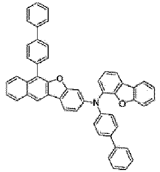 | **76%** |
| **0169** | 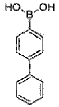 | 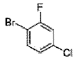 | 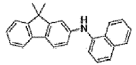 | 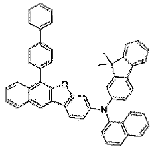 | **81%** |
| **0200** | 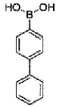 | 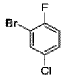 | 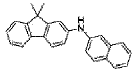 | 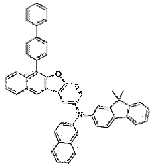 | **71%** |
| **0204** | 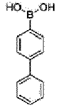 | 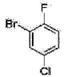 | 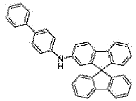 | 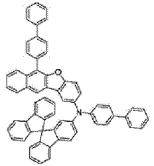 | **74%** |
| **0216** | 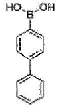 | 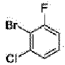 | 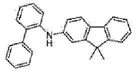 | 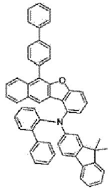 | **79%** |
| **0219** | 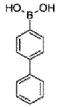 | 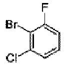 | 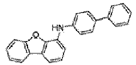 | 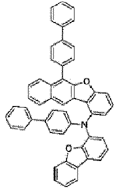 | **73%** |
| **0228** | 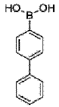 | 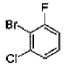 | 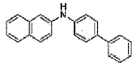 | 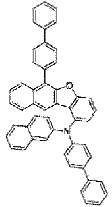 | **83%** |
| **0243** | 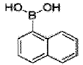 | 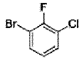 | 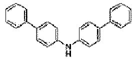 | 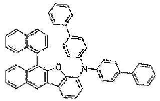 | **77%** |
| **0264** | 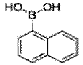 | 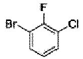 | 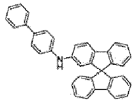 | 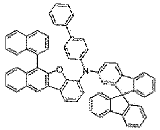 | **70%** |
| **0276** | 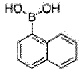 | 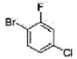 | 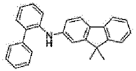 | 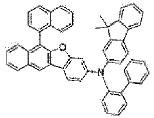 | **76%** |
| **0292** | 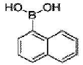 | 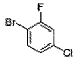 | 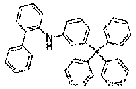 | 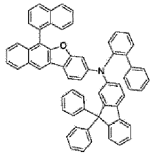 | **75%** |
| **0315** | 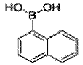 | 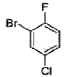 | 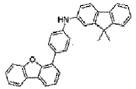 | 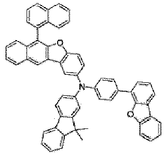 | **71%** |
| **0320** | 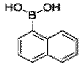 | 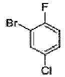 | 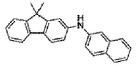 | 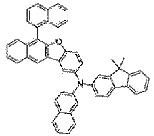 | **78%** |
| **0333** | 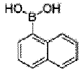 | 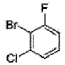 | 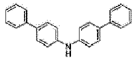 | 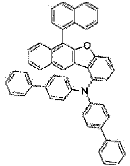 | **72%** |
| **0334** | 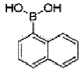 | 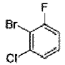 | 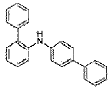 | 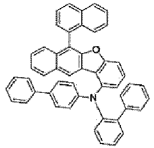 | **67%** |
| **0336** | 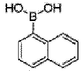 | 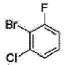 | 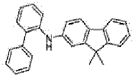 | 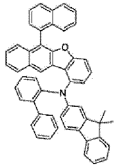 | **68%** |
| **0355** | 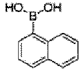 | 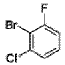 | 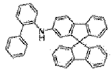 | 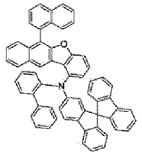 | **71%** |
| **0365** | 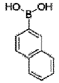 | 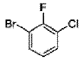 | 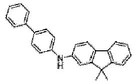 | 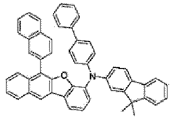 | **76%** |
| **0380** | 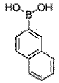 | 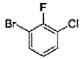 | 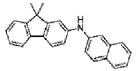 | 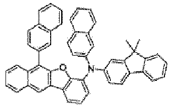 | **83%** |
| **0390** | 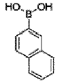 | 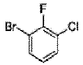 | 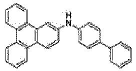 | 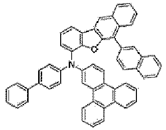 | **68%** |
| **0399** | 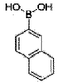 | 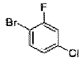 | 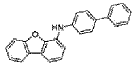 | 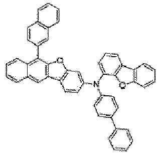 | **75%** |
| **0421** | 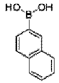 | 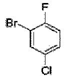 | 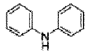 | 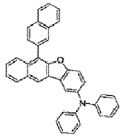 | **74%** |
| **0425** | 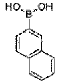 | 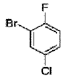 | 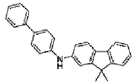 | 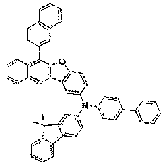 | **81%** |
| **0452** | 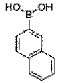 | 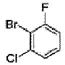 | 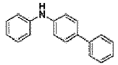 | 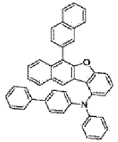 | **77%** |
| **0464** | 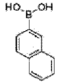 | 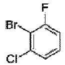 | 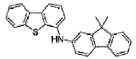 | 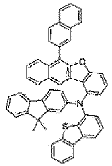 | **78%** |
| **0466** | 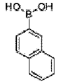 | 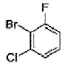 | 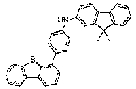 | 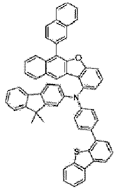 | **65%** |
| **0482** | 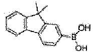 | 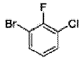 | 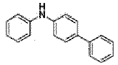 | 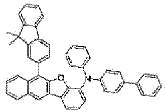 | **71%** |
| **0485** | 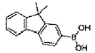 | 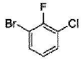 | 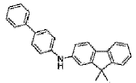 | 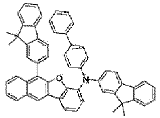 | **79%** |
| **0498** | 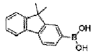 | 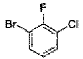 | 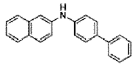 | 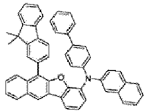 | **70%** |
| **0512** | 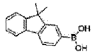 | 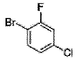 | 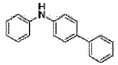 | 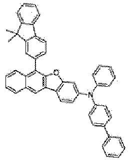 | **75%** |
| **0603** | 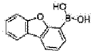 | 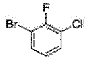 | 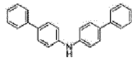 | 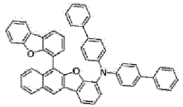 | **73%** |
| **0605** | 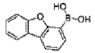 | 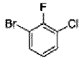 | 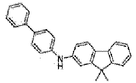 | 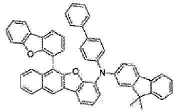 | **73%** |
| **0607** | 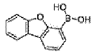 | 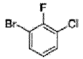 | 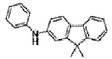 | 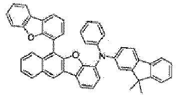 | **71%** |
| **0634** | 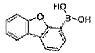 | 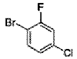 | 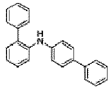 | 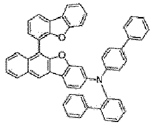 | **77%** |
| **0636** | 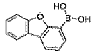 | 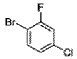 | 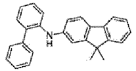 | 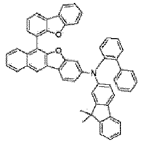 | **62%** |
| **0678** | 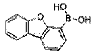 | 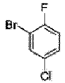 | 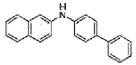 | 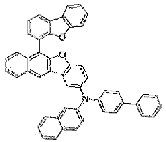 | **71%** |
| **0699** | 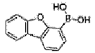 | 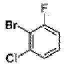 | 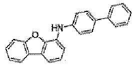 | 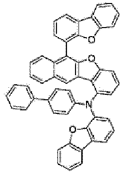 | **75%** |
| **0723** | 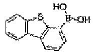 | 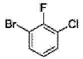 | 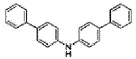 | 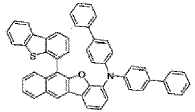 | **82%** |
| **0753** | 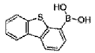 | 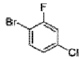 | 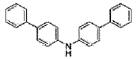 | 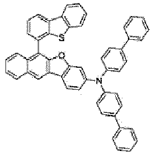 | **61%** |
| **0791** | 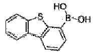 | 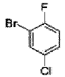 | 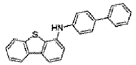 | 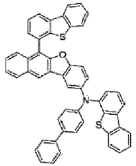 | **76%** |
| **0797** | 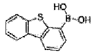 | 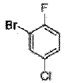 | 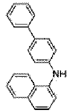 | 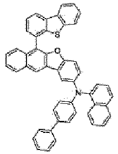 | **75%** |
| **0813** | 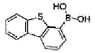 | 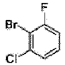 | 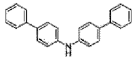 | 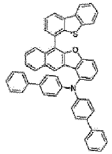 | **76%** |
| **0824** | 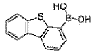 | 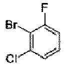 | 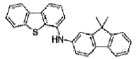 | 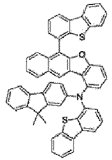 | **71%** |
| **0843** | 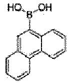 | 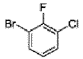 | 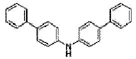 | 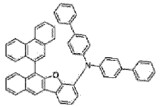 | **71%** |
| **0902** | 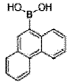 | 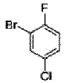 | 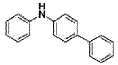 | 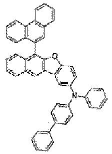 | **79%** |
| **0947** | 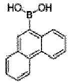 | 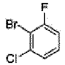 | 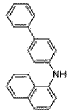 | 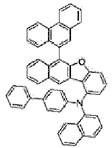 | **79%** |
| **0961** | 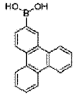 | 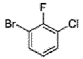 | 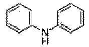 | 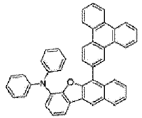 | **73%** |
| **0992** | 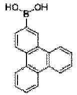 | 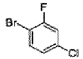 | 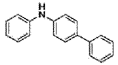 | 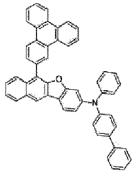 | **73%** |
| **1038** | 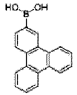 | 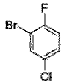 | 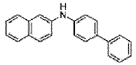 | 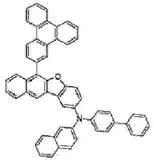 | **77%** |
| **1067** | 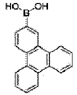 | 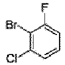 | 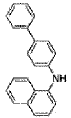 | 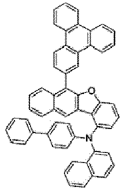 | **70%** |
| **1082** | 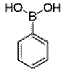 | 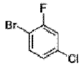 | 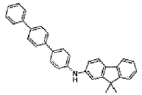 | 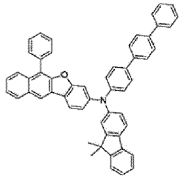 | **76%** |
| **1083** | 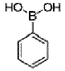 | 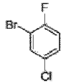 | 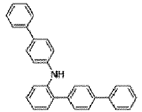 | 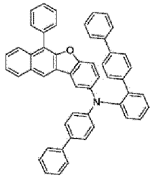 | **75%** |
| **1084** | 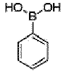 | 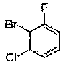 | 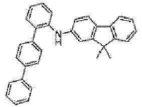 | 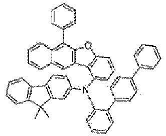 | **72%** |
| **1146** | 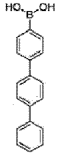 | 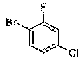 | 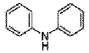 | 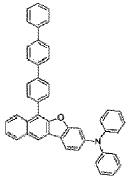 | **78%** |

Heterocyclic compounds corresponding to Chemical Formula 1 other than the compounds described in Preparation Example 1 and Table 1 were also prepared in the same manner as in the preparation examples described above.

Synthesis identification results for the compounds prepared above are as described in the following [Table 2] and [Table 3]. Table 2 shows measurement values of ¹H NMR (CDCl₃, 300 MHz), and Table 3 shows measurement values of FD-mass spectrometry (FD-MS: field desorption mass spectrometry).

**[Table 2]**

| **Compound** | **¹H NMR (CDCl₃, 300 MHz)** |
|---|---|
| **0002** | δ=8.55 (1H, d), 8.08 (1H, d), 7.52-7.41 (15H, m), 7.25 (1H, d), 7.20 (2H, m), 7.07 (1H, t), 6.81 (1H, t), 6.69 (2H, d), 6.63 (2H, d), 6.39 (1H, d) |
| **0003** | δ=8.55 (1H, d), 8.08 (1H, d), 7.55-7.41 (22H, m), 7.25 (1H, d), 7.07 (1H, t), 6.69 (4H, d), 6.39 (1H, d) |
| **0005** | δ=8.55 (1H, d), 8.08 (1H, d), 7.87 (1H, d), 7.62-7.38 (18H, m), 7.28-7.25 (2H, m), 7.07 (1H, t), 6.75 (1H, s), 6.69 (2H, d), 6.58 (1H, d), 6.39 (1H, d), 1.72 (6H, s) |
| **0015** | δ=8.55 (1H, d), 8.08 (1H, d), 7.89-7.81 (4H, m), 7.66-7.25 (19H, m), 7.07 (1H, t), 6.75 (1H, s), 6.69 (2H, d), 6.58 (1H, d), 6.39 (1H, d), 1.72 (6H, s) |
| **0023** | δ=8.55 (1H, d), 8.08 (1H, d), 7.87 (1H, d), 7.55-7.07 (26H, m), 6.81 (1H, t), 6.75 (1H, s), 6.63 (2H, d), 6.58 (1H, d), 6.39 (1H, d) |
| **0033** | δ=8.55 (1H, d), 8.08 (1H, d), 7.64 (1H, d), 7.55-7.41 (23H, m), 6.69 (4H, d), 6.33 (1H, d) |
| **0034** | δ=8.55 (1H, d), 8.08 (1H, d), 7.64 (1H, d), 7.55-7.41 (20H, m), 7.16 (1H, t), 7.08 (1H, d), 6.87 (1H, t), 6.69 (3H, d), 6.33 (1H, d) |
| **0038** | δ=8.55 (1H, d), 8.08 (1H, d), 7.87 (2H, d), 7.64-7.38 (16H, m), 7.28 (2H, t), 6.75 (2H, s), 6.58 (2H, d), 6.33 (1H, d), 1.72 (12H, s) |
| **0063** | δ=8.55 (1H, d), 8.08 (1H, d), 7.65 (1H, s), 7.55-7.41 (23H, m), 6.69 (4H, d), 6.39 (1H, d) |
| **0065** | δ=8.55 (1H, d), 8.08 (1H, d), 7.87 (1H, d), 7.62-7.38 (20H, m), 7.28 (1H, t), 6.75 (1H, s), 6.69 (2H, d), 6.58 (1H, d), 6.39 (1H, d), 1.72 (6H, s) |
| **0066** | δ=8.55 (1H, d), 8.08 (1H, d), 7.87 (1H, d), 7.65-7.38 (17H, m), 7.28 (1H, t), 7.16 (1H, t), 7.08 (2H, d), 6.87 (1H, t), 6.75 (1H, s), 6.69 (1H, d), 6.58 (1H, d), 6.39 (1H, d), 1.72 (6H, s) |
| **0077** | δ=8.55 (1H, d), 8.08-8.07 (2H, m), 8.02 (1H, d), 7.65 (1H, s), 7.57-7.38 (20H, m), 6.98 (1H, d), 6.69 (2H, d), 6.39 (1H, d) |
| **0092** | δ=8.55 (1H, d), 8.08 (1H, d), 7.55-7.41 (15H, m), 7.20 (2H, m), 7.13 (1H, t), 7.02 (1H, d), 6.81 (1H, t), 6.69 (2H, d), 6.63 (2H, d), 6.33 (1H, d) |
| **0093** | δ=8.55 (1H, d), 8.08 (1H, d), 7.55-7.41 (22H, m), 7.13 (1H, t), 7.02 (1H, d), 6.69 (4H, d), 6.33 (1H, d) |
| **0095** | δ=8.55 (1H, d), 8.08 (1H, d), 7.87 (1H, d), 7.55-7.38 (18H, m), 7.28 (1H, t), 7.13 (1H, t), 7.02 (1H, d), 6.75 (1H, s), 6.69 (2H, d), 6.58 (1H, d), 6.33 (1H, d), 1.72 (6H, s) |
| **0108** | δ=8.55 (1H, d), 8.08 (1H, d), 7.88-7.74 (4H, m), 7.55-7.36 (18H, m), 7.13 (1H, t), 7.02 (1H, d), 6.69 (2H, d), 6.33 (1H, d) |
| **0110** | δ=8.55 (1H, d), 8.08 (1H, d), 7.88-7.74 (5H, m), 7.55-7.28 (15H, m), 7.13 (1H, t), 7.02 (1H, d), 6.75 (1H, s), 6.58 (1H, d), 6.33 (1H, d) 1.72 (6H, s) |
| **0114** | δ=8.55 (1H, d), 8.08 (1H, d), 7.87 (2H, d), 7.75 (1H, d), 7.55-7.28 (23H, m), 7.19-7.13 (3H, m), 7.02 (1H, d), 6.69 (2H, d), 6.55 (1H, s), 6.39 (1H, d), 6.33 (1H, d) |
| **0124** | δ=8.55 (1H, d), 8.08 (1H, d), 7.55-7.41 (19H, m), 7.25 (4H, s), 7.16-7.07 (4H, m), 6.87 (1H, t), 6.69 (3H, d), 6.39 (1H, d) |
| **0126** | δ=8.55 (1H, d), 8.08 (1H, d), 7.87 (1H, d), 7.62-7.41 (15H, m), 7.28-7.25 (6H, m), 7.16-7.07 (3H, m), 6.87 (1H, t), 6.75 (1H, s), 6.69 (1H, d), 6.58 (1H, d), 6.39 (1H, d) |
| **0159** | δ=8.55 (1H, d), 8.08 (1H, d), 7.89 (1H, d), 7.66 (1H, d), 7.64 (1H, d), 7.55-7.25 (23H, m), 7.07 (1H, t), 6.69 (2H, d), 6.39 (1H, d), 6.33 (1H, d) |
| **0169** | δ=8.55 (1H, d), 8.08-8.02 (3H, m), 7.87 (1H, d), 7.64-7.38 (17H, m), 7.28-7.25 (5H, m), 6.98 (1H, d), 6.75 (1H, s), 6.58 (1H, d), 6.33 (1H, d), 1.72 (6H, s) |
| **0200** | δ=8.55 (1H, d), 8.08 (1H, d), 7.88-7.74 (5H, m), 7.62-7.25 (21H, m), 6.75 (1H, s), 6.58 (1H, d), 6.39 (1H, d), 1.72 (6H, s) |
| **0204** | δ=8.55 (1H, d), 8.08 (1H, d), 7.87 (2H, d), 7.75 (1H, d), 7.65 (1H, s), 7.55-7.16 (30H, m), 6.69 (2H, d), 6.55 (1H, s), 6.39 (2H, d) |
| **0216** | δ=8.55 (1H, d), 8.08 (1H, d), 7.87 (1H, d), 7.62-7.38 (15H, m), 7.28-7.25 (5H, m), 7.16-7.02 (5H, m), 6.87 (1H, t), 6.75-6.69 (2H, m), 6.58 (1H, d), 6.33 (1H, d), 1.72 (6H, s) |
| **0219** | δ=8.55 (1H, d), 8.08 (1H, d), 7.89 (1H, d), 7.66 (1H, d), 7.55-7.25 (22H, m), 7.13-7.02 (3H, m), 6.69 (2H, d), 6.39 (1H, d), 6.33 (1H, d) |
| **0228** | δ=8.55 (1H, d), 8.08 (1H, d), 7.88-774 (4H, m), 7.55-7.36 (18H, m), 7.25 (4H, s), 7.13 (1H, t), 7.02 (1H, d), 6.69 (2H, d), 6.33 (1H, d) |
| **0243** | δ=8.55 (2H, d), 8.42 (1H, d), 8.08-8.04 (3H, m), 7.55-7.41 (20H, m), 7.25 (1H, d), 7.07 (1H, t), 6.69 (4H, d), 6.39 (1H, d) |
| **0264** | δ=8.55 (2H, d), 8.42 (1H, d), 8.08-8.04 (3H, m), 7.87 (2H, d), 7.75 (1H, d), 7.55-7.16 (24H, m), 7.07 (1H, t), 6.69 (2H, d), 6.55 (1H, s), 6.39 (2H, d) |
| **0276** | δ=8.55 (2H, d), 8.42 (1H, d), 8.08-8.04 (3H, m), 7.87 (1H, d), 7.64-7.38 (15H, m), 7.28 (1H, t), 7.16-7.08 (3H, m), 6.87 (1H, t), 6.75 (1H, s), 6.69 (1H, d), 6.58 (1H, d), 6.33 (1H, d), 1.72 (6H, s) |
| **0292** | δ=8.55 (2H, d), 8.42 (1H, d), 8.08-8.04 (3H, m), 7.87 (1H, d), 7.61-7.08 (29H, m), 6.87 (1H, t), 6.75 (1H, s), 6.69 (1H, d), 6.58 (1H, d), 6.33 (1H, d) |
| **0315** | δ=8.55 (2H, d), 8.42 (1H, d), 8.08-8.04 (3H, m), 7.89-7.81 (4H, m), 7.66-7.54 (11H, m), 7.45-7.28 (7H, m), 6.75 (1H, s), 6.69 (2H, d), 6.58 (1H, d), 6.39 (1H, d), 1.72 (6H, s) |
| **0320** | δ=8.55 (2H, d), 8.42 (1H, d), 8.08-8.04 (3H, m), 7.88-7.74 (5H, m), 7.65-7.28 (15H, m), 6.75 (1H, s), 6.58 (1H, d), 6.39 (1H, d), 1.72 (6H, s) |
| **0333** | δ=8.55 (2H, d), 8.42 (1H, d), 8.08-8.04 (3H, m), 7.61-7.41 (20H, m), 7.13 (1H, t), 7.02 (1H, d), 6.69 (4H, d), 6.33 (1H, d) |
| **0334** | δ=8.55 (2H, d), 8.42 (1H, d), 8.08-8.04 (3H, m), 7.61-7.41 (17H, m), 7.16-7.02 (5H, m), 6.87 (1H, t), 6.69 (3H, d), 6.33 (1H, d) |
| **0336** | δ=8.55 (2H, d), 8.42 (1H, d), 8.08-8.04 (3H, m), 7.87 (1H, d), 7.62-7.38 (13H, m), 7.28 (1H, t), 7.16-7.02 (5H, m), 6.87 (1H, t), 6.75 (1H, s), 6.69 (1H, d), 6.58 (1H, d), 6.33 (1H, d), 1.72 (6H, s) |
| **0355** | δ=8.55 (2H, d), 8.42 (1H, d), 8.08-8.04 (3H, m), 7.87 (2H, d), 7.75 (1H, d), 7.61-7.02 (25H, m), 6.87 (1H, t), 6.69 (1H, d), 6.55 (1H, s), 6.39 (1H, d), 6.33 (1H, d) |
| **0365** | δ=8.55 (1H, d), 8.08-7.87 (5H, m), 7.73 (1H, d), 7.62-7.38 (15H, m), 7.28-7.25 (2H, m), 7.07 (1H, t), 6.75 (1H, s), 6.69 (2H, d), 6.58 (1H, d), 6.39 (1H, d), 1.72 (6H, s) |
| **0380** | δ=8.55 (1H, d), 8.08-7.73 (10H, m), 7.62-7.25 (14H, m), 7.07 (1H, t), 6.75 (1H, s), 6.58 (1H, d), 6.39 (1H, d), 1.72 (6H, s) |
| **0390** | δ=8.93 (2H, d), 8.55 (1H, d), 8.13-7.82 (12H, m), 7.87 (1H, d), 7.59-7.41 (13H, m), 7.25 (1H, d), 7.07-7.02 (2H, m), 6.69 (2H, d), 6.39 (1H, d) |
| **0399** | δ=8.55 (1H, d), 8.08-7.89 (5H, m), 7.66-7.25 (20H, m), 7.07 (1H, t), 6.69 (2H, d), 6.39 (1H, d), 6.33 (1H, d) |
| **0421** | δ=8.55 (1H, d), 8.08-7.92 (4H, m), 7.73 (1H, d), 7.65-7.55 (6H, m), 7.45 (1H, d), 7.41 (1H, d), 7.20 (4H, t), 6.81 (2H, t), 6.63 (4H, d), 6.39 (1H, d) |
| **0425** | δ=8.55 (1H, d), 8.08-7.87 (5H, m), 7.73 (1H, d), 7.65-7.38 (18H, m), 7.28 (1H, t), 6.75 (1H, s), 6.69 (2H, d), 6.58 (1H, d), 6.39 (1H, d), 1.72 (6H, s) |
| **0452** | δ=8.55 (1H, d), 8.08-7.92 (4H, m), 7.73 (1H, d), 7.59-7.41 (13H, m), 7.20-7.13 (3H, m), 7.02 (1H, d), 6.81 (1H, t), 6.69 (2H, d), 6.63 (2H, d), 6.33 (1H, d) |
| **0464** | δ=8.55 (1H, d), 8.45 (1H, d), 8.08-7.81 (7H, m), 7.73 (1H, d), 7.59-7.38 (11H, m), 7.28-7.27 (2H, m), 7.13 (1H, t), 7.02 (1H, d), 6.86 (1H, d), 6.75 (1H, s), 6.58 (1H, d), 6.33 (1H, d), 1.72 (6H, s) |
| **0466** | δ=8.55 (1H, d), 8.45-8.41 (2H, m), 8.20 (1H, d), 8.08-7.87 (6H, m), 7.73 (1H, d), 7.59-7.38 (14H, m), 7.28 (1H, t), 7.13 (1H, t), 7.02 (1H, d), 6.75 (1H, s), 6.69 (2H, d), 6.58 (1H, d), 6.33 (1H, d), 1.72 (6H, s) |
| **0482** | δ=8.55 (1H, d), 8.08 (1H, d), 7.93 (1H, d), 7.87 (1H, d), 7.77 (1H, s), 7.63-7.38 (13H, m), 7.25 (1H, d), 7.20 (2H, t), 7.07 (1H, t), 6.81 (1H, t), 6.69 (2H, d), 6.63 (2H, d), 6.39 (1H, d), 1.72 (6H, s) |
| **0485** | δ=8.55 (1H, d), 8.08 (1H, d), 7.93-7.87 (3H, m), 7.77 (1H, s), 7.55-7.38 (16H, m), 7.28 (1H, t), 7.25 (1H, d), 7.07 (1H, t), 6.75 (1H, s), 6.69 (2H, d), 6.58 (1H, d), 6.39 (1H, d), 1.72 (6H, s) |
| **0498** | δ=8.55 (1H, d), 8.08 (1H, d), 7.93-7.74 (7H, m), 7.63-7.25 (18H, m), 7.07 (1H, t), 6.69 (2H, d), 6.39 (1H, d), 1.72 (6H, s) |
| **0512** | δ=8.55 (1H, d), 8.08 (1H, d), 7.93 (1H, d), 7.87 (1H, d), 7.77 (1H, s), 7.64-7.38 (15H, m), 7.28 (1H, t), 7.20 (2H, t), 6.81 (1H, t), 6.69 (2H, d), 6.63 (2H, d), 6.33 (1H, d), 1.72 (6H, s) |
| **0603** | δ=8.55 (1H, d), 8.08 (1H, d), 7.89-7.81 (3H, m), 7.66 (1H, d), 7.55-7.25 (21H, m), 7.07 (1H, t), 6.69 (4H, d), 6.39 (1H, d) |
| **0605** | δ=8.55 (1H, d), 8.08 (1H, d), 7.89-7.81 (4H, m), 7.66-7.25 (19H, m), 7.07 (1H, t), 6.75 (1H, s), 6.69 (2H, d), 6.58 (1H, d), 6.39 (1H, d), 1.72 (6H, s) |
| **0607** | δ=8.55 (1H, d), 8.08 (1H, d), 7.89-7.81 (4H, m), 7.66-7.55 (5H, m), 7.45-7.20 (9H, m), 7.07 (1H, t), 6.81 (1H, t), 6.75 (1H, s), 6.63 (2H, d), 6.58 (1H, d), 6.39 (1H, d), 1.72 (6H, s) |
| **0634** | δ=8.55 (1H, d), 8.08 (1H, d), 7.89-7.81 (3H, m), 7.66 (1H, d), 7.64 (1H, d), 7.55-7.32 (18H, m), 7.16 (1H, t), 7.08 (2H, d), 6.87 (1H, t), 6.69 (3H, d), 6.33 (1H, d) |
| **0636** | δ=8.55 (1H, d), 8.08 (1H, d), 7.89-7.81 (4H, m), 7.64-7.28 (17H, m), 7.16-7.08 (3H, m), 6.87 (1H, t), 6.75 (1H, s), 6.69 (1H, d), 6.58 (1H, d), 6.33 (1H, d), 1.72 (6H, s) |
| **0678** | δ=8.55 (1H, d), 8.08 (1H, d), 7.89-7.74 (7H, m), 7.66-7.65 (2H, m), 7.55-7.32 (17H, m), 6.69 (2H, d), 6.39 (1H, d) |
| **0699** | δ=8.55 (1H, d), 8.08 (1H, d), 7.89-7.81 (4H, m), 7.66 (2H, d), 7.55-7.25 (16H, m), 7.13-7.02 (3H, m), 6.69 (2H, d), 6.39 (1H, d), 6.33 (1H, d) |
| **0723** | δ=8.55 (1H, d), 8.45-8.41 (2H, m), 8.20 (1H, d), 8.08 (1H, d), 7.98 (1H, d), 7.58-7.41 (20H, m), 7.25 (1H, d), 7.07 (1H, t), 6.69 (4H, d), 6.39 (1H, d) |
| **0753** | δ=8.55 (1H, d), 8.45-8.41 (2H, m), 8.20 (1H, d), 8.08 (1H, d), 7.98 (1H, d), 7.64-7.41 (22H, m), 6.69 (4H, d), 6.33 (1H, d) |
| **0791** | δ=8.55 (1H, d), 8.45-8.41 (2H, m), 8.20 (1H, d), 8.08 (1H, d), 7.98 (1H, d), 7.81 (1H, d), 7.65 (1H, s), 7.58-7.41 (16H, m), 7.27 (1H, t), 6.86 (1H, d), 6.69 (2H, d), 6.39 (1H, d) |
| **0797** | δ=8.55 (1H, d), 8.45-8.41 (2H, m), 8.20 (1H, d), 8.08-7.98 (4H, m), 7.65-7.38 (19H, m), 6.98 (1H, d), 6.69 (2H, d), 6.39 (1H, d) |
| **0813** | δ=8.55 (1H, d), 8.45-8.41 (2H, m), 8.20 (1H, d), 8.08 (1H, d), 7.98 (1H, d), 7.58-7.41 (20H, m), 7.13 (1H, t), 7.02 (1H, d), 6.69 (4H, d), 6.33 (1H, d) |
| **0824** | δ=8.55 (1H, d), 8.45-8.41 (2H, m), 8.20 (1H, d), 8.08 (1H, d), 7.98 (2H, d), 7.87 (1H, d), 7.81 (1H, d), 7.62-7.38 (11H, m), 7.28-7.27 (2H, m), 7.13 (1H, t), 7.02 (1H, d), 6.86 (1H, d), 6.75 (1H, s), 6.58 (1H, d), 6.33 (1H, d), 1.72 (6H, s) |
| **0843** | δ=8.93 (2H, d), 8.55 (1H, d), 8.12-8.08 (3H, m), 7.93-7.82 (5H, m), 7.55-7.41 (17H, m), 7.25 (1H, d), 7.07 (1H, t), 6.69 (4H, d), 6.39 (1H, d) |
| **0902** | δ=8.93 (2H, d), 8.55 (1H, d), 8.12-8.08 (3H, m), 7.93-7.82 (5H, m), 7.65 (1H, s), 7.55-7.41 (11H, m), 7.20 (1H, t), 6.81 (1H, t), 6.69 (4H, d), 6.39 (1H, d) |
| **0947** | δ=8.93 (2H, d), 8.55 (1H, d), 8.12-8.08 (3H, m), 7.93-7.82 (5H, m), 7.57-7.41 (14H, m), 7.13 (1H, t), 7.02 (1H, d), 6.98 (1H, d), 6.69 (2H, d), 6.33 (1H, d) |
| **0961** | δ=9.15 (1H, s), 8.93 (2H, d), 8.55 (1H, d), 8.18-8.04 (5H, m), 7.88-7.82 (4H, m), 7.55 (2H, d), 7.45 (1H, s), 7.25-7.20 (5H, m), 7.07 (1H, t), 6.81 (2H, t), 6.63 (4H, d), 6.39 (1H, d) |
| **0992** | δ=9.15 (1H, s), 8.93 (2H, d), 8.55 (1H, d), 8.18-8.04 (5H, m), 7.88-7.82 (4H, m), 7.64 (1H, d), 7.55-7.41 (11H, m), 7.20 (2H, t), 6.81 (1H, t), 6.69-6.63 (4H, m), 6.33 (1H, d) |
| **1038** | δ=9.15 (1H, s), 8.93 (2H, d), 8.55 (1H, d), 8.18-8.08 (5H, m), 7.88-7.74 (8H, m), 7.65 (1H, s), 7.55-7.36 (14H, m), 6.69 (2H, d), 6.39 (1H, d) |
| **1067** | δ=9.15 (1H, s), 8.93 (2H, d), 8.55 (1H, d), 8.18-8.02 (7H, m), 7.88-7.82 (4H, m), 7.57-7.41 (14H, m), 7.13 (1H, t), 7.02 (1H, d), 6.98 (1H, d), 6.69 (2H, d), 6.33 (1H, d) |
| **1082** | δ=8.55 (1H, d), 8.08 (1H, d), 7.87 (1H, d), 7.64-7.38 (20H, m), 7.28-7.25 (5H, m), 6.75 (1H, s), 6.69 (2H, d), 6.58 (1H, d), 6.33 (1H, d), 1.72 (6H, s) |
| **1083** | δ=8.55 (1H, d), 8.08 (1H, d), 7.65 (1H, s), 7.55-7.41 (22H, m), 7.25 (4H, s), 7.16 (1H, t), 6.87 (1H, t), 6.69 (3H, d), 6.39 (1H, d) |
| **1084** | δ=8.55 (1H, d), 8.08 (1H, d), 7.87 (1H, d), 7.55-7.38 (17H, m), 7.28-7.25 (5H, m), 7.16-7.13 (2H, m), 7.02 (1H, d), 6.87 (1H, t), 6.75 (1H, s), 6.69 (1H, d), 6.58 (1H, d), 6.33 (1H, d), 1.72 (6H, s) |
| **1146** | δ=8.55 (1H, d), 8.08 (1H, d), 7.64 (1H, d), 7.55-7.41 (9H, m), 7.25-7.20 (12H, m), 6.81 (2H, t), 6.63 (4H, d), 6.33 (1H, d) |

**[Table 3]**

| **Compou nd** | **FD-MS** | **Compou nd** | **FD-MS** |
|---|---|---|---|
| **0002** | m/z=537.65 (C₄₉H₂₇NO=537.21) | **0003** | m/z=613.74 (C₄₆H₃₁NO=613.24) |
| **0005** | m/z=653.81 (C₄₉H₃₅NO=653.27) | **0015** | m/z=743.89 (C₅₅H₃₇NO₂=743.28) |
| **0023** | m/z=701.85 (C₅₃H₃₅NO=701.27) | **0033** | m/z=613.74 (C₄₆H₃₁NO=613.24) |
| **0034** | m/z=613.74 (C₄₆H₃₁NO=613.24) | **0038** | m/z=693.87 (C₅₂H₃₉NO=693.30) |
| **0063** | m/z=613.74 (C₄₆H₃₁NO=613.24) | **0065** | m/z=653.81 (C₄₉H₃₅NO=653.27) |
| **0066** | m/z=653.81 (C₄₉H₃₅NO=653.27) | **0077** | m/z=587.71 (C₄₄H₂₉NO=587.22) |
| **0092** | m/z=537.65 (C₄₀H₂₇NO=537.21) | **0093** | m/z=613.74 (C₄₆H₃₁NO=613.24) |
| **0095** | m/z=653.81 (C₄₉H₃₅NO=653.27) | **0108** | m/z=587.71 (C₄₄H₂₉NO=587.22) |
| **0110** | m/z=627.77 (C₄₇H₃₃NO=627.26) | **0114** | m/z=775. 93 (C₅₉H₃₇NO=775.29) |
| **0124** | m/z=689.84 (C₅₂H₃₅NO=689.27) | **0126** | m/z=729.90 (C₅₅H₃₉NO=729.30) |
| **0159** | m/z=703. 82 (C₅₅H₃₃NO₂=703.25) | **0169** | m/z=703.87 (C₅₃H₃₇NO=703.29) |
| **0200** | m/z=703.87 (C₅₃H₃₇NO=703.29) | **0204** | m/z=852.03 (C₆₅H₄₁NO=851.32) |
| **0216** | m/z=729.90 (C₅₅H₃₉NO=729.30) | **0219** | m/z=703.82 (C₅₅H₃₃NO₂=703.25) |
| **0228** | m/z=663. 80 (C₅₀H₃₃NO=663.26) | **0243** | m/z=663. 80 (C₅₀H₃₃NO=663.26) |
| **0264** | m/z=825. 99 (C₆₃H₃₉NO=825.30) | **0276** | m/z=703.87 (C₅₃H₃₇NO=703.29) |
| **0292** | m/z=828.01 (C₆₃H₄₁NO=827.32) | **0315** | m/z=793.95 (C₃₉H₃₉NO₂=793.30) |
| **0325** | m/z=825.99 (C₆₃H₃₉NO=825.30) | **0333** | m/z=663.80 (C₅₀H₃₃NO=663.26) |
| **0334** | m/z=663. 80 (C₅₀H₃₃NO=663.26) | **0336** | m/z=703.87 (C₅₃H₃₇NO=703.29) |
| **0355** | m/z=825.99 (C₆₃H₃₉NO=825.30) | **0365** | m/z=703.87 (C₅₃H₃₇NO=703.29) |
| **0380** | m/z=677.83 (C₅₁H₃₅NO=677.27) | **0390** | m/z=737.88 (C₅₆H₃₅NO=737.27) |
| **0399** | m/z=677.79 (C₅₈H₃₁NO₂=677.24) | **0421** | m/z=511.61 (C₃₈H₂₅NO=511.19) |
| **0425** | m/z=703.87 (C₅₃H₃₇NO=703.29) | **0452** | m/z=587.71 (C₄₄H₂₉NO=587.22) |
| **0464** | m/z=733.92 (C₅₃H₃₅NOS=733.24) | **0466** | m/z=810.01 (C₅₉H₃₉NOS=809.28) |
| **0482** | m/z=653.81 (C₄₉H₃₅NO=653.27) | **0485** | m/z=769.97 (C₅₈H₄₃NO=769.33) |
| **0498** | m/z=703.87 (C₅₃H₃₇NO=703.29) | **0512** | m/z=653.81 (C₄₉H₃₅NO=653.27) |
| **0603** | m/z=703.82 (C₅₅H₃₃NO₂=703.25) | **0605** | m/z=743.89 (C₅₅H₃₇NO₂=743.28) |
| **0607** | m/z=667.79 (C₄₉H₃₃NO₂=667.25) | **0634** | m/z=703.82 (C₅₅H₃₃NO₂=703.25) |
| **0636** | m/z=743.89 (C₅₅H₃₇NO₂=743.28) | **0678** | m/z=677.79 (C₅₀H₃₁NO₂=677.24) |
| **0699** | m/z=717.81 (C₅₂H₃₁NO₃=717. 23) | **0723** | m/z=719.89 (C₅₂H₃₃NO₋S=719.23) |
| **0753** | m/z=719.89 (C₅₂H₃₃NO₋S=719.23) | **0791** | m/z=749.94 (C₅₂H₃₄NO₋S₂=749.18) |
| **0797** | m/z=693.85 (C₅₀H₃₁NO₋S=693.21) | **0813** | m/z=719.89 (C₅₂H₃₃NO₋S=719.23) |
| **0824** | m/z=790.00 (C₅₅H₃₅NO₋S₂=789.22) | **0843** | m/z=713.86 (C₅₄H₃₅NO₋=713.27) |
| **0902** | m/z=637.77 (C₄₈H₃₁NO₋=637.24) | **0947** | m/z=687.82 (C₅₂H₃₃NO₋=687.26) |
| **0961** | m/z=611.73 (C₄₆H₂₉NO₋=611.22) | **0992** | m/z=687.82 (C₅₂H₃₃NO₋=687.26) |
| **1038** | m/z=737.88 (C₅₆H₃₅NO₋=737.27) | **1067** | m/z=737.88 (C₅₆H₃₅NO₋=737.27) |
| **1082** | m/z=729.90 (C₅₅H₃₉NO=729.30) | **1083** | m/z=689.84 (C₅₂H₃₅NO=689.27) |
| **1084** | m/z=729.90 (C₅₅H₃₉NO=729.30) | **1146** | m/z=613.74 (C₄₆H₃₁NO=613.24) |

### <Experimental Example 1>

### (1) Manufacture of Organic Light Emitting Device

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

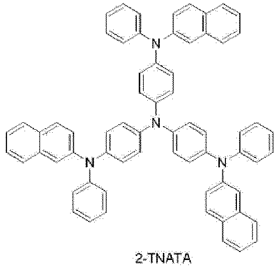

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

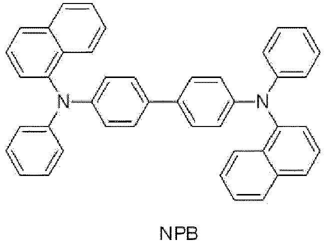

A light emitting layer was thermal vacuum deposited thereon as follows. As the light emitting layer, a compound of 9-[4-(4,6-diphenyl-1,3,5-triazin-2-yl)phenyl]-9'-phenyl-3,3'-bi-9H-carbazole was deposited to 400 Å as a host, and, as a green phosphorescent dopant, Ir(ppy)₃ was doped by 7% and deposited. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

Organic light emitting devices were manufactured in the same manner as in Experimental Example 1 except that compounds shown in the following Table 4 were used instead of the compound NPB used when forming the hole transfer layer, and driving voltage and light emission efficiency of the organic electroluminescent devices according to Experimental Example 1 are as shown in the following Table 4.

### (2) Driving Voltage and Light Emission Efficiency of Organic Light Emitting Device

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Properties of the organic electroluminescent devices of the present disclosure are as shown in the following Table 4.

**[Table 4]**

| | Compound | Driving Voltage (V) | Light Emission Efficiency (cd/A) | Lifetime (T90) |
|---|---|---|---|---|
| Example 1 | 0002 | 4.16 | 119.52 | 153 |
| Example 2 | 0005 | 4.11 | 119.81 | 152 |
| Example 3 | 0023 | 3.96 | 123.31 | 165 |
| Example 4 | 0034 | 4.09 | 125.31 | 156 |
| Example 5 | 0038 | 3.87 | 122.12 | 194 |
| Example 6 | 0063 | 4.08 | 120.54 | 177 |
| Example 7 | 0065 | 4.01 | 118.91 | 172 |
| Example 8 | 0092 | 4.13 | 119.76 | 193 |
| Example 9 | 0095 | 3.98 | 120.91 | 169 |
| Example 10 | 0110 | 3.89 | 122.23 | 195 |
| Example 11 | 0114 | 4.11 | 122.95 | 172 |
| Example 12 | 0124 | 4.04 | 124.76 | 197 |
| Example 13 | 0126 | 3.99 | 123.82 | 168 |
| Example 14 | 0169 | 3.95 | 120.32 | 165 |
| Example 15 | 0200 | 3.98 | 124.17 | 200 |
| Example 16 | 0204 | 4.08 | 122.85 | 185 |
| Example 17 | 0216 | 4.09 | 125.53 | 163 |
| Example 18 | 0243 | 4.09 | 123.10 | 167 |
| Example 19 | 0264 | 3.97 | 122.78 | 158 |
| Example 20 | 0276 | 4.13 | 120.98 | 148 |
| Example 21 | 0315 | 4.15 | 119.79 | 157 |
| Example 22 | 0333 | 4.04 | 119.86 | 175 |
| Example 23 | 0334 | 3.87 | 121.44 | 166 |
| Example 24 | 0355 | 4.02 | 124.79 | 182 |
| Example 25 | 0380 | 4.17 | 119.64 | 165 |
| Example 26 | 0390 | 4.13 | 123.63 | 157 |
| Example 27 | 0399 | 4.05 | 121.76 | 187 |
| Example 28 | 0421 | 3.92 | 120.79 | 172 |
| Example 29 | 0464 | 3.99 | 119.97 | 179 |
| Example 30 | 0466 | 4.11 | 126.42 | 180 |
| Example 31 | 0482 | 3.87 | 120.78 | 179 |
| Example 32 | 0498 | 4.10 | 123.33 | 168 |
| Example 33 | 0605 | 4.04 | 121.54 | 184 |
| Example 34 | 0607 | 4.10 | 120.81 | 187 |
| Example 35 | 0678 | 3.83 | 119.82 | 173 |
| Example 36 | 0753 | 4.15 | 120.76 | 171 |
| Example 37 | 0791 | 3.90 | 121.96 | 185 |
| Example 38 | 0797 | 4.00 | 122.76 | 176 |
| Example 39 | 0843 | 4.08 | 123.39 | 166 |
| Example 40 | 0902 | 3.83 | 121.67 | 157 |
| Example 41 | 0947 | 4.11 | 124.41 | 182 |
| Example 42 | 1038 | 4.18 | 123.76 | 169 |
| Example 43 | 1067 | 3.97 | 120.58 | 173 |
| Example 44 | 1146 | 3.92 | 122.77 | 179 |
| Comparative Example 1 | NPB | 4.55 | 101.27 | 117 |
| Comparative Example 2 | M1 | 4.35 | 112.17 | 134 |
| Comparative Example 3 | M2 | 4.27 | 111.57 | 129 |
| Comparative Example 4 | M3 | 4.25 | 110.32 | 136 |

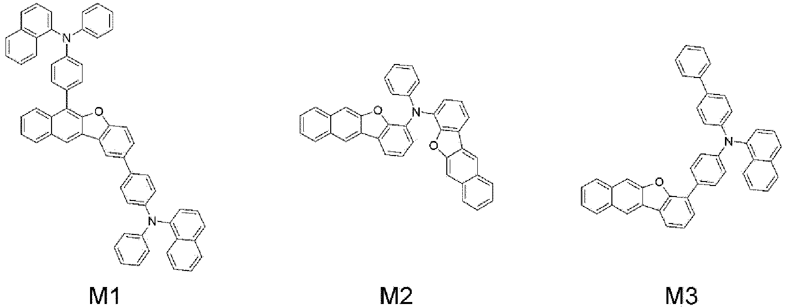

It was seen that the devices of Examples 1 to 44 according to one embodiment of the present disclosure had low driving voltage and superior efficiency and lifetime compared to the devices of Comparative Examples 1 to 4.

### <Experimental Example 2>

### (1) Manufacture of Organic Light Emitting Device

A transparent ITO electrode thin film obtained from glass for an OLED (manufactured by Samsung-Corning Co., Ltd.) was ultrasonic cleaned using trichloroethylene, acetone, ethanol and distilled water consecutively for 5 minutes each, stored in isopropanol, and used. Next, the ITO substrate was installed in a substrate folder of a vacuum deposition apparatus, and the following 4,4',4"-tris(N,N-(2-naphthyl)-phenylamino)triphenylamine (2-TNATA) was introduced to a cell in the vacuum deposition apparatus.

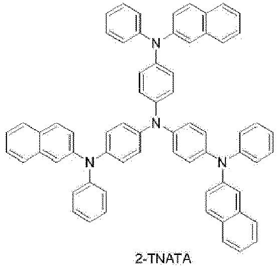

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

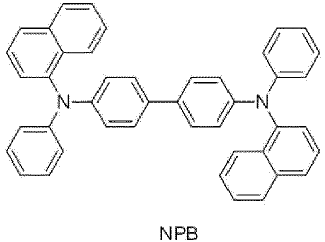

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

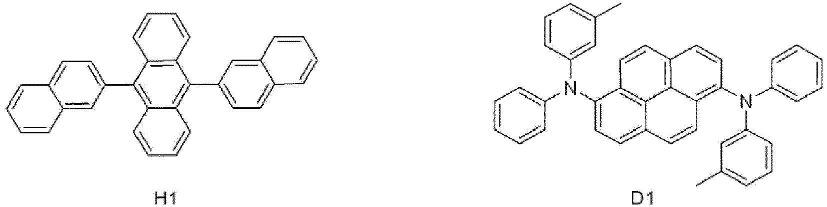

Subsequently, a compound of the following Structural Formula E1 was deposited to a thickness of 300 Å as an electron transfer layer.

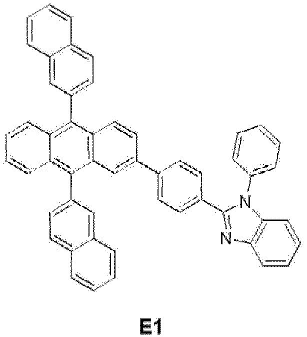

As an electron injection layer, lithium fluoride (LiF) was deposited to a thickness of 10 Å, and an Al cathode was employed to a thickness of 1,000 Å, and as a result, an OLED was manufactured. Meanwhile, all the organic compounds required to manufacture the OLED were vacuum sublimation purified under 10⁻⁸ torr to 10⁻⁶ torr by each material to be used in the OLED manufacture.

Organic electroluminescent devices were manufactured in the same manner as in Experimental Example 2 except that, after forming the hole transfer layer NPB to a thickness of 150 Å, an electron blocking layer was formed on the hole transfer layer to a thickness of 50 Å using a compound shown in the following Table 5. Results of measuring driving voltage, light emission efficiency and lifetime of the blue organic light emitting devices manufactured according to the present disclosure are as shown in the following Table 5.

**[Table 5]**

| | Compound | Driving Voltage (V) | Light Emission Efficiency (cd/A) | Lifetime (T95) |
|---|---|---|---|---|
| Example 45 | 0003 | 5.22 | 7.25 | 57 |
| Example 46 | 0015 | 5.38 | 6.79 | 57 |
| Example 47 | 0033 | 5.14 | 6.88 | 51 |
| Example 48 | 0066 | 5.32 | 6.83 | 52 |
| Example 49 | 0077 | 5.14 | 6.72 | 59 |
| Example 50 | 0108 | 5.36 | 6.94 | 56 |
| Example 51 | 0159 | 5.24 | 7.13 | 59 |
| Example 52 | 0228 | 5.33 | 6.98 | 61 |
| Example 53 | 0292 | 5.42 | 6.98 | 55 |
| Example 54 | 0320 | 5.47 | 6.92 | 62 |
| Example 55 | 0336 | 5.38 | 6.98 | 57 |
| Example 56 | 0425 | 5.44 | 6.97 | 58 |
| Example 57 | 0452 | 5.31 | 7.19 | 50 |
| Example 58 | 0485 | 5.26 | 6.92 | 53 |
| Example 59 | 0512 | 5.43 | 7.03 | 52 |
| Example 60 | 0634 | 5.23 | 7.29 | 59 |
| Example 61 | 0636 | 5.35 | 6.90 | 51 |
| Example 62 | 0723 | 5.37 | 6.94 | 51 |
| Example 63 | 0813 | 5.13 | 7.01 | 52 |
| Example 64 | 0824 | 5.48 | 6.90 | 53 |
| Example 65 | 0961 | 5.37 | 6.79 | 57 |
| Example 66 | 0992 | 5.44 | 6.85 | 51 |
| Example 67 | 1082 | 5.29 | 7.01 | 57 |
| Example 68 | 1083 | 5.13 | 7.06 | 61 |
| Example 69 | 1084 | 5.37 | 6.87 | 52 |
| Comparative Example 5 | M1 | 5.62 | 6.41 | 47 |
| Comparative Example 6 | M2 | 5.63 | 6.65 | 45 |
| Comparative Example 7 | M3 | 5.55 | 6.56 | 44 |

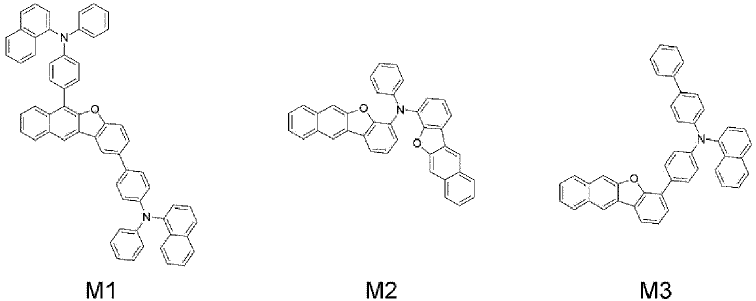

### <Experimental Example 3>

### (1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. The light emitting layer was deposited to a thickness of 500 Å by using, as a host, a compound described in the following Table 6, using an n-host (n-type host) having a favorable electron transfer ability as as a single host or a first host and using a p-host (p-type host) having a favorable hole transfer ability as a second host depositing two host compounds in one source of supply, and doping (piq)₂(Ir) (acac) to the host by 3% with respect to the host material weight as a red phosphorescent dopant or doping Ir(ppy)₃ to the host by 7% with respect to the host material weight as a green phosphorescent dopant.

After that, BCP was deposited to a thickness of 60 Å as a hole blocking layer, and Alq₃ was deposited to a thickness of 200 Å thereon as an electron transfer layer.

Herein, when using two hosts, Compounds X, Y and Z used as the n-host (first host of the following Table 6) are as follows.

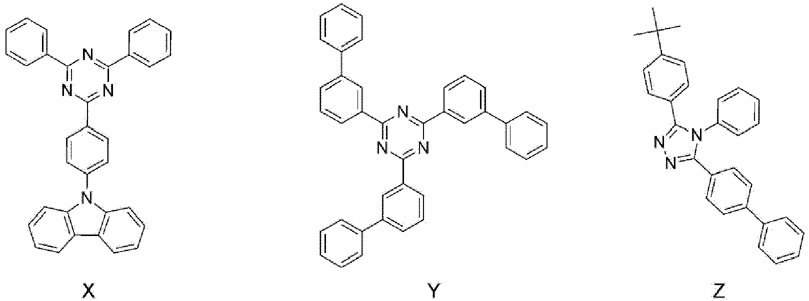

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured.

Specifically, the compounds used as the host in Examples 70 to 94 and Comparative Examples 8 to 13 are as shown in the following Table 6.

Herein, Compounds M1 to M3 used either as the single host or the second host of Comparative Examples 8 to 13 of the following Table 6 are as follows.

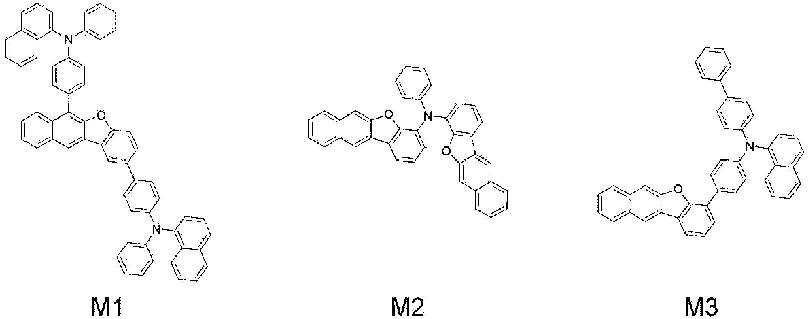

Meanwhile, all the organic compounds required to manufacture the organic light emitting device were vacuum sublimation purified under 10⁻⁸ torr to 10⁻⁶ torr for each material to be used in the organic light emitting device manufacture.

### (2) Driving Voltage and Light Emission Efficiency of Organic Light Emitting Device

For each of the organic light emitting devices manufactured as above, electroluminescent (EL) properties were measured using M7000 manufactured by McScience Inc., and with the measurement results, T₉₅ was measured when standard luminance was 6,000 cd/m² through a lifetime measurement system (M6000) manufactured by McScience Inc.. Results of measuring driving voltage, light emission efficiency, light emitting color and lifetime of the organic light emitting devices manufactured according to the present disclosure are as shown in the following Table 6.

**[Table 6]**

| | First Host | Second Host | Driving Voltage (V) | Efficienc y (cd/A) | Light Emitting Color | Lifetime (T₉₅) |
|---|---|---|---|---|---|---|
| Example 70 | 0093 | | 4.02 | 42.1 | Red | 113 |
| Example 71 | | | 3.95 | 67.6 | Green | 85 |
| Example 72 | 0219 | | 4.06 | 41.5 | Red | 109 |
| Example 73 | | | 4.09 | 72.5 | Green | 87 |
| Example 74 | 0365 | | 3.99 | 41.8 | Red | 118 |
| Example 75 | | | 3.92 | 68.1 | Green | 99 |
| Example 76 | 0603 | | 3.89 | 44.2 | Red | 104 |
| Example 77 | | | 3.77 | 71.4 | Green | 102 |
| Example 78 | 0699 | | 3.82 | 45.9 | Red | 111 |
| Example 79 | | | 3.83 | 72.9 | Green | 93 |
| Example 80 | X | 0093 | 3.80 | 45.9 | Red | 153 |
| Example 81 | | 0219 | 3.83 | 47.4 | Red | 174 |
| Example 82 | | 0365 | 3.85 | 51.1 | Red | 151 |
| Example 83 | | 0603 | 3.97 | 49.3 | Red | 168 |
| Example 84 | | 0699 | 3.98 | 48.8 | Red | 151 |
| Example 85 | Y | 0093 | 3.79 | 100.4 | Green | 154 |
| Example 86 | | 0219 | 3.84 | 97.5 | Green | 151 |
| Example 87 | | 0365 | 3.83 | 102.7 | Green | 166 |
| Example 88 | | 0603 | 3.95 | 111.2 | Green | 152 |
| Example 89 | | 0699 | 4.04 | 98.1 | Green | 167 |
| Example 90 | Z | 0093 | 3.82 | 54.3 | Red | 169 |
| Example 92 | | 0219 | 3.87 | 45.2 | Red | 170 |
| Example 92 | | 0365 | 3.98 | 44.8 | Red | 175 |
| Example 93 | | 0603 | 3.78 | 49.9 | Red | 149 |
| Example 94 | | 0699 | 3.86 | 51.0 | Red | 155 |
| Comparative Example 8 | M1 | | 4.43 | 21.5 | Red | 52 |
| Comparative Example 9 | X | M1 | 4.38 | 41.3 | Red | 110 |
| Comparative Example 10 | M2 | | 4.54 | 63.7 | Green | 65 |
| Comparative Example 11 | Y | M2 | 4.35 | 89.5 | Green | 135 |
| Comparative Example 12 | M3 | | 4.43 | 23.6 | Red | 62 |
| Comparative Example 13 | Z | M3 | 4.31 | 43.1 | Red | 109 |

From Experimental Example 3, it was identified that the organic light emitting devices of Examples 70 to 79 forming the light emitting layer using the compound according to the present application as a single host material had superior light emission efficiency and lifetime compared to the organic light emitting devices of Comparative Examples 8, 10 and 12 that did not use the compound according to the present application as a single host material when forming the light emitting layer using a single host material.

In addition, from Experimental Example 3, it was identified that the organic light emitting devices of Examples 80 to 94 forming the light emitting layer using both a first host material corresponding to the n-host and the compound according to the present application as a second host material corresponding to the p-host had superior light emission efficiency and lifetime compared to the organic light emitting devices of Comparative Examples 9, 11 and 13 forming the light emitting layer using both a first host material corresponding to the n-host and a compound that is not the compound according to the present application as a second host material corresponding to the p-host.

In addition, the organic light emitting devices of Examples 70 to 79 forming the light emitting layer using the compound according to the present application as a single host material had similar or sometimes superior light emission efficiency and lifetime compared to the organic light emitting devices of Comparative Examples 9, 11 and 13 forming the light emitting layer using both a first host material corresponding to the n-host and a compound that is not the compound according to the present application as a second host material corresponding to the p-host.

Considering that superior light emission efficiency and lifetime are generally obtained when using an n-host (n-type host) having a favorable electron transfer ability as a first host and a p-host (p-type host) having a favorable hole transfer ability as a second host compared to when using a single host material, this means that an organic light emitting device may have significantly improved light emission efficiency and lifetime when using the compound according to the present application as a host material.

This is considered to be due to the fact that, when using the compound according to the present application as a host material, holes and electrons are efficiently injected to the light emitting layer from each charge transfer layer. As described above, this is also considered to be due to the influences of orientation and space size formed by interactions between materials during deposition.

This is due to the fact that efficiently injecting holes and electrons to the light emitting layer is also affected by orientation and space size formed by interactions between materials during deposition, and as described above, this is considered to be an effect obtained by differences in the orientation properties and the space sizes between the compound of the present application and M1 to M3.

The present disclosure is not limited to the above-described examples and may be embodied in various different forms, and those skilled in the art may understand that the present disclosure may be embodied in other specific forms without changing technical ideas and essential features. Therefore, the examples described above are for illustrative purposes in all aspects and need to be construed as non-limiting.

## Claims

1. A heterocyclic compound represented by the following Chemical Formula 1: 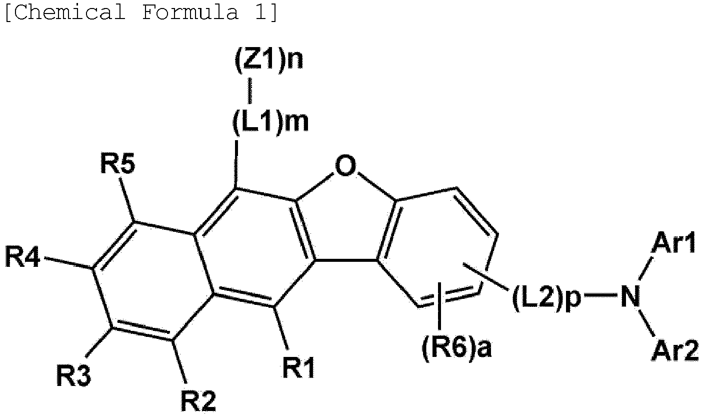
wherein, in Chemical Formula 1,
R1 to R6 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring;
L1 and L2 are the same as or different from each other, and each independently a direct bond; a substituted or unsubstituted C6 to C60 arylene group; or a substituted or unsubstituted C2 to C60 heteroarylene group;
Ar1 and Ar2 are the same as or different from each other, and each independently a substituted or unsubstituted C1 to C60 alkyl group; a substituted or unsubstituted C6 to C60 aryl group; a substituted or unsubstituted fluorenyl group; or a substituted or unsubstituted C2 to C60 heteroaryl group;
Z1 is a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted fluorenyl group; a substituted or unsubstituted C2 to C60 heteroaryl group; -P(=O)RR'; or -SiRR'R";
p and m are an integer of 0 to 4;
n is an integer of 1 to 6;
a is an integer of 0 to 3; and
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

2. The heterocyclic compound of Claim 1, wherein Chemical Formula 1 is represented by any one of the following Chemical Formulae 2 to 5: 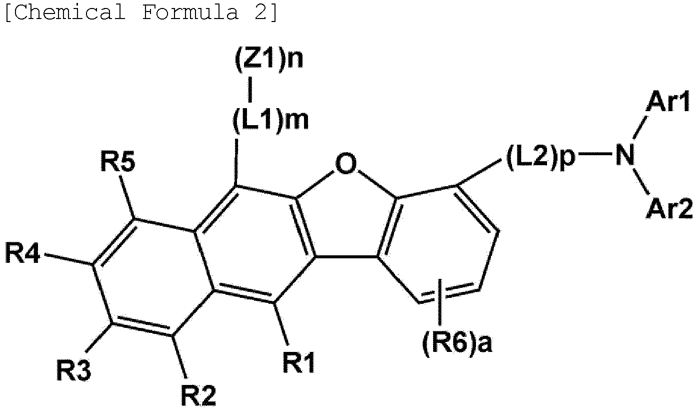 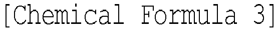 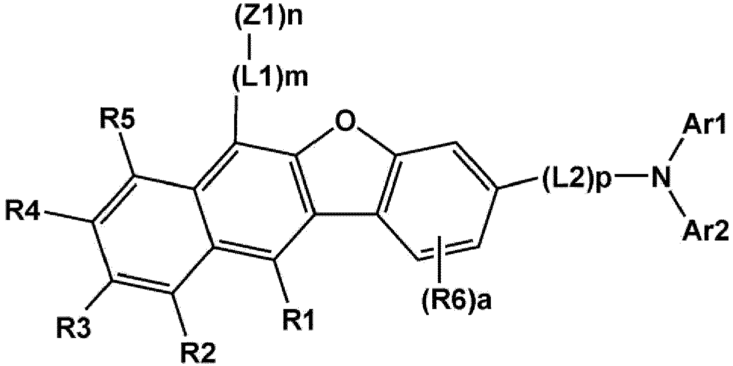 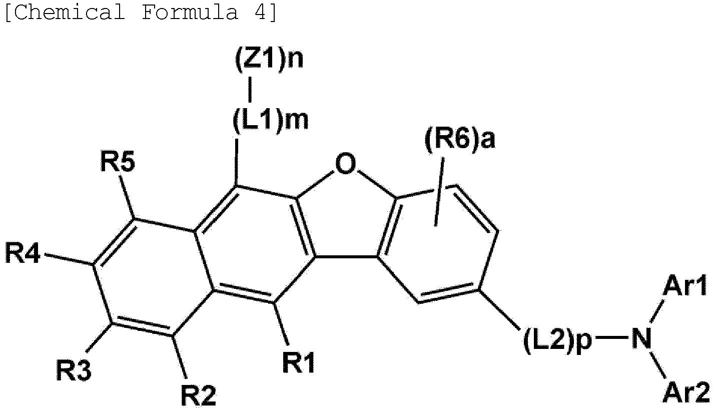 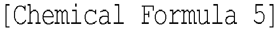 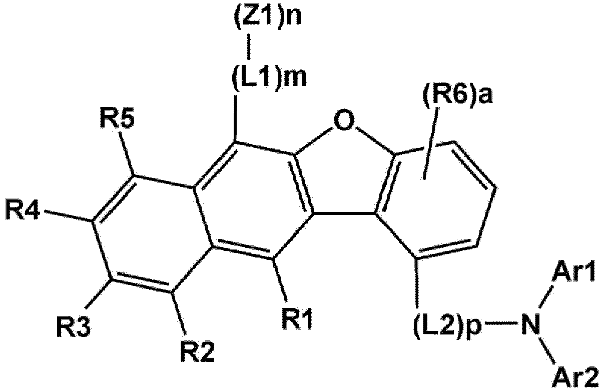
in Chemical Formulae 2 to 5,
R1 to R6, L1, L2, Z1, Ar1, Ar2, p, m, n and a have the same definitions as in Chemical Formula 1.

3. The heterocyclic compound of Claim 1, wherein Chemical Formula 1 is represented by any one of the following Chemical Formulae 6 to 9: 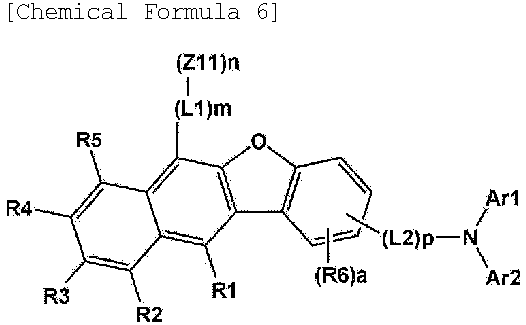 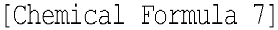 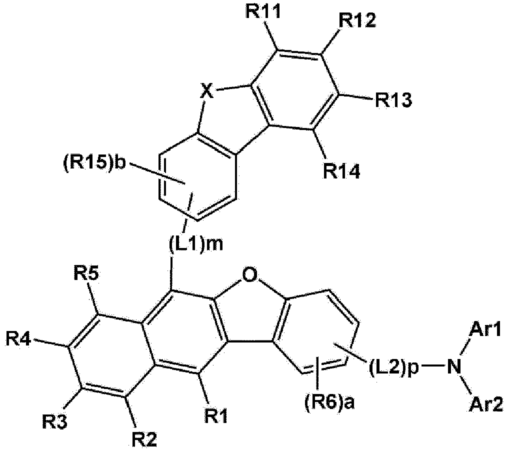 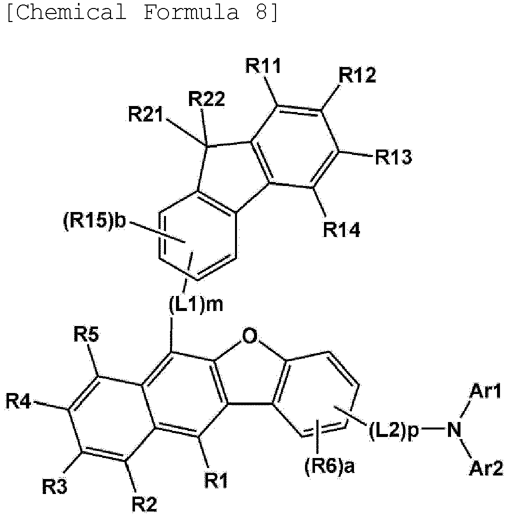 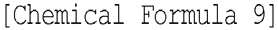 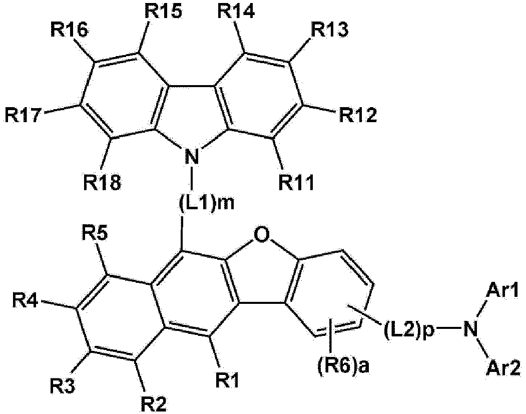
in Chemical Formulae 6 to 9,
R1 to R6, L1, L2, Ar1, Ar2, p, m, n and a have the same definitions as in Chemical Formula 1;
Z11 is a substituted or unsubstituted C6 to C60 aryl group;
X is O; or S;
R11 to R18 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring;
R21 and R22 are the same as or different from each other, and each independently a substituted or unsubstituted C1 to C60 alkyl group; or a substituted or unsubstituted C6 to C60 aryl group, or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring;
b is an integer of 0 to 3; and
R, R' and R" have the same definitions as in Chemical Formula 1.

4. The heterocyclic compound of Claim 1, wherein 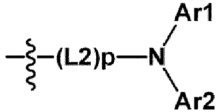 of Chemical Formula 1 is represented by any one of the following Chemical Formulae 1-1 to 1-3: 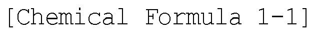 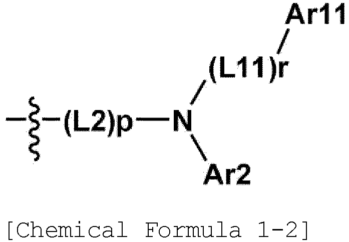 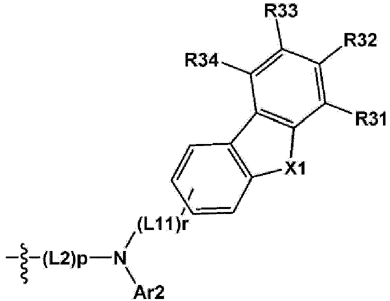 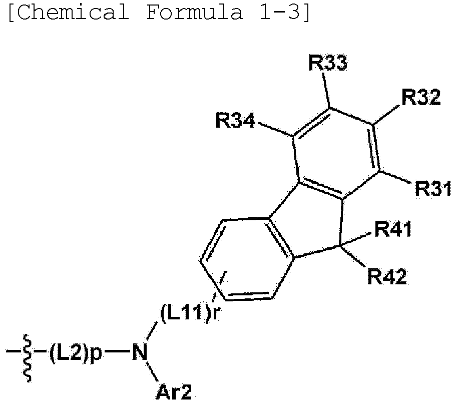
in Chemical Formulae 1-1 to 1-3,
L2, p and Ar2 have the same definitions as in Chemical Formula 1; 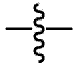
means a position linked to Chemical Formula 1;
X1 is O; or S;
L11 is a direct bond; a substituted or unsubstituted C6 to C60 arylene group; or a substituted or unsubstituted C2 to C60 heteroarylene group;
Ar11 is a substituted or unsubstituted C6 to C60 aryl group;
R31 to R34 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring;
R41 and R42 are the same as or different from each other, and each independently a substituted or unsubstituted C1 to C60 alkyl group; or a substituted or unsubstituted C6 to C60 aryl group, or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring;
r is an integer of 0 to 3; and
R, R' and R" have the same definitions as in Chemical Formula 1.

5. The heterocyclic compound of Claim 1, wherein R1 to R6 are hydrogen; or deuterium.

6. The heterocyclic compound of Claim 1, wherein Chemical Formula 1 is represented by any one of the following compounds:

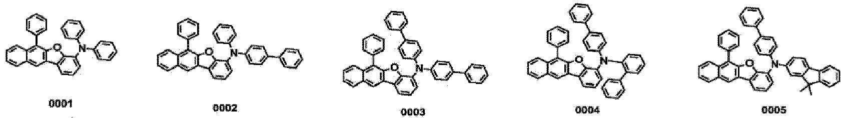

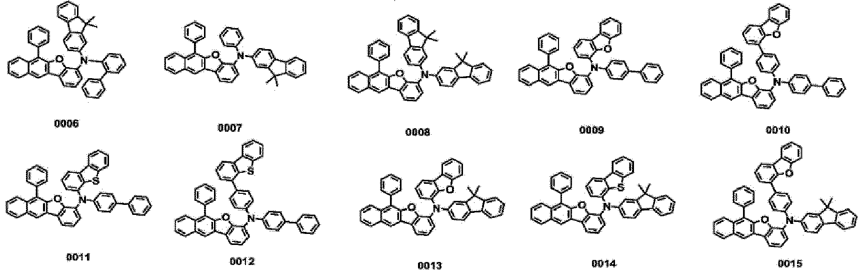

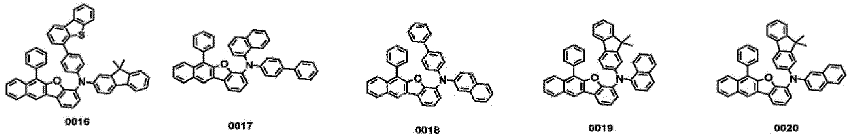

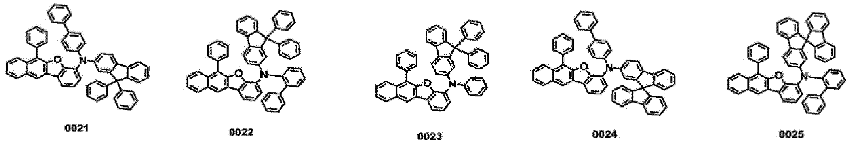

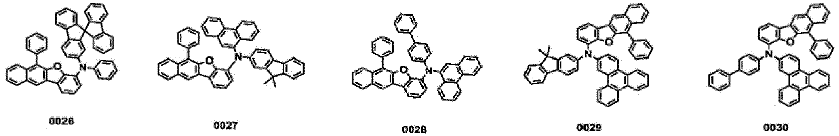

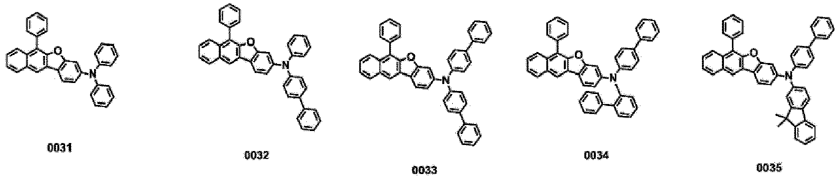

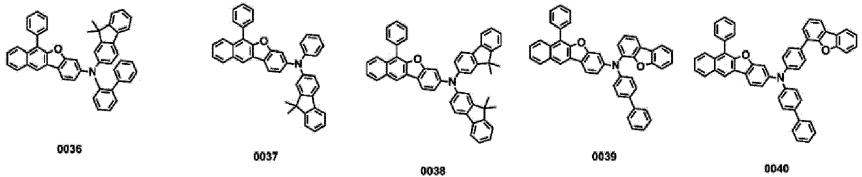

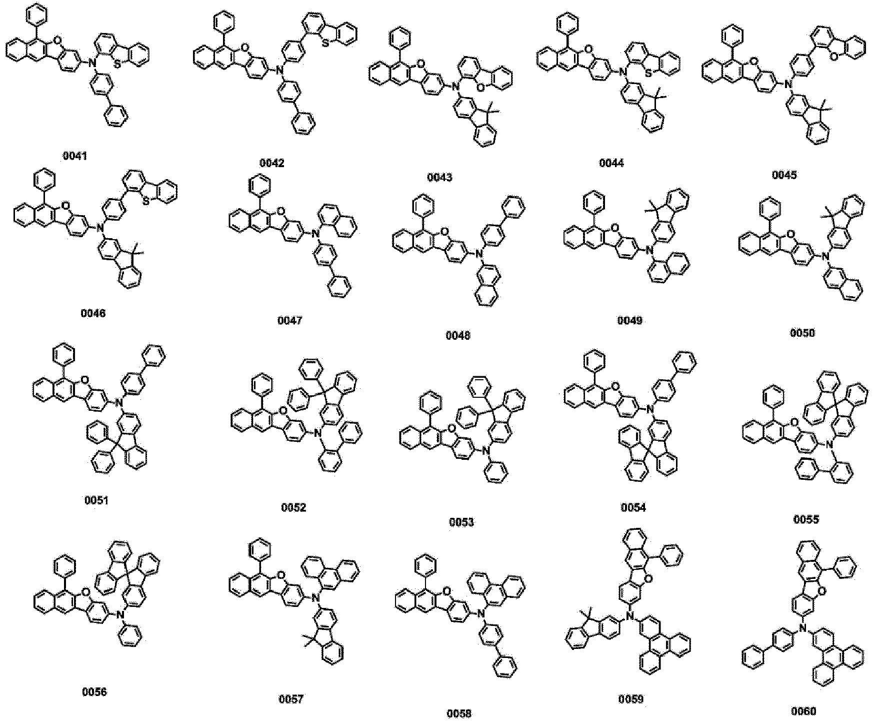

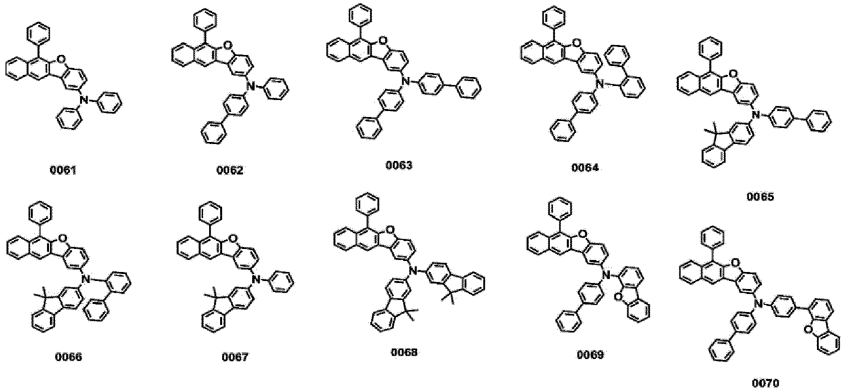

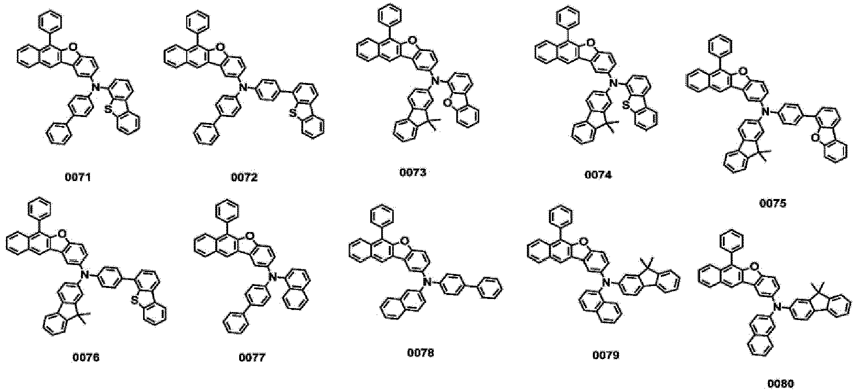

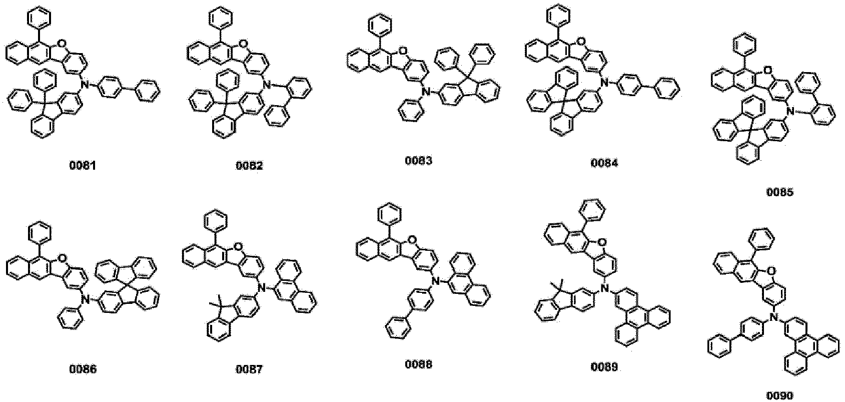

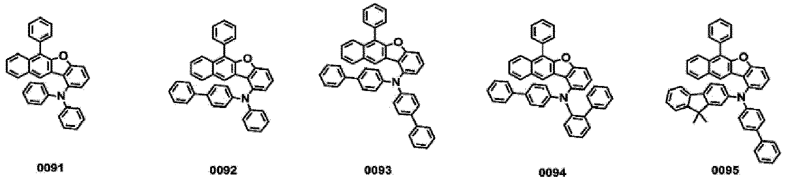

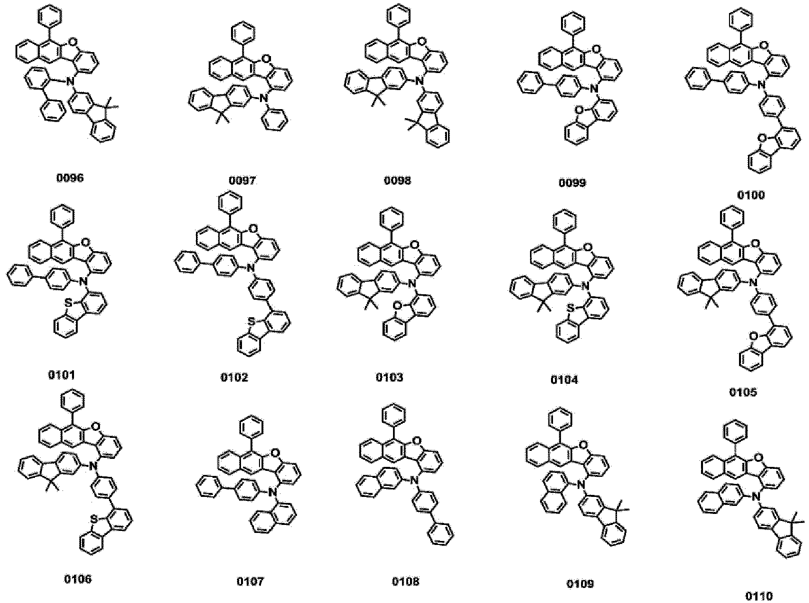

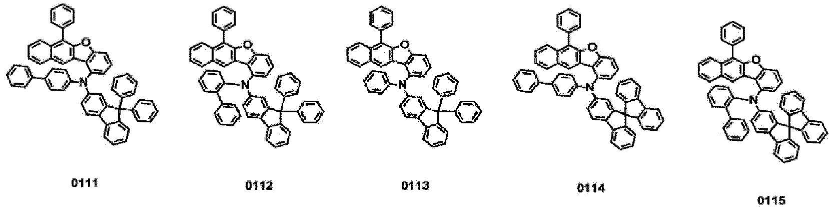

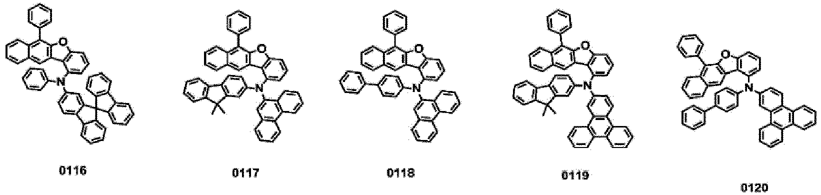

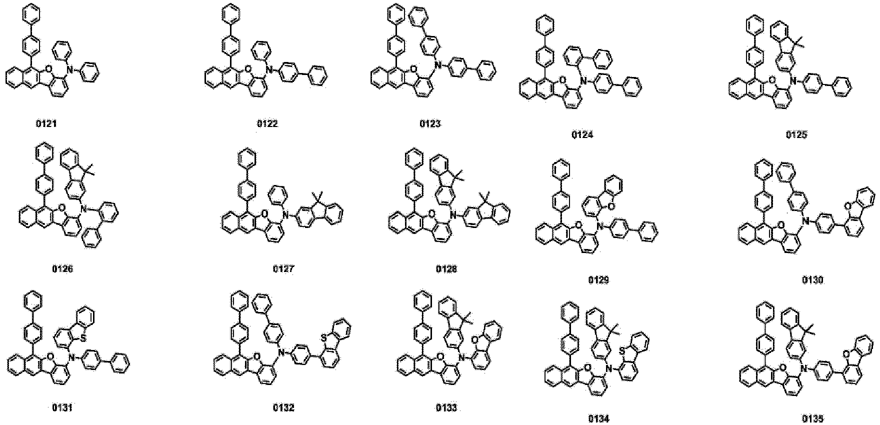

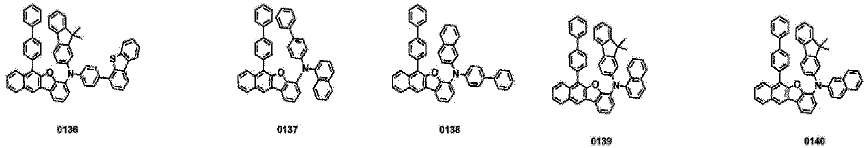

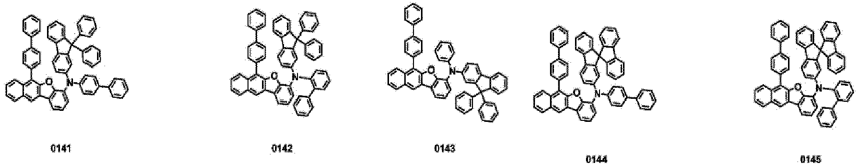

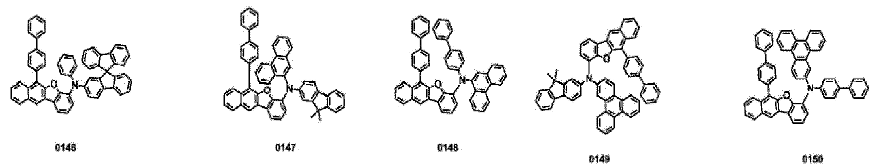

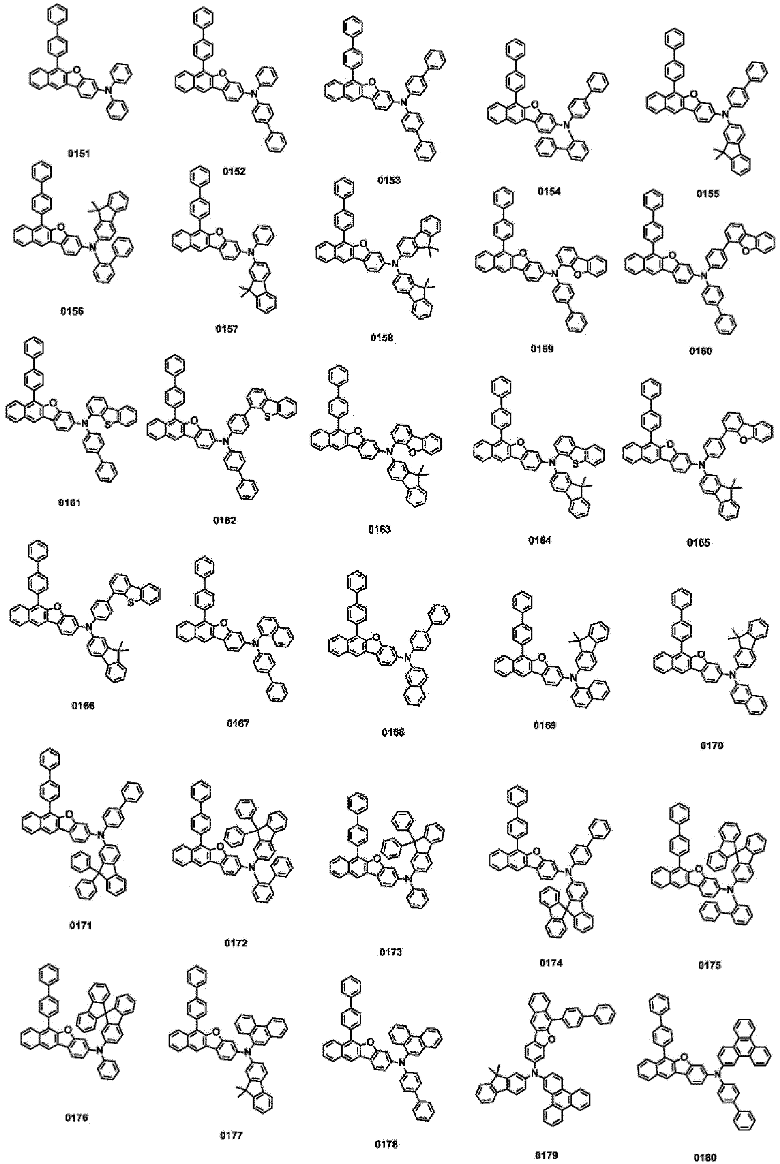

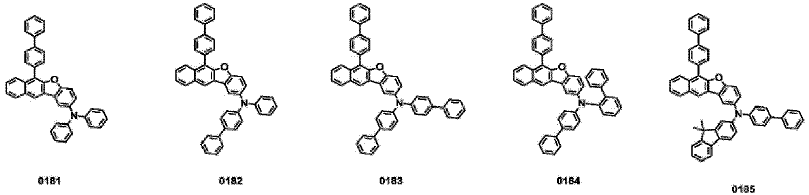

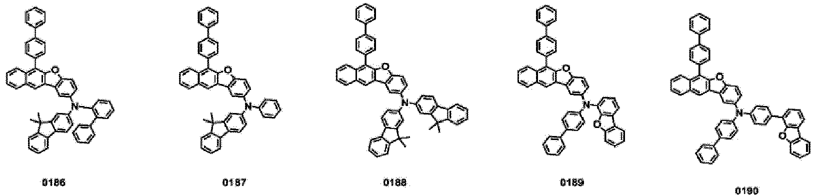

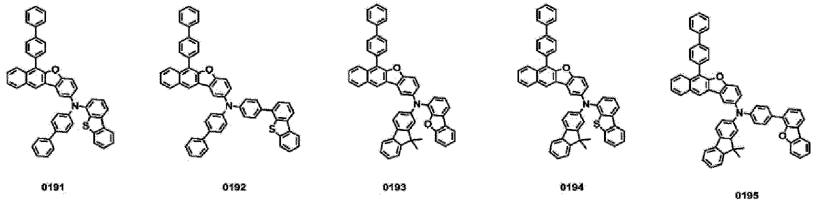

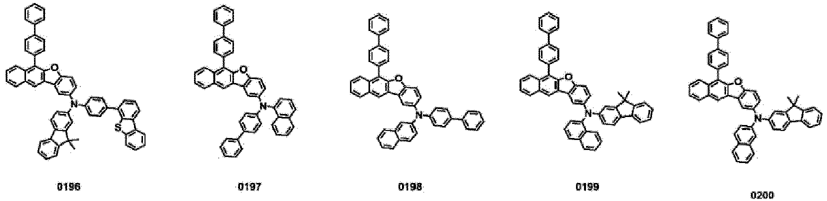

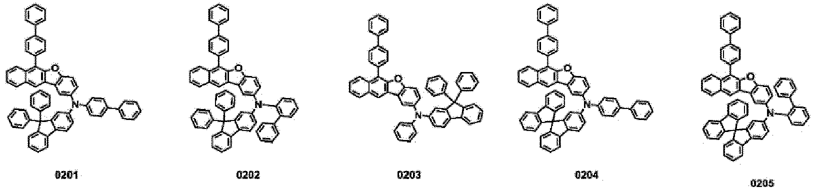

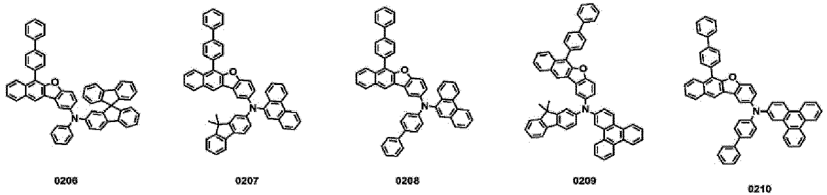

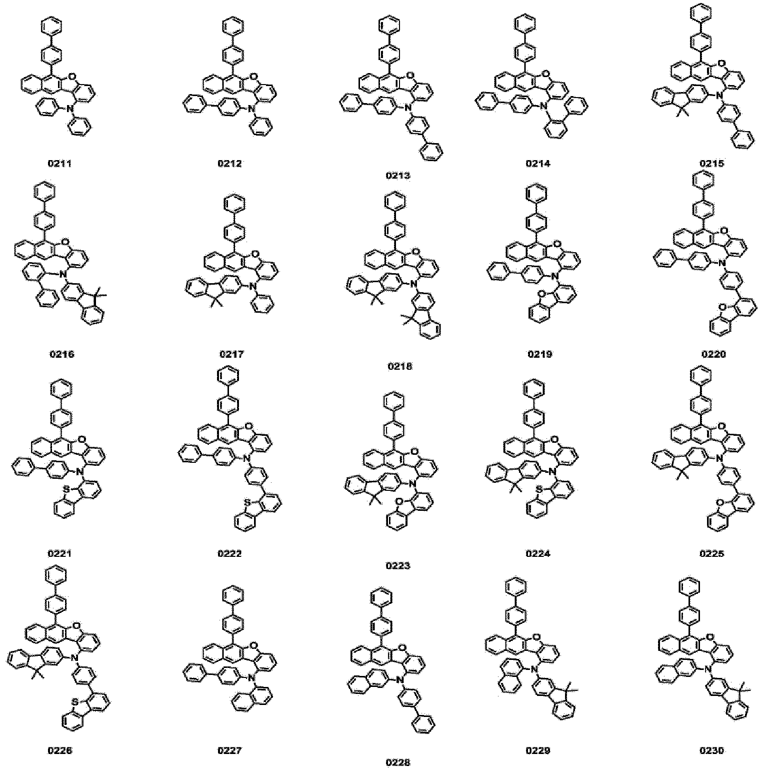

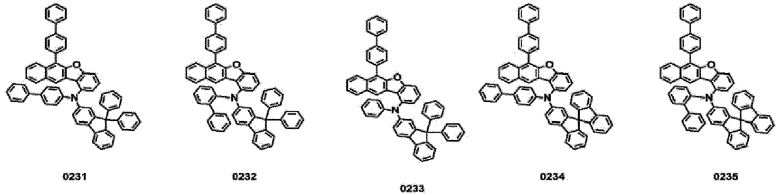

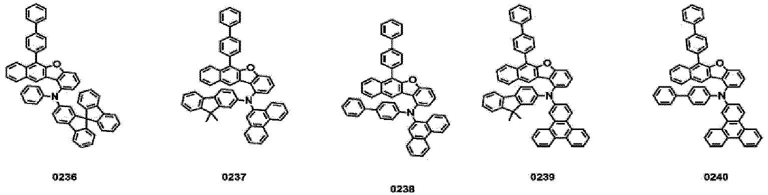

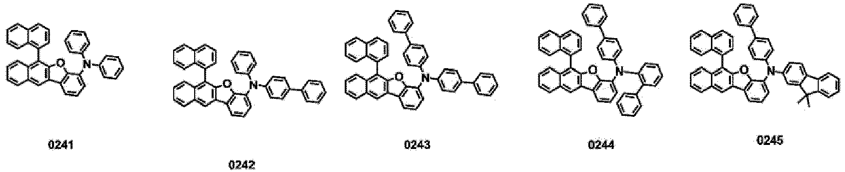

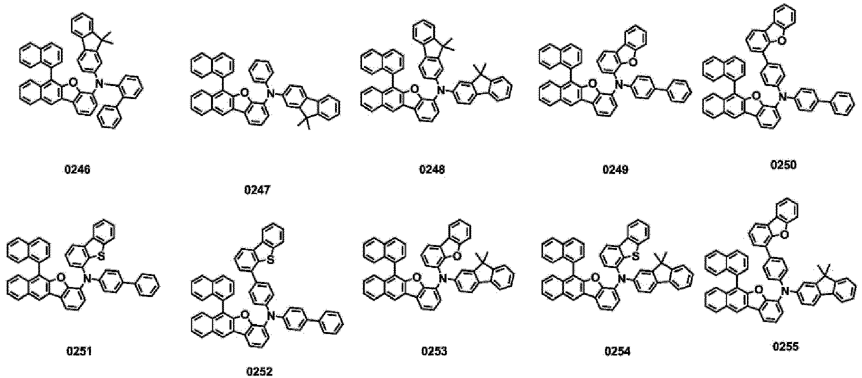

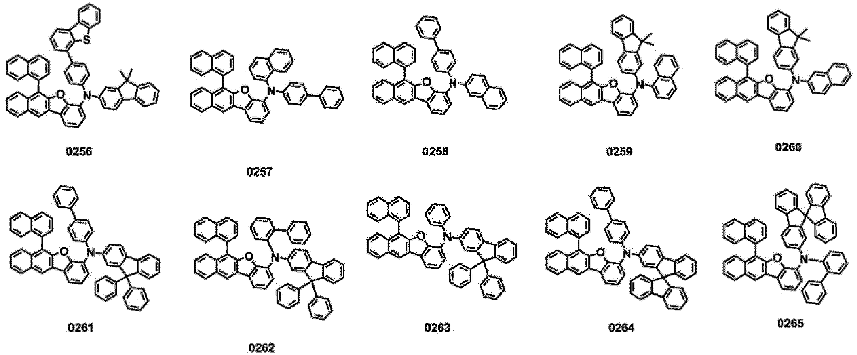

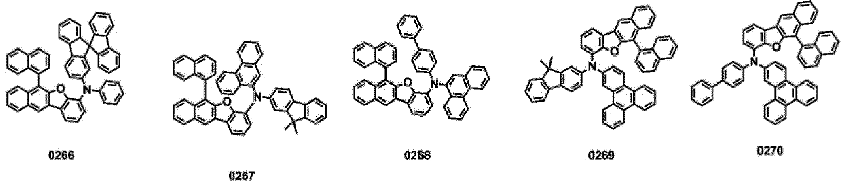

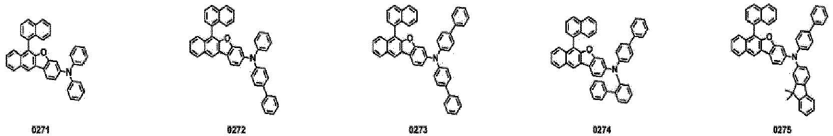

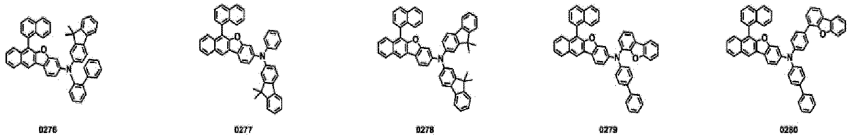

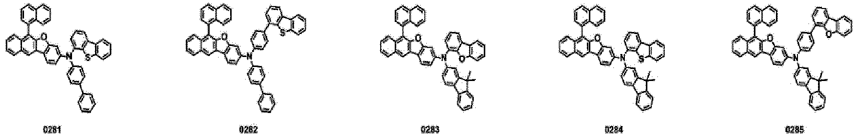

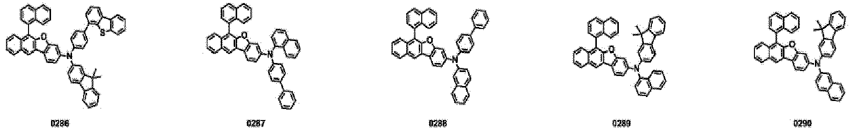

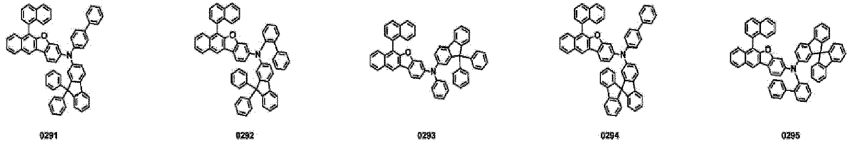

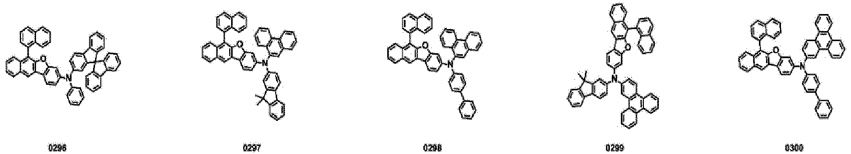

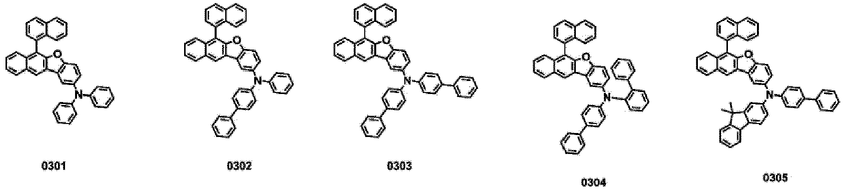

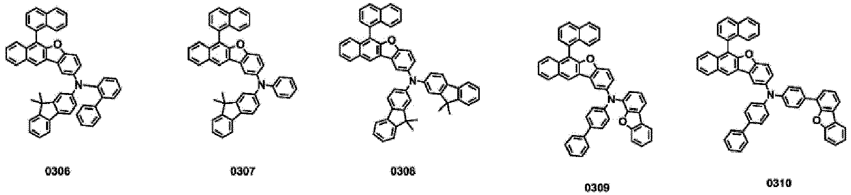

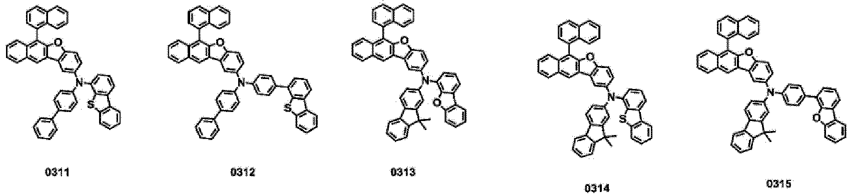

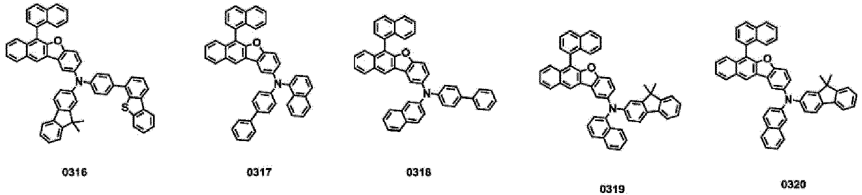

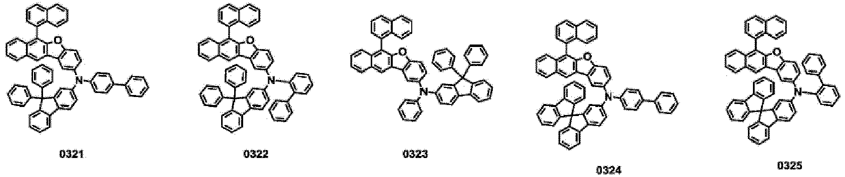

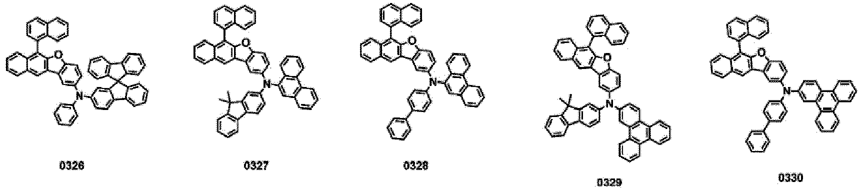

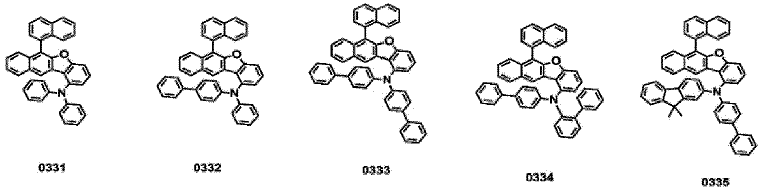

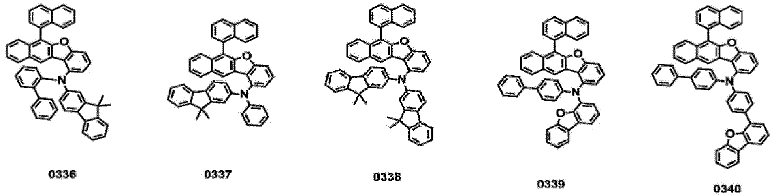

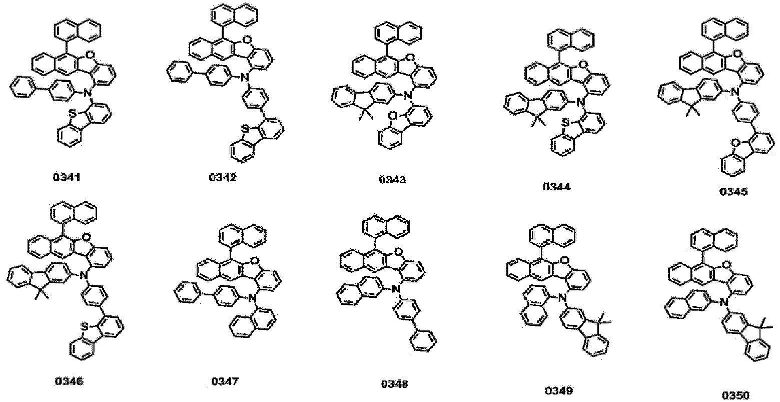

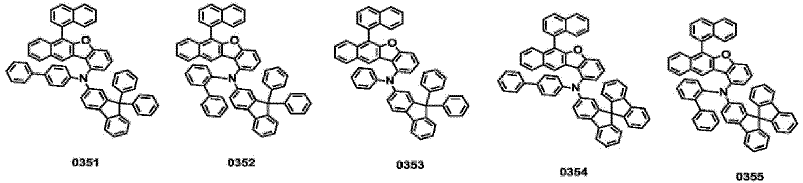

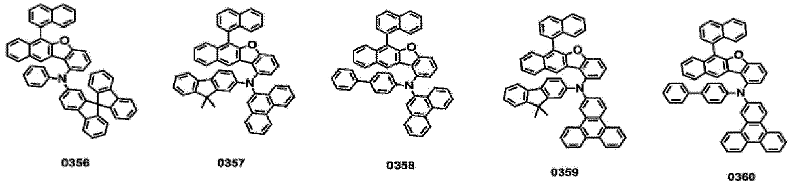

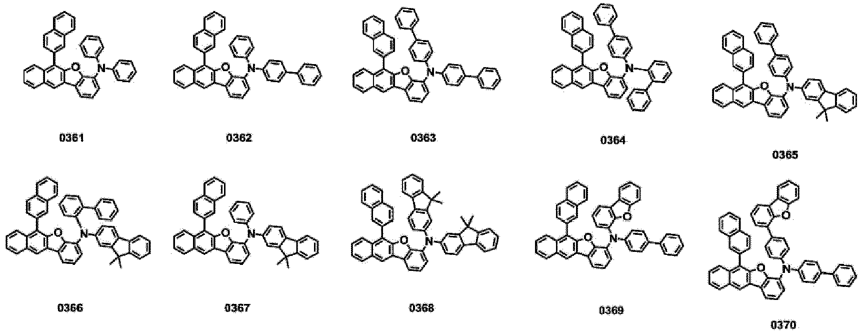

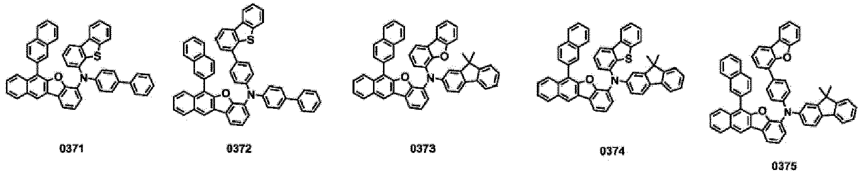

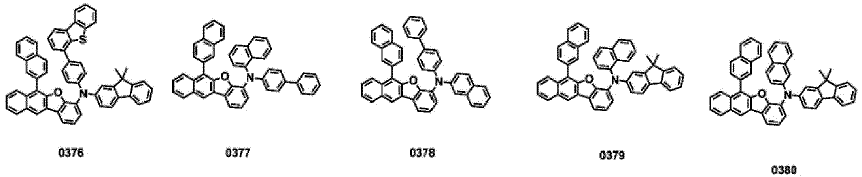

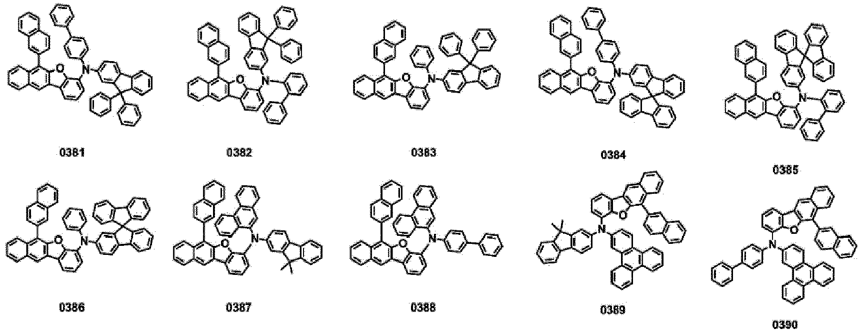

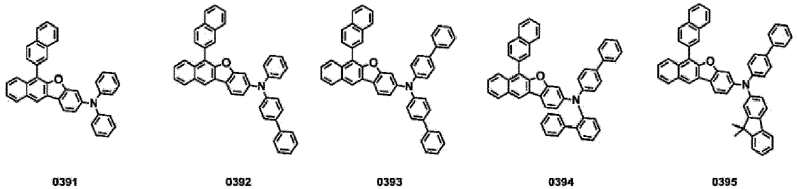

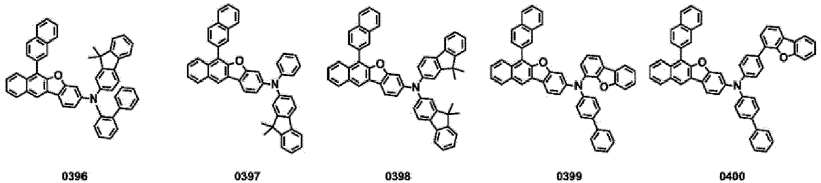

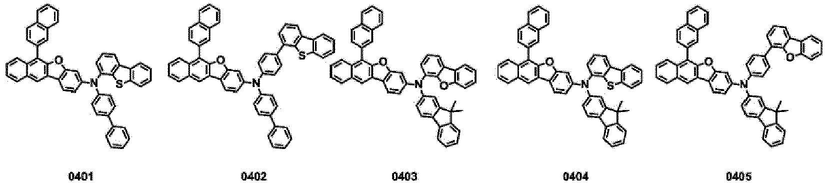

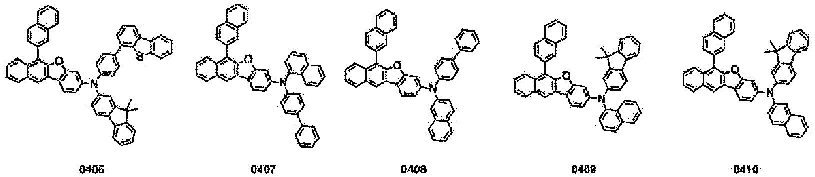

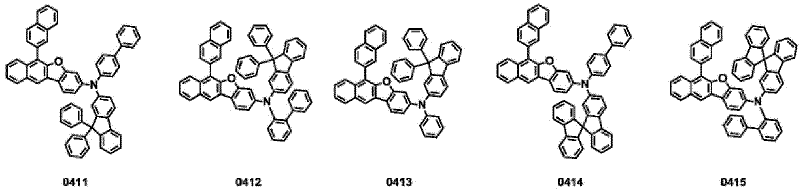

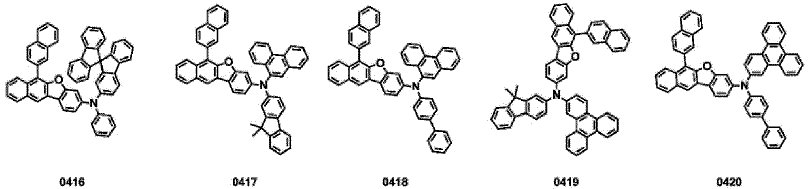

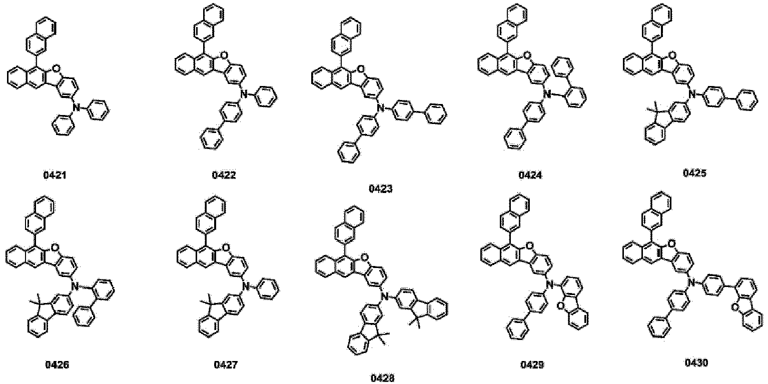

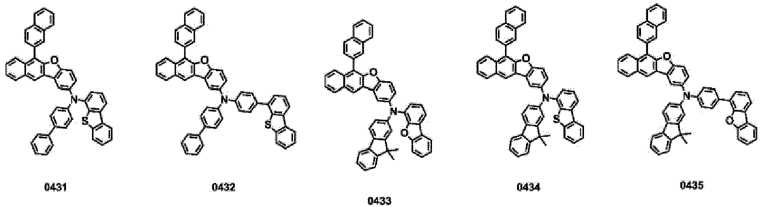

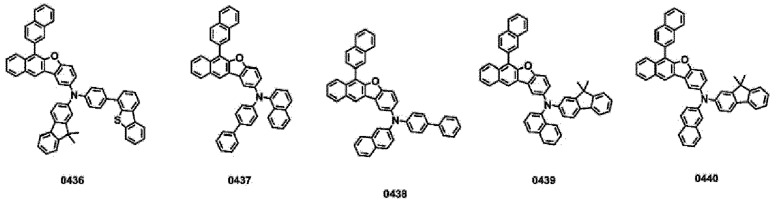

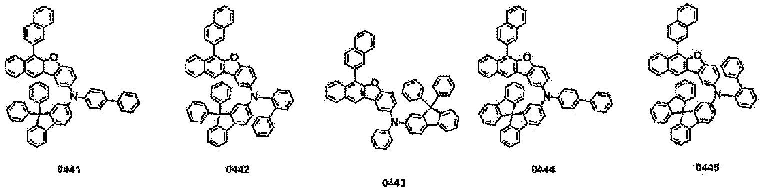

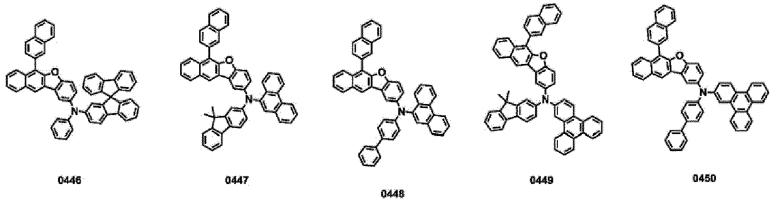

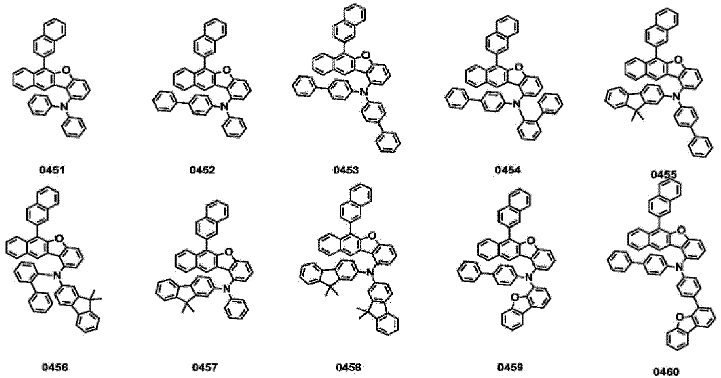

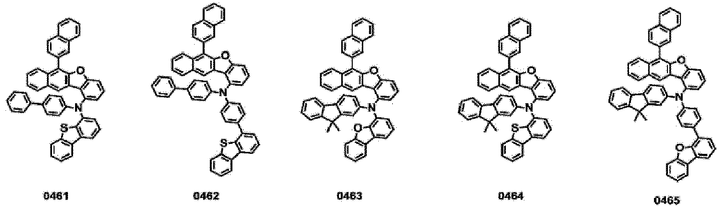

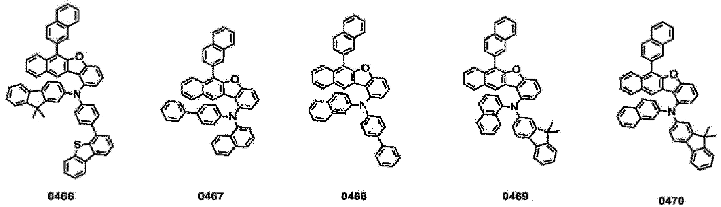

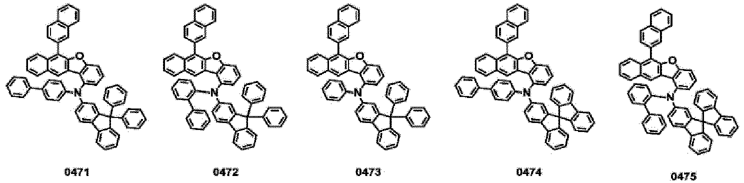

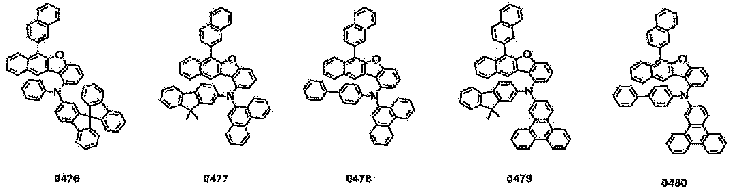

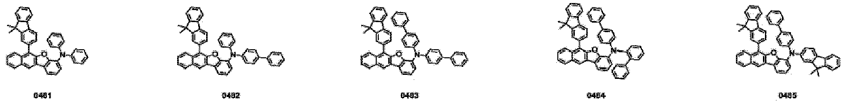

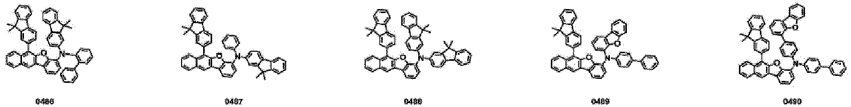

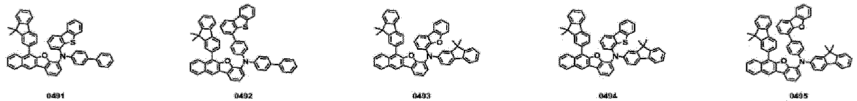

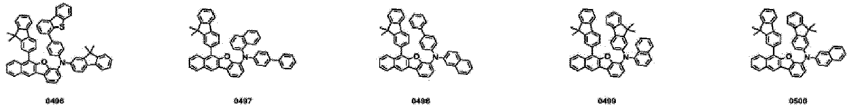

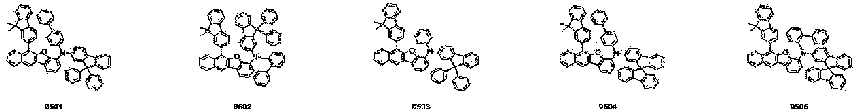

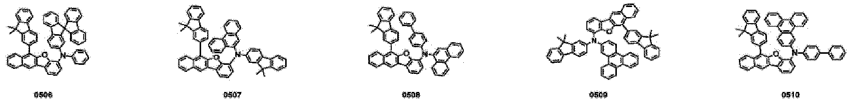

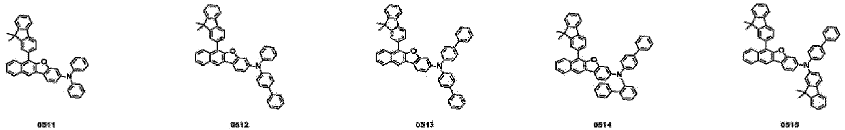

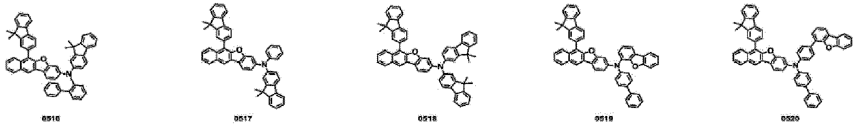

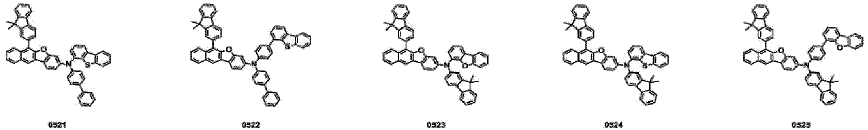

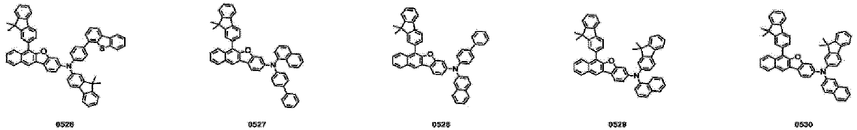

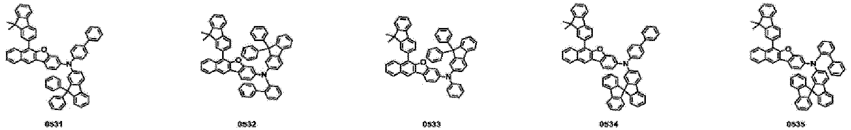

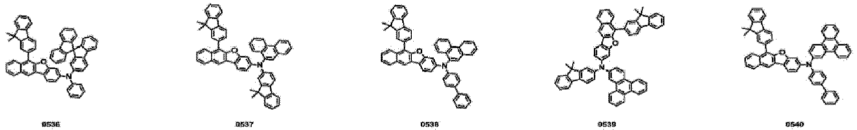

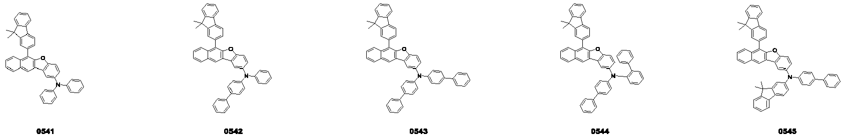

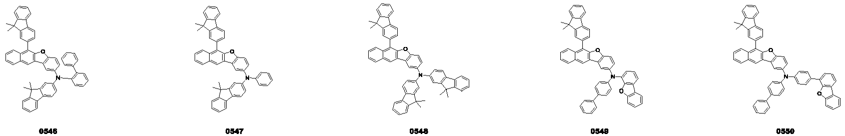

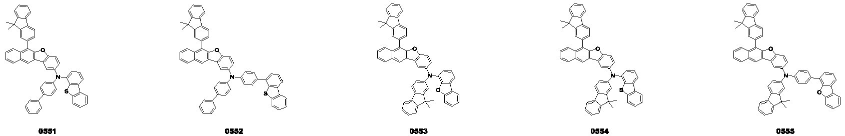

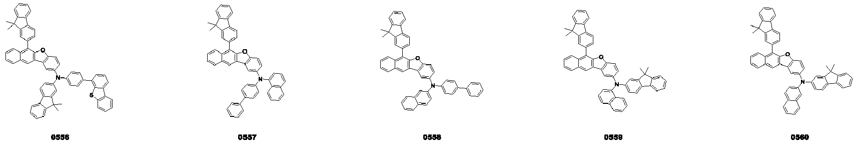

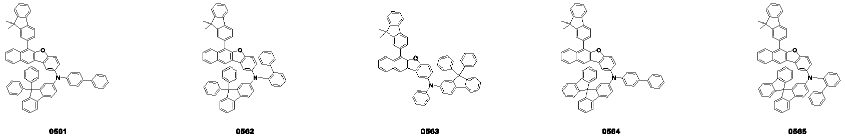

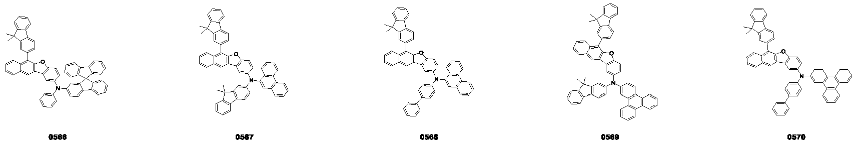

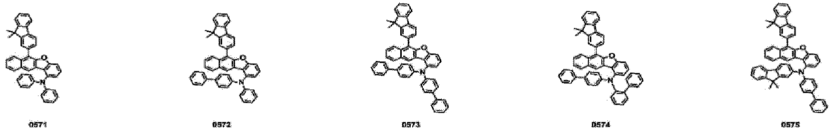

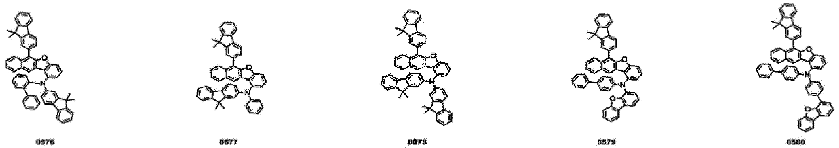

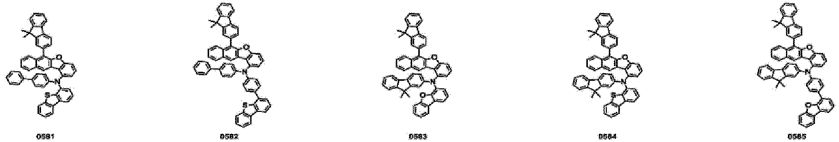

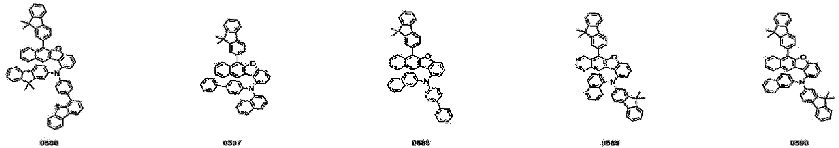

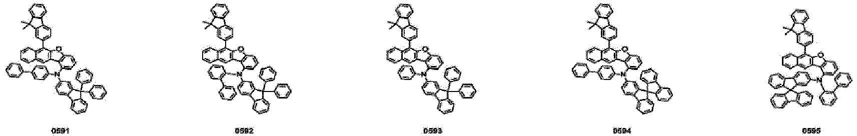

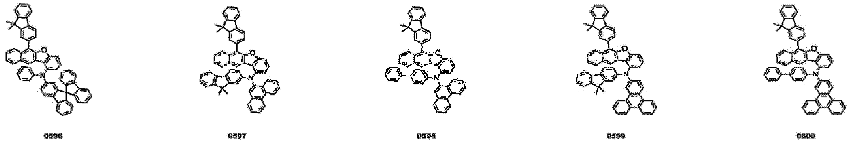

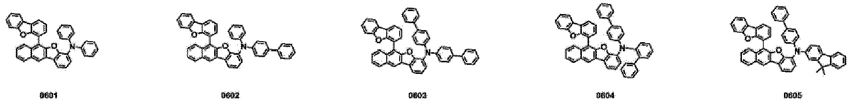

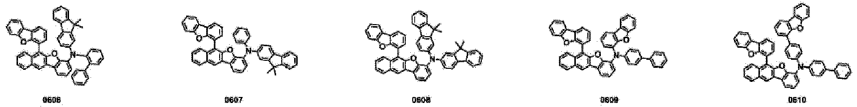

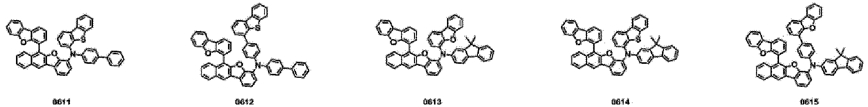

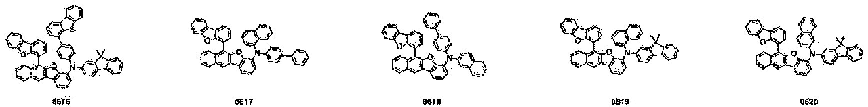

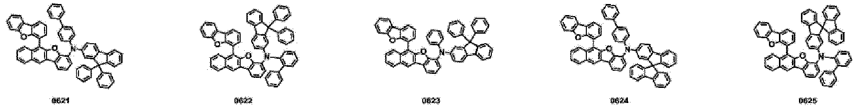

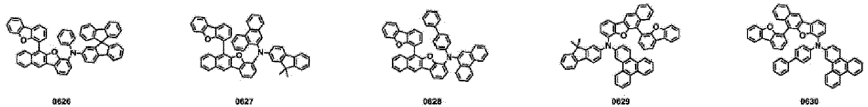

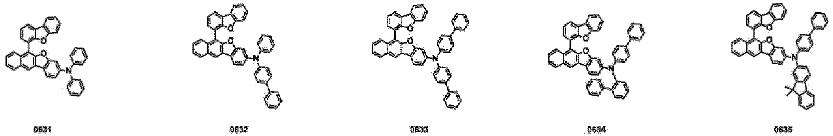

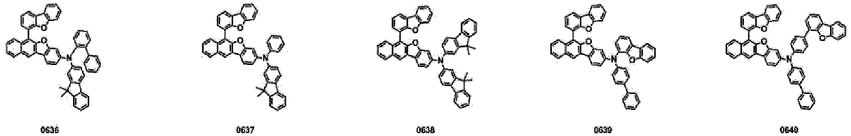

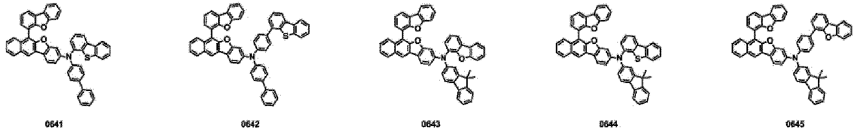

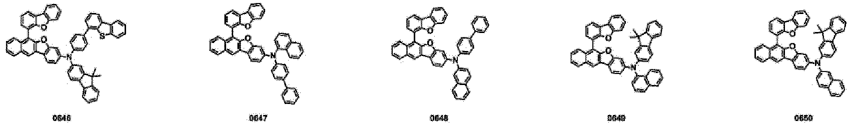

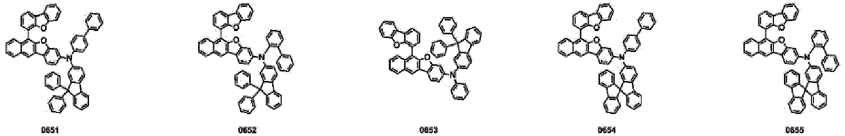

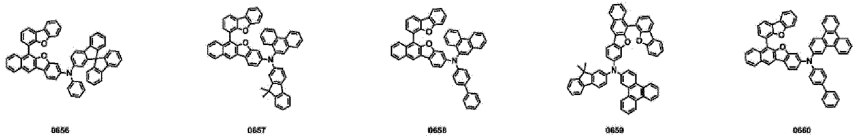

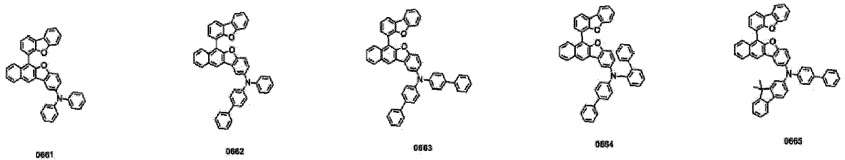

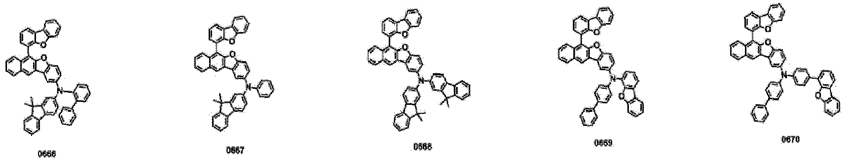

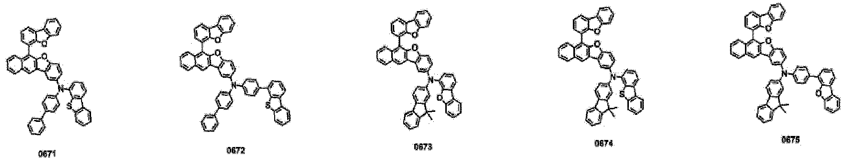

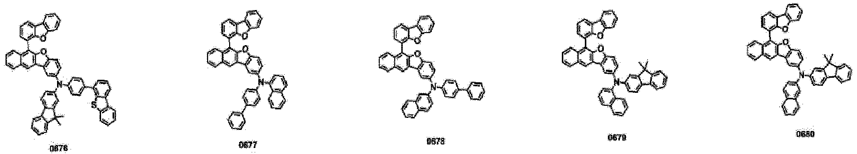

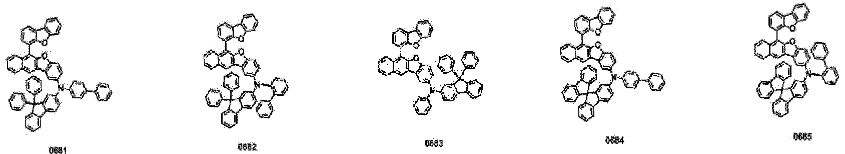

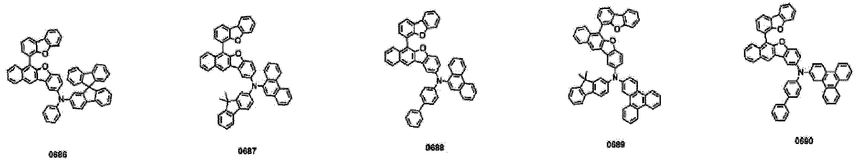

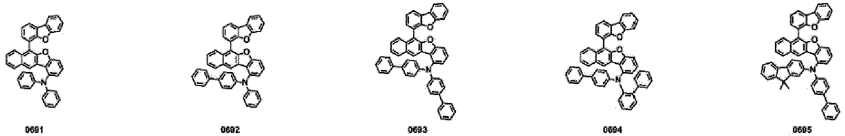

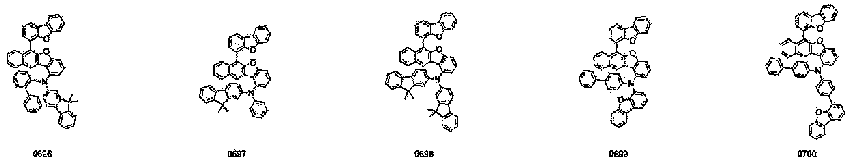

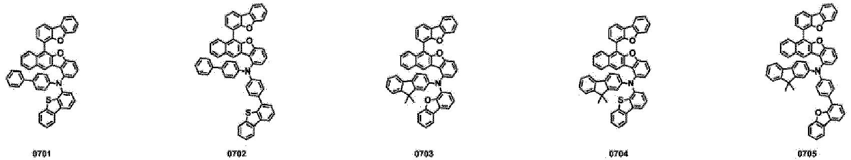

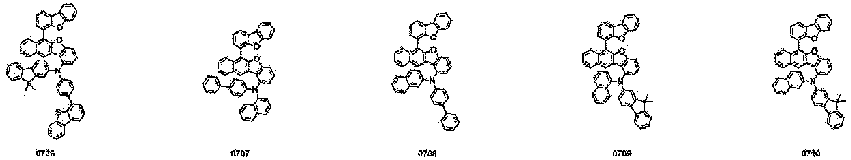

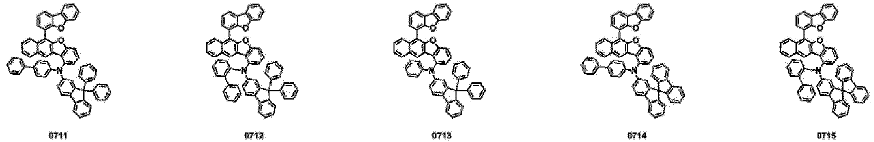

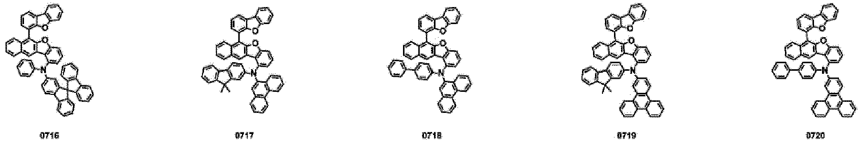

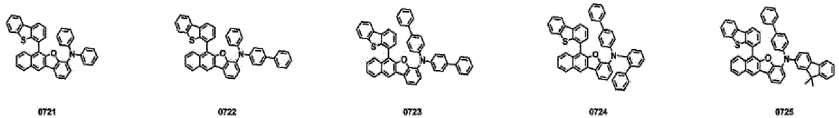

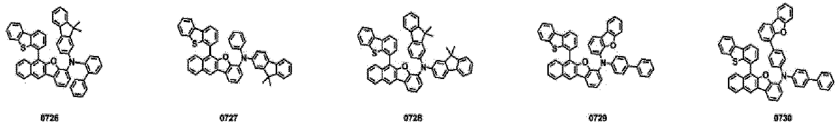

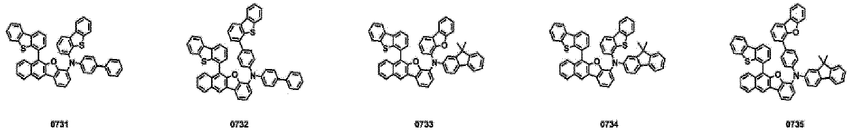

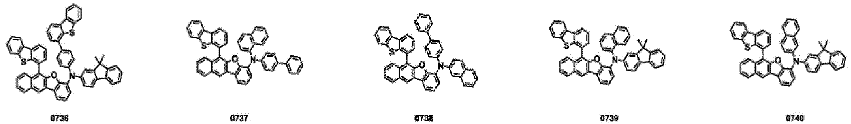

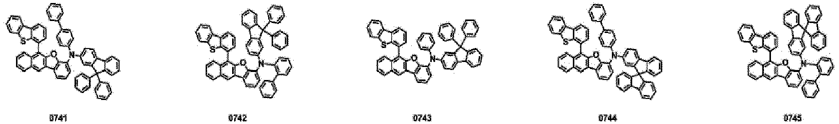

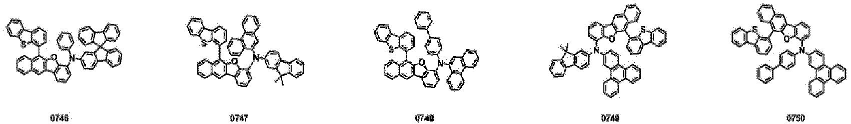

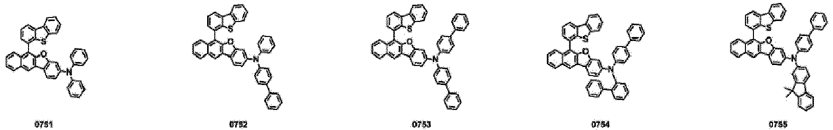

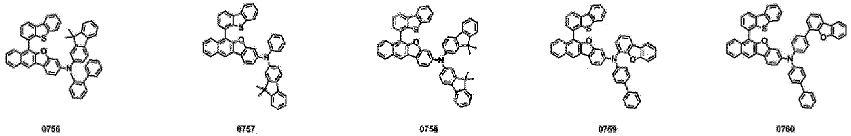

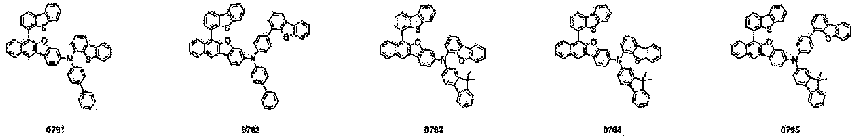

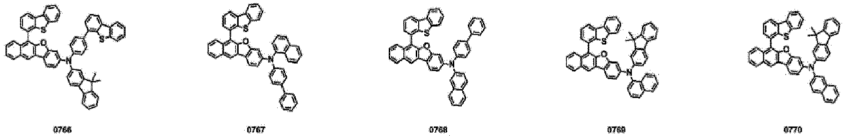

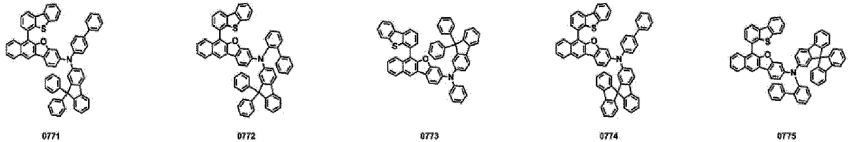

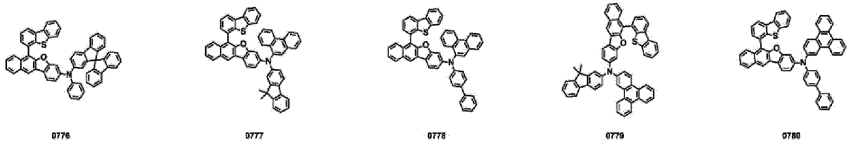

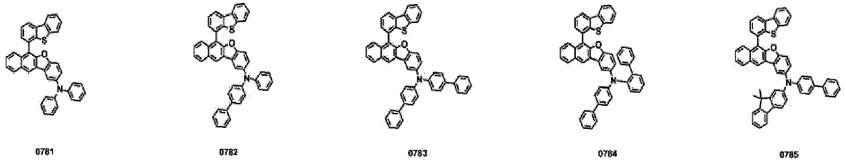

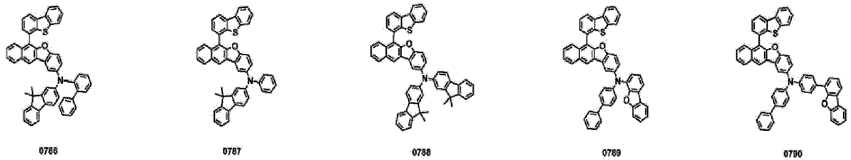

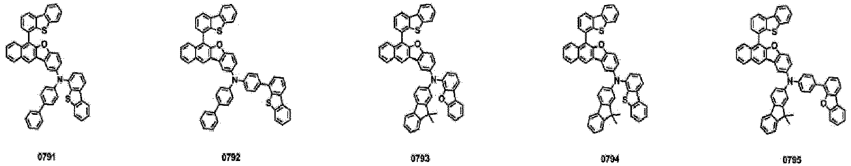

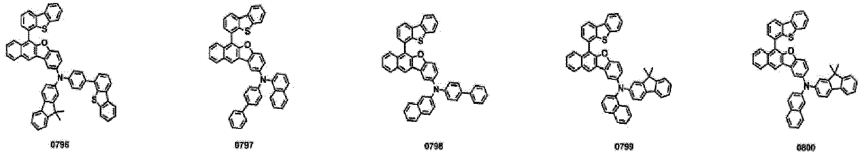

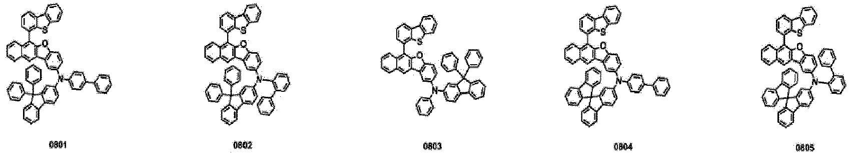

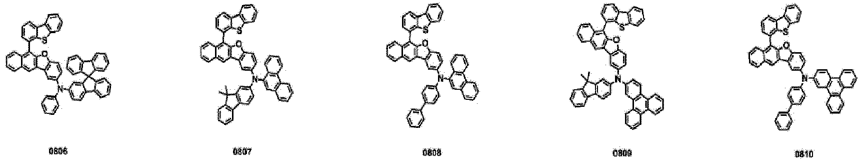

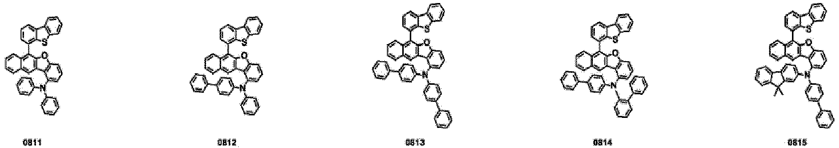

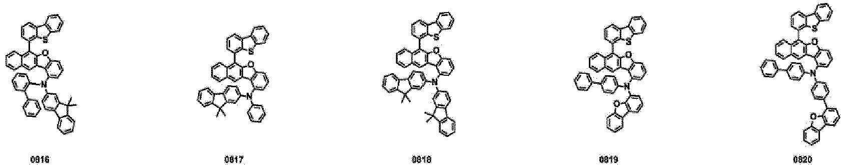

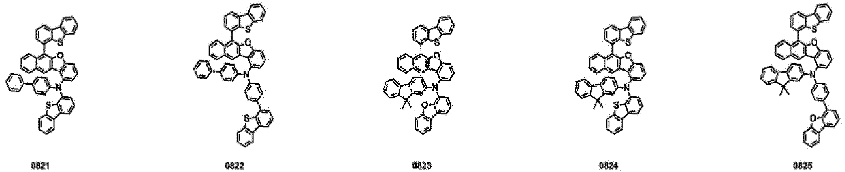

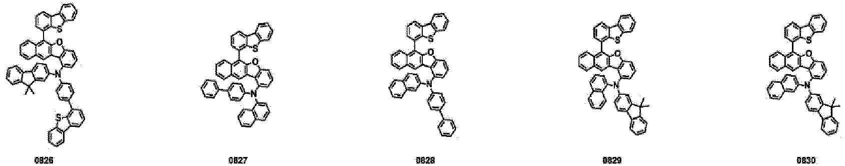

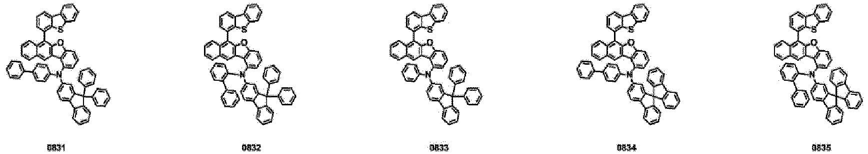

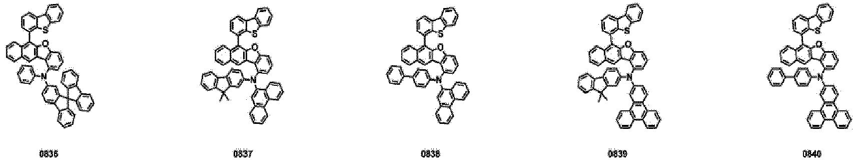

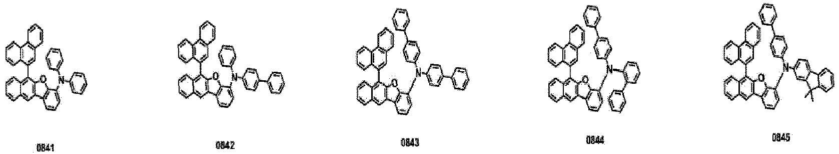

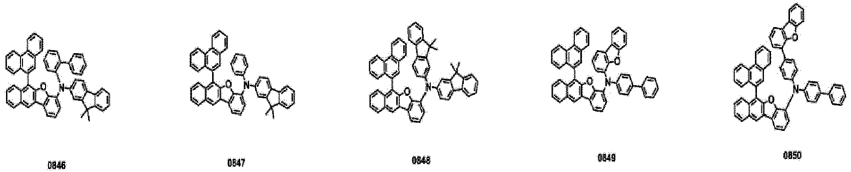

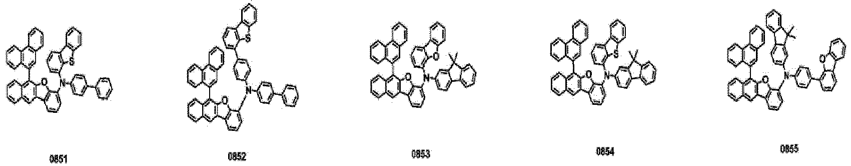

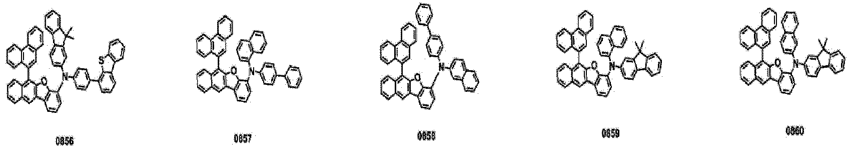

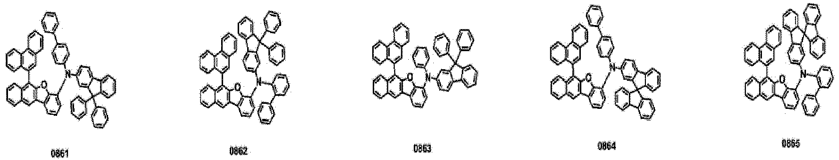

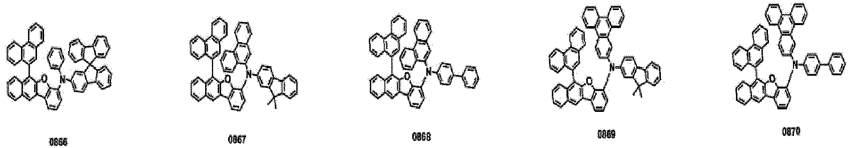

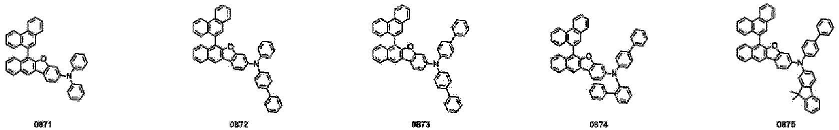

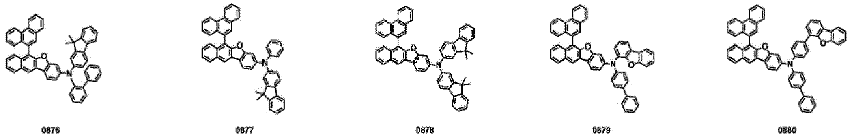

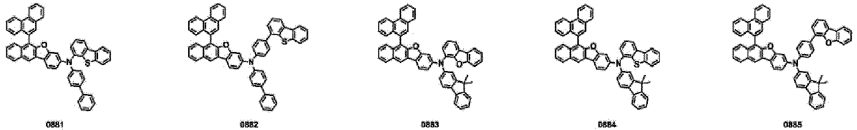

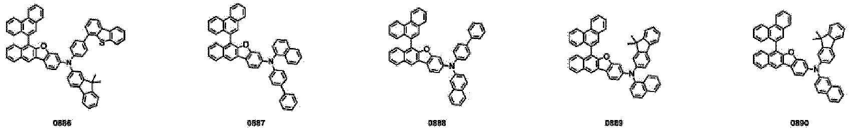

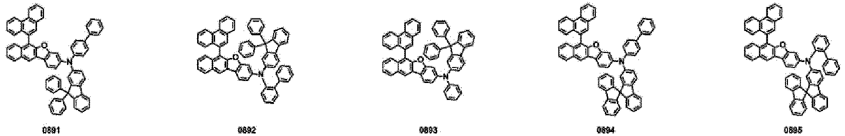

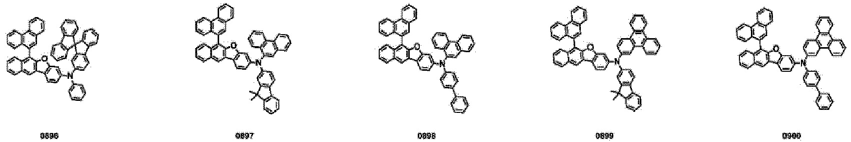

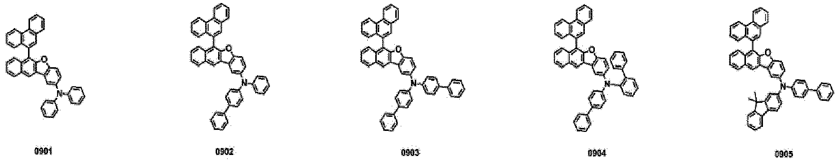

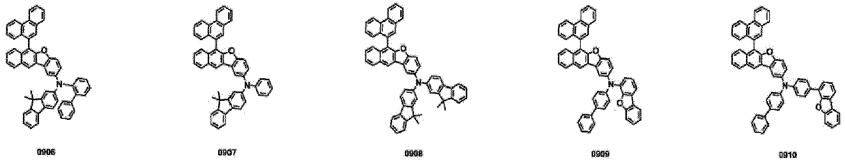

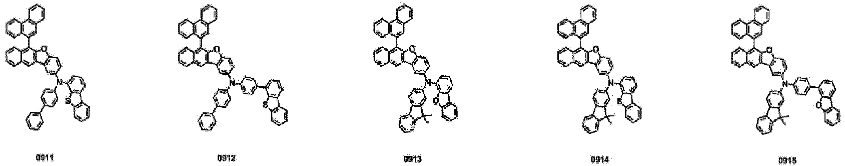

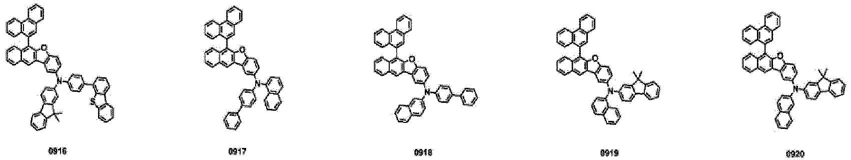

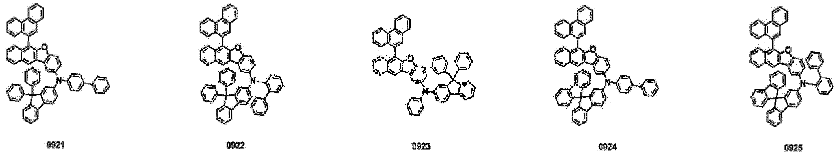

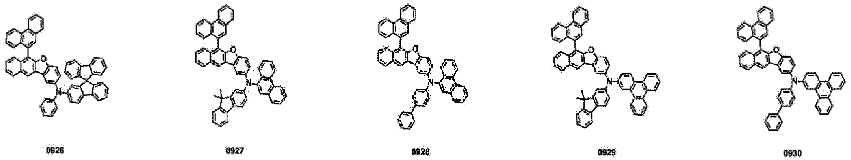

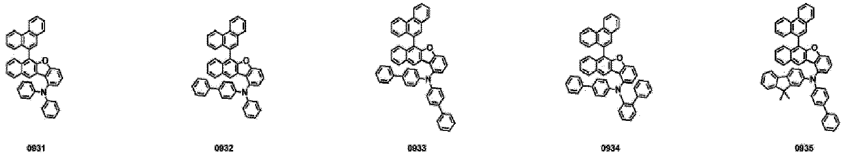

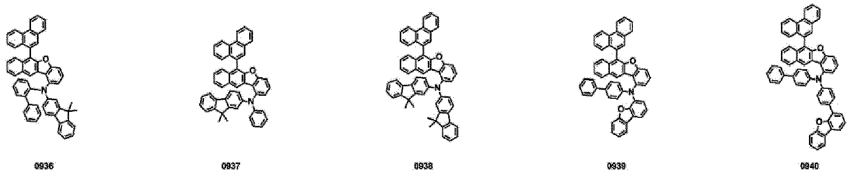

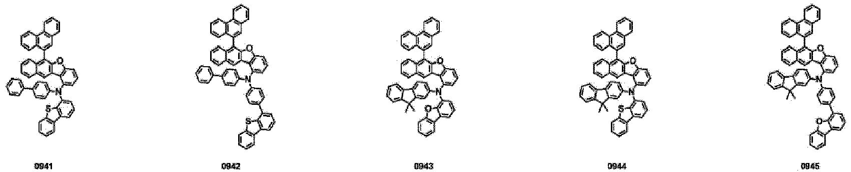

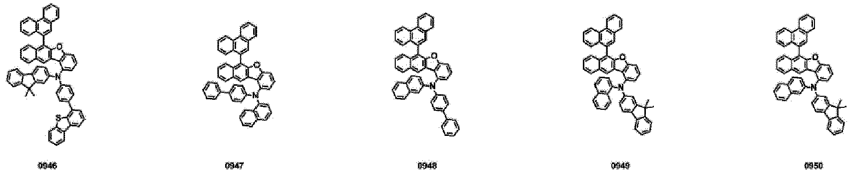

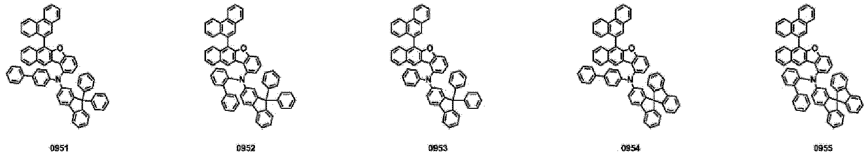

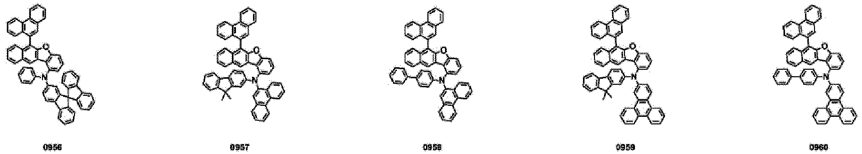

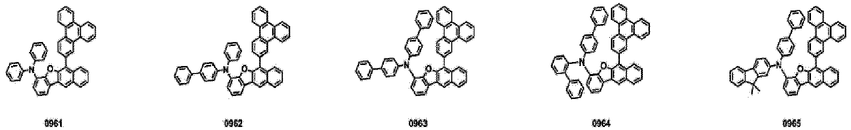

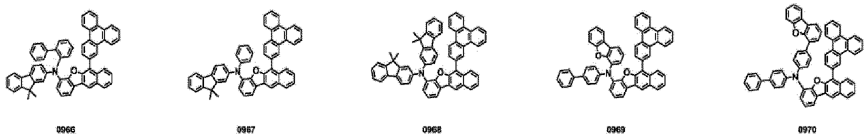

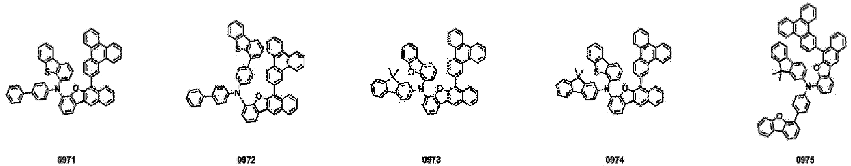

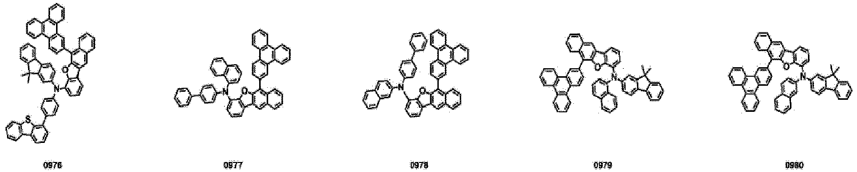

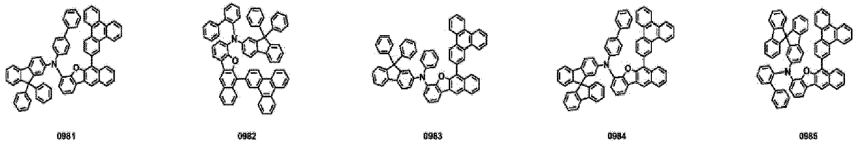

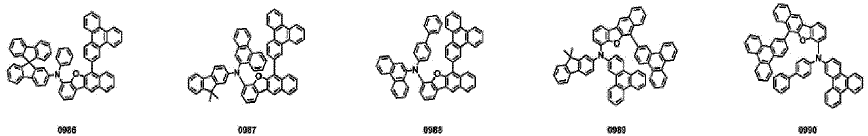

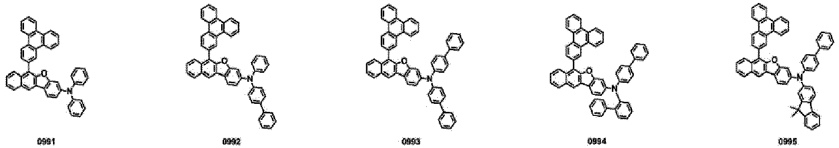

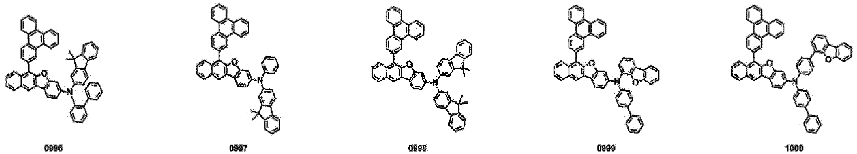

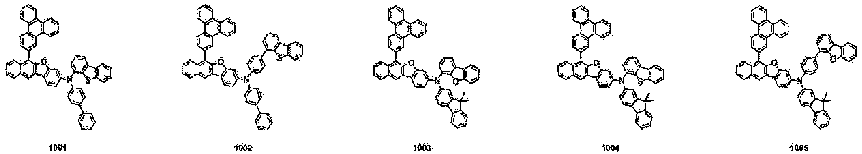

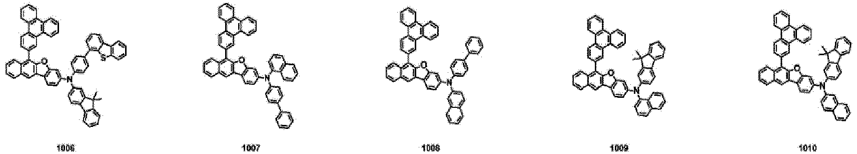

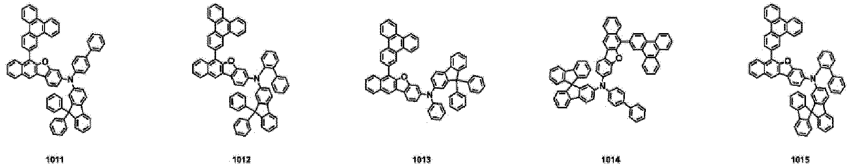

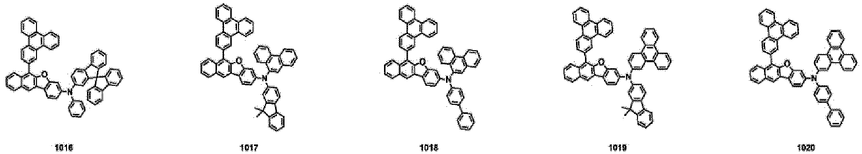

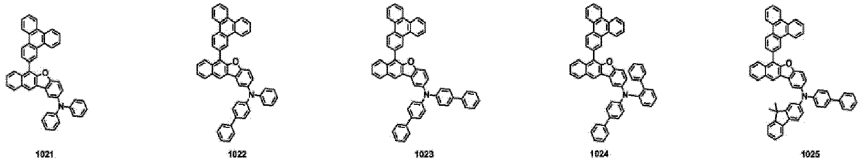

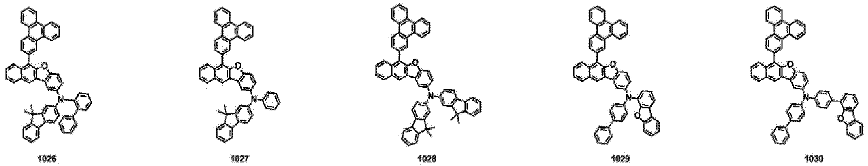

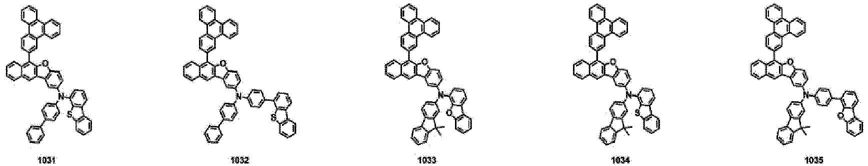

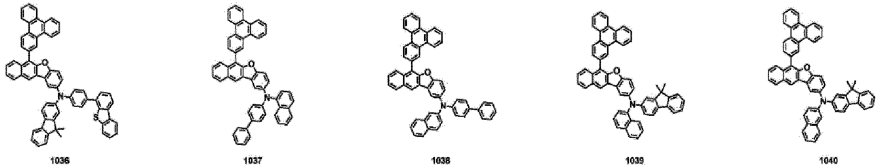

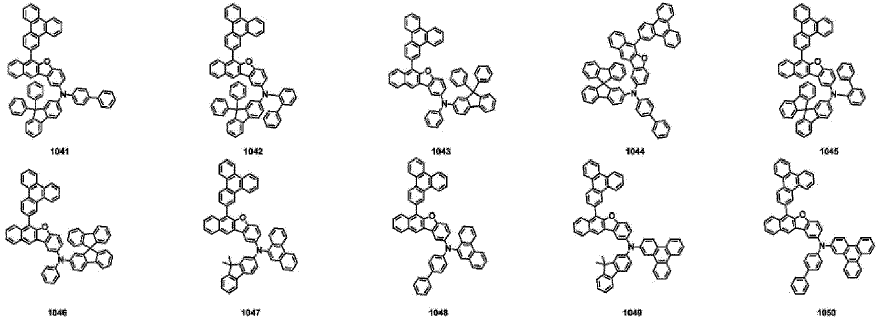

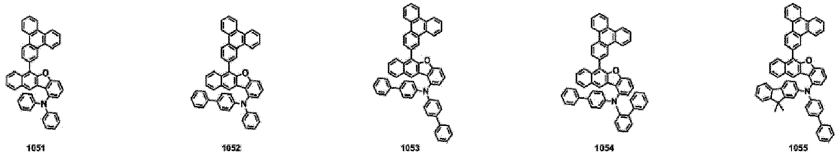

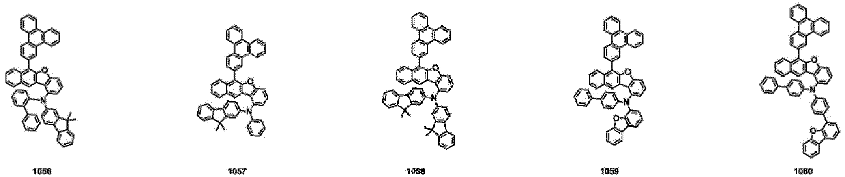

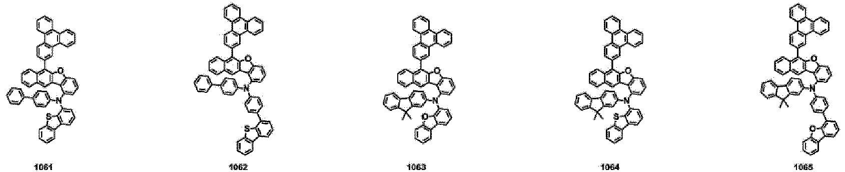

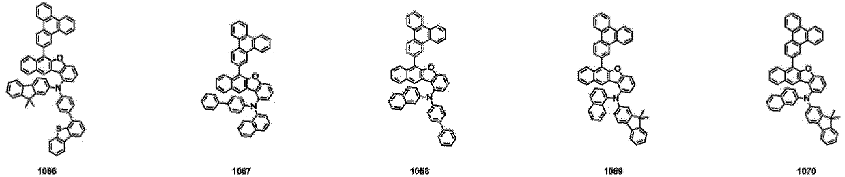

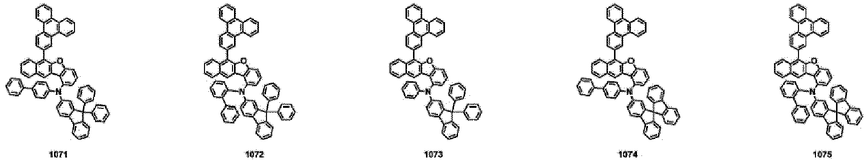

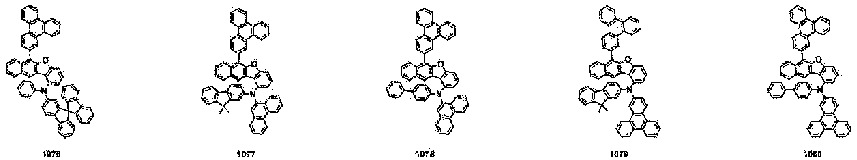

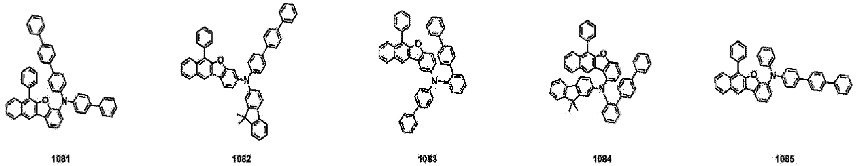

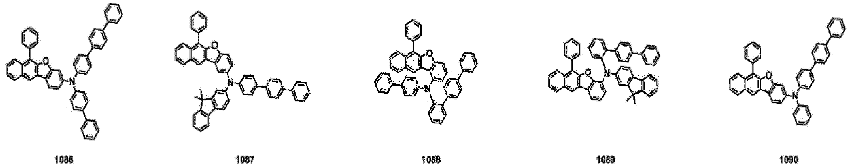

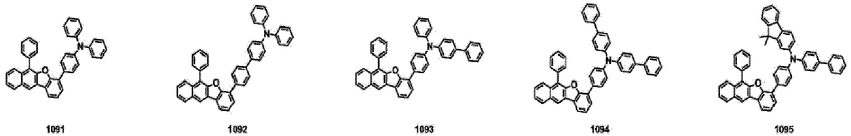

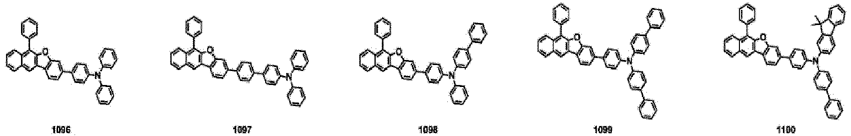

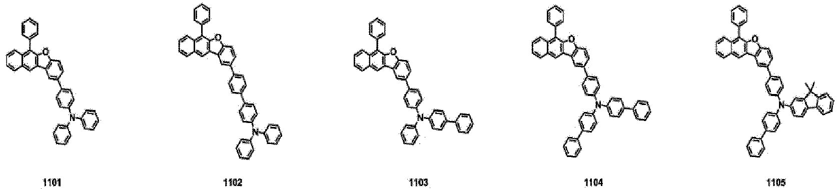

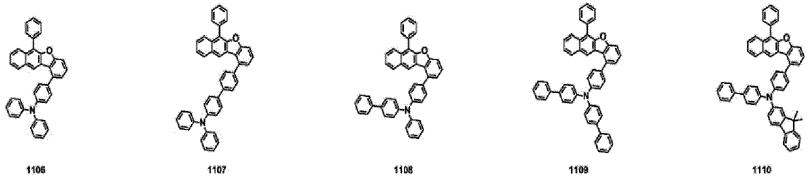

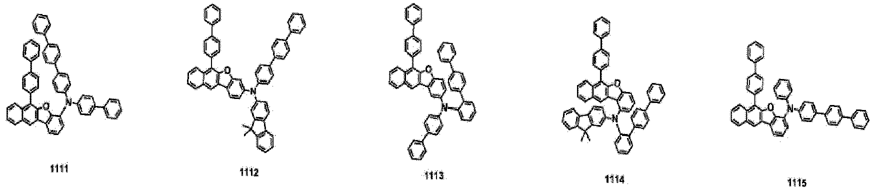

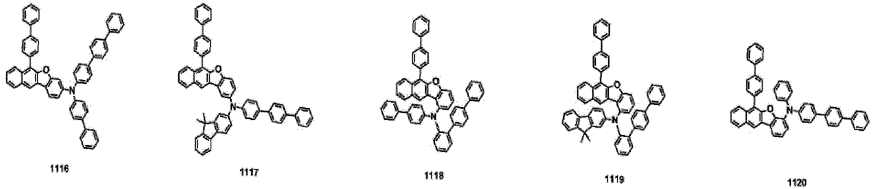

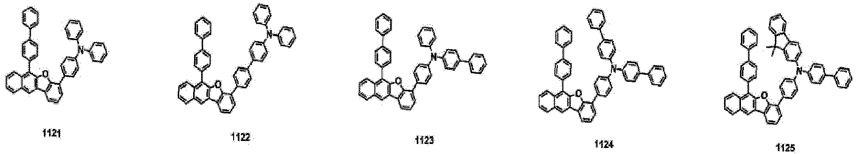

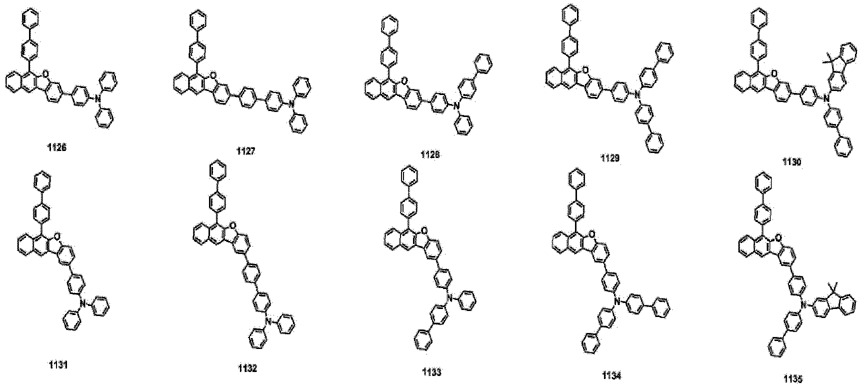

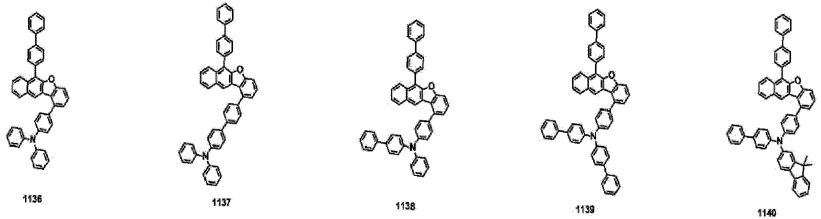

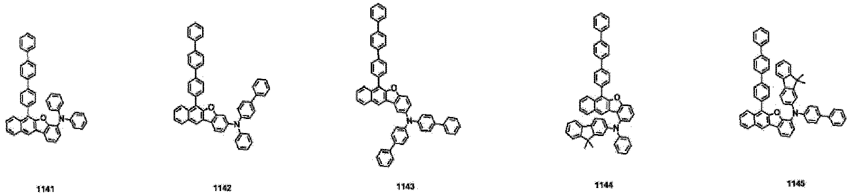

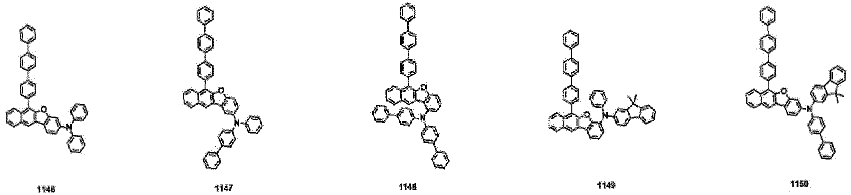

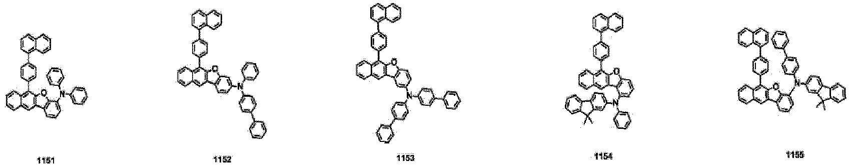

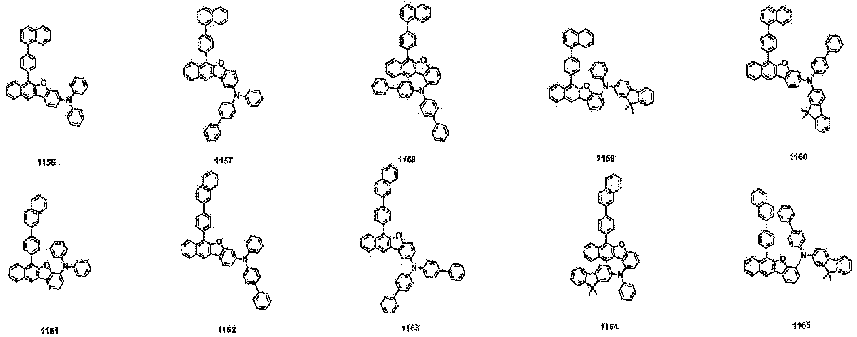

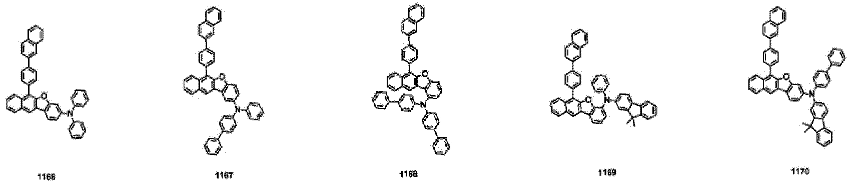

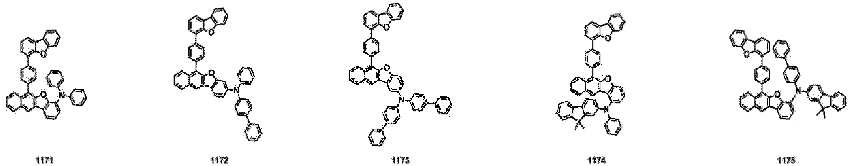

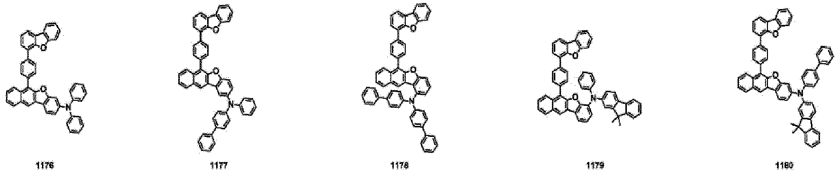

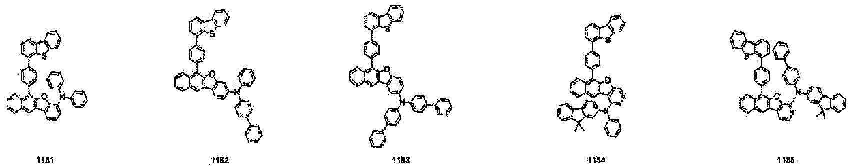

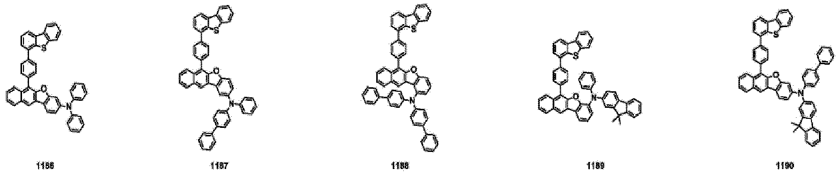

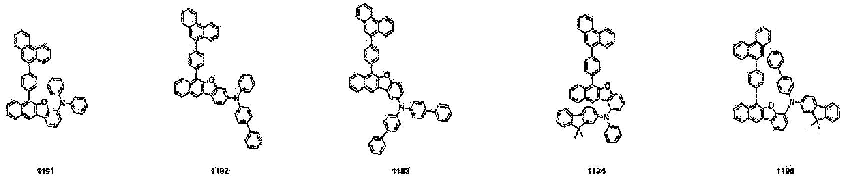

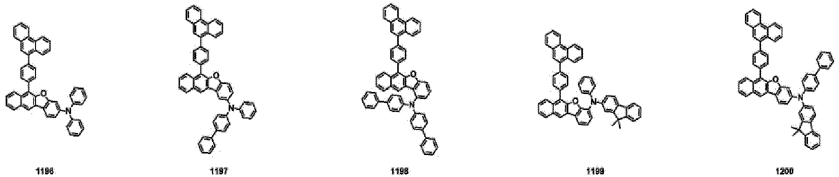

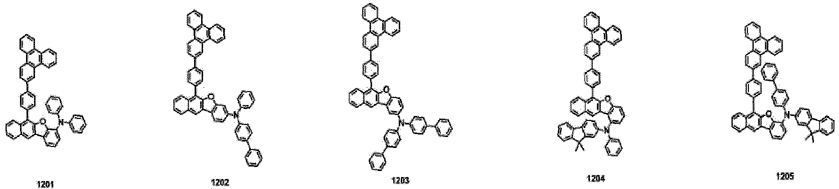

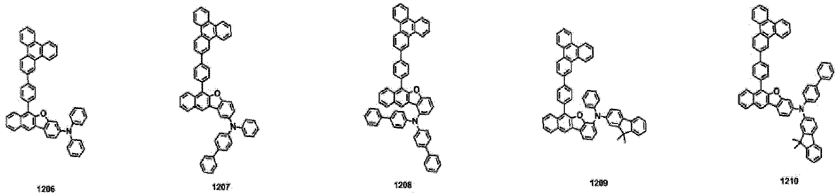

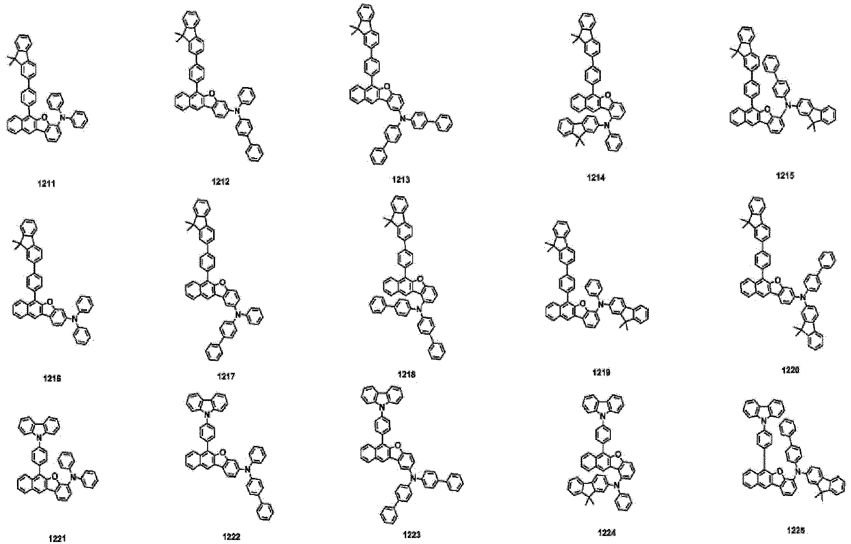

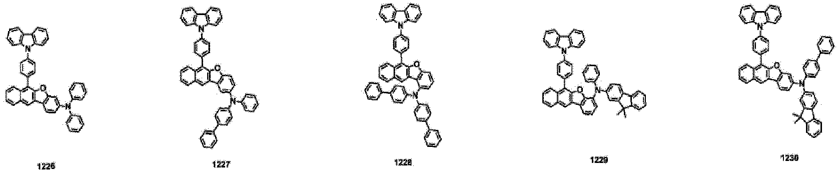

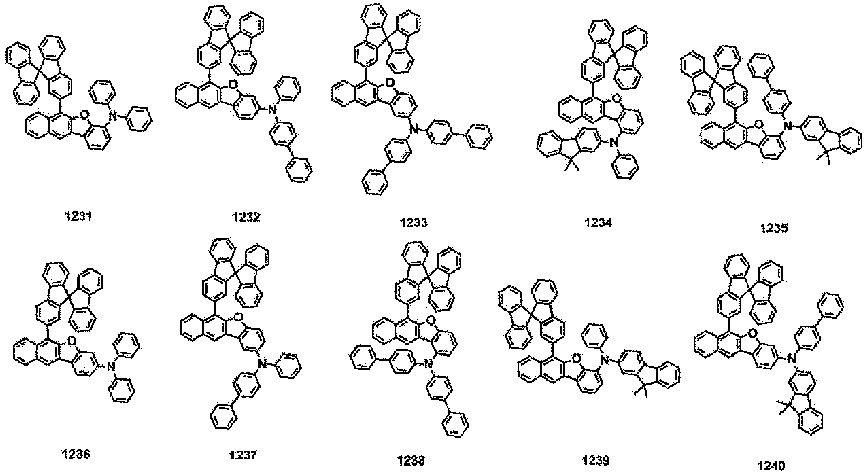

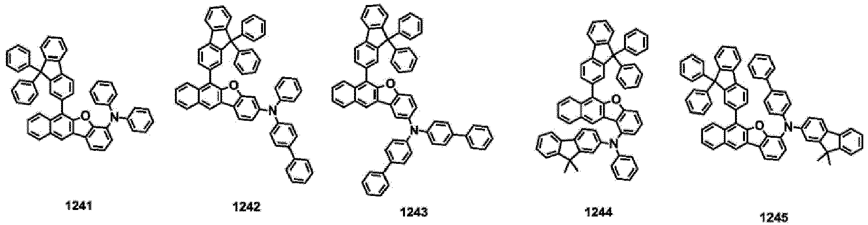

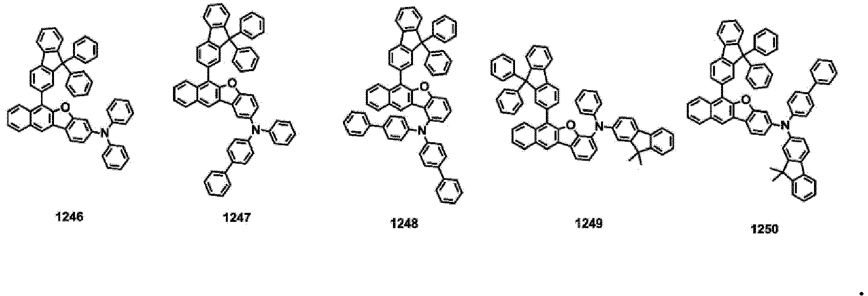

7. An organic light emitting device comprising:
a first electrode;
a second electrode provided opposite to the first electrode; and
one or more organic material layers provided between the first electrode and the second electrode,
wherein one or more layers of the organic material layers comprise the heterocyclic compound of any one of Claims 1 to 6.

8. The organic light emitting device of Claim 7, wherein the organic material layer comprises a light emitting layer, and the light emitting layer comprises the heterocyclic compound.

9. The organic light emitting device of Claim 7, wherein the organic material layer comprises an electron injection layer or an electron transfer layer, and the electron injection layer or the electron transfer layer comprises the heterocyclic compound.

10. The organic light emitting device of Claim 7, wherein the organic material layer comprises an electron blocking layer or a hole blocking layer, and the electron blocking layer or the hole blocking layer comprises the heterocyclic compound.

11. The organic light emitting device of Claim 7, wherein the organic material layer comprises a hole injection layer or a hole transfer layer, and the hole injection layer or the hole transfer layer comprises the heterocyclic compound.

12. The organic light emitting device of Claim 7, further comprising one, two or more layers selected from the group consisting of a light emitting layer, a hole injection layer, a hole transfer layer, an electron injection layer, an electron transfer layer, an electron blocking layer and a hole blocking layer.
